# EUROPÄISCHE PATENTANMELDUNG

(11) **EP 1 306 371 A1**
(43) Veröffentlichungstag der Anmeldung: **02.05.2003**
(21) Anmeldenummer: 03000371.9
(22) Anmeldetag: 07.11.1997
(51) Int. Cl.: C07D 207/267, C07D 207/26

(54) **Cyclische Imine**

(30) Priorität: 20.11.1996 DE 19648011
(62) Teilanmeldung aus: 97950138.4
(71) Anmelder: Bayer CropScience AG, 40789 Monheim (DE)
(72) Erfinder: Plant, Andrew, Wokingham, Berkshire RG41 5PD (GB); Kleefeld, Gerd, 41468 Neuss-Üdesheim (DE); Pötter, Thorsten, 51061 Köln (DE); Erdelen, Christoph, 42799 Leichlingen (DE); Mencke, Norbert, 51381 Leverkusen (DE); Turberg, Andreas, 40699 Erkrath (DE); Wachendorf-Neumann, 56566 Neuwied (DE)

(57) **Zusammenfassung**

Lactame der Formeln (XI) und (XI-a) 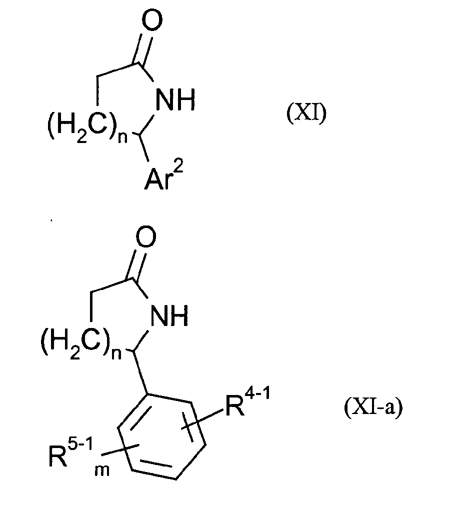 in welcher n, Ar², R⁴⁻¹ und R⁵⁻¹ die in der Beschreibung angegebenen Bedeutungen haben.

## Beschreibung

Die Erfindung betrifft neue cyclische Imine, mehrere Verfahren zu ihrer Herstellung und ihre Verwendung als Schädlingsbekämpfungsmittel.

Bisher sind nur wenige substituierte cyclische α,α'-Diphenylimine bekannt geworden: Drei im 2-Phenylring alkoxysubstituierte 2,5-Diphenyl-1-pyrroline [5-(2,5-Dimethoxyphenyl)-2-phenyl-3,4-dihydro-2H-pyrrol und 5-(4-Methoxyphenyl)-2-phenyl-3,4-dihydro-2H-pyrrol aus Chem. Ber. **96,** 93 (1963) und die entsprechende 4-Propoxy-Verbindung aus J. Prakt. Chem., Serie 4, **1,** 57 (1955)] sowie das nicht weiter substituierte 2,6-Diphenyl-3,4,5,6-tetrahydropyridin [vgl. z.B. Bull. Soc. Chim. Fr. **1974**, 258 u. Chem. Ber. **116**, 3931 (1983)].

Über deren Eignung zur Verwendung als Schädlingsbekämpfungsmittel ist nichts bekannt.

Es wurden neue cyclische Imine der Formel (I) 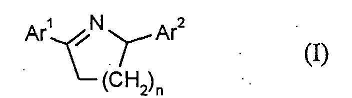 gefunden, in welcher
- n: für 1, 2 oder 3 steht,
- Ar¹: für den Rest 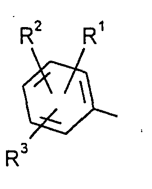 steht und
- Ar²: für den Rest 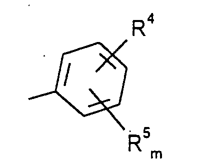 steht, in denen
- m: für 0, 1, 2, 3 oder 4 steht,
- R¹: für Halogen, Cyano, Nitro, Alkyl, Alkoxy, Halogenalkyl, Halogenalkoxy, Alkoxyalkyl, -S(O)ₒR⁶ oder -NR⁷R⁸ steht,
- R² und R³: unabhängig voneinander für Wasserstoff, Halogen, Cyano, Nitro, Alkyl, Alkoxy, Halogenalkyl, Halogenalkoxy, Alkoxyalkyl, -S(O)ₒR⁶ oder -NR⁷R⁸ stehen,
- R⁴: für Halogen, Cyano, Trialkylsilyl, -CO-NR¹⁰R¹¹, Tetrahydropyranyl oder für eine der folgenden Gruppierungen

(l) -X―A

(m) -B―Z―D

(n) -Y―E

steht,
- R⁵: für Wasserstoff, Halogen, Cyano, Nitro, Alkyl, Alkoxy, Halogenalkyl, Halogenalkoxy, Alkoxyalkoxy oder -S(O)ₒR⁶ steht,
- O: für 0, 1 oder 2 steht,
- R⁶: für Alkyl oder Halogenalkyl steht,
- R⁷ und R⁸: unabhängig voneinander für Wasserstoff oder Alkyl, oder gemeinsam für Alkylen stehen,
- R¹⁰ und R¹¹: unabhängig voneinander für Wasserstoff, Alkyl, Halogenalkyl oder für jeweils gegebenenfalls einfach oder mehrfach durch Reste aus der Liste W¹ substituiertes Phenyl oder Phenylalkyl stehen,
- X: für eine direkte Bindung, Sauerstoff, Schwefel, Carbonyl, Carbonyloxy, Oxycarbonyl, Alkylen, Alkenylen, Alkinylen, Alkylenoxy, Oxyalkylen, Thioalkylen, Alkylendioxy oder Dialkylsilylen steht,
- A: für jeweils gegebenenfalls einfach oder mehrfach durch Reste aus der Liste W¹ substituiertes Phenyl, Naphthyl oder Tetrahydronaphthyl oder für jeweils gegebenenfalls einfach oder mehrfach durch Reste aus der Liste W² substituiertes 5- bis 10-gliedriges, 1 oder 2 aromatische Ringe enthaltendes Heterocyclyl mit einem oder mehreren Heteroatomen aus der Reihe Stickstoff, Sauerstoff und Schwefel steht,
- B: für gegebenenfalls einfach oder zweifach durch Reste aus der Liste W¹ substituiertes p-Phenylen steht,
- Z: für Sauerstoff oder Schwefel steht,
- D: für Wasserstoff, Alkyl, Alkenyl, Alkinyl, Halogenalkyl, Halogenalkenyl, jeweils gegebenenfalls durch Halogen, Alkyl, Alkenyl, Halogenalkenyl, Phenyl, Styryl, Halogenphenyl oder Halogenstyryl substituiertes Cycloalkyl oder Cycloalkylalkyl, für jeweils gegebenenfalls durch Halogen oder Alkyl substituiertes Cycloalkenyl oder Cycloalkenylalkyl, für jeweils gegebenenfalls durch Nitro, Halogen, Alkyl, Alkoxy, Halogenalkyl oder Halogenalkoxy substituiertes Phenylalkyl, Naphthylalkyl, Tetrahydronaphthylalkyl oder 5- oder 6-ringgliedriges Hetarylalkyl mit 1 oder 2 Heteroatomen aus der Reihe Stickstoff, Sauerstoff und Schwefel, für -CO-R¹², -CO-NR¹³R¹⁴ oder für die Gruppierung

-(CH₂)ₚ―(CR¹⁵R¹⁶)_{q}―(CH₂)ᵣ―G

steht, oder
- Z und D: gemeinsam für gegebenenfalls durch Nitro, Halogen, Alkyl, Alkoxy, Halogenalkyl oder Halogenalkoxy substituiertes Phenoxyalkyl stehen,
- Y: für eine direkte Bindung, Sauerstoff, Schwefel, Carbonyl, Carbonyloxy, Oxycarbonyl, Alkylen, Alkenylen, Alkinylen, Alkylenoxy, Oxyalkylen, Thioalkylen, Alkylendioxy oder für gegebenenfalls einfach oder zweifach durch Reste aus der Liste W¹ substituiertes p-Phenylen steht,
- E: für Wasserstoff, Alkyl, Alkenyl, Alkinyl, Halogenalkyl, Halogenalkenyl, jeweils gegebenenfalls durch Halogen, Alkyl, Alkenyl, Halogenalkenyl, Phenyl, Styryl, Halogenphenyl oder Halogenstyryl substituiertes Cycloalkyl, für jeweils gegebenenfalls durch Halogen oder Alkyl substituiertes Cycloalkenyl, für gegebenenfalls einfach bis vierfach durch Reste aus der Liste W¹ substituiertes Phenyl oder für jeweils gegebenenfalls einfach bis vierfach durch Reste aus der Liste W² substituiertes 5- oder 6-gliedriges Hetaryl mit 1 oder 2 Heteroatomen aus der Reihe Stickstoff, Sauerstoff und Schwefel oder für die Gruppierung

-(CH₂)ₚ―(CR¹⁵R¹⁶)_{q}―(CH₂)ᵣ―G

steht,
- R¹²: für Alkyl, Alkoxy, Alkenyl, Alkenyloxy, jeweils gegebenenfalls durch Halogen, Alkyl, Alkenyl, Halogenalkyl oder Halogenalkenyl substituiertes Cycloalkyl, Cycloalkyloxy oder Cycloalkylalkyloxy oder für jeweils gegebenenfalls durch Nitro, Halogen, Alkyl, Alkoxy, Halogenalkyl oder Halogenalkoxy substituiertes Phenyl oder Naphthyl steht,
- R¹³: für Wasserstoff oder Alkyl steht,
- R¹⁴: für Alkyl, Halogenalkyl, jeweils gegebenenfalls durch Halogen, Alkyl, Alkenyl, Halogenalkyl oder Halogenalkenyl substituiertes Cycloalkyl oder Cycloalkylalkyl oder für jeweils gegebenenfalls durch Halogen, Alkyl, Alkoxy, Halogenalkyl oder Halogenalkoxy substituiertes Phenyl oder Phenylalkyl steht,
- p, q und r: unabhängig voneinander für 0, 1, 2 oder 3 stehen, wobei deren Summe kleiner 6 ist,
- R¹⁵ und R¹⁶: unabhängig voneinander für Wasserstoff oder Alkyl stehen,
- G: für Cyano, für einen gegebenenfalls durch Halogen, Alkyl oder Halogenalkyl und an der Verknüpfungsstelle gegebenenfalls durch den Rest R¹⁷ substituierten 5- oder 6-gliedrigen Heterocyclus mit 1 bis 3 gleichen oder verschiedenen Heteroatomen aus der Reihe Stickstoff, Sauerstoff und Schwefel oder eine der folgenden Gruppierungen

(a) -CO-R¹⁷

(b) -CO-OR¹⁸

(c) -CO-NR¹⁹R²⁰

(d) -CS-NR¹⁹R²⁰

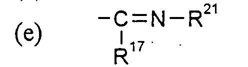

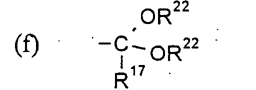

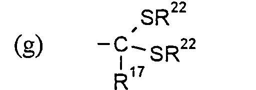

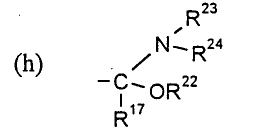

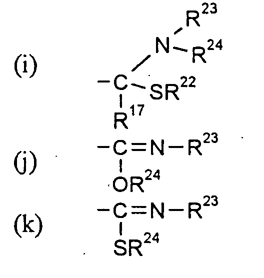

steht,
- R¹⁷: für Wasserstoff, Alkyl, Alkenyl, Halogenalkyl, Halogenalkenyl, gegebenenfalls durch Halogen, Alkyl oder Halogenalkyl substituiertes Cycloalkyl oder für gegebenenfalls einfach bis fünffach durch Alkylcarbonylamino, Alkylcarbonylalkylamino und/oder Reste aus der Liste W³ substituiertes Phenyl steht,
- R¹⁸: für Wasserstoff, Alkyl, Alkenyl, Halogenalkyl, Halogenalkenyl, jeweils gegebenenfalls durch Halogen, Alkyl oder Halogenalkyl substituiertes Cycloalkyl oder Cycloalkylalkyl oder für gegebenenfalls einfach bis fünffach durch Reste aus der Liste W³ substituiertes Arylalkyl steht,
- R¹⁹ und R²⁰: unabhängig voneinander für Wasserstoff, Alkyl, Alkenyl, Halogenalkyl, Halogenalkenyl, Alkoxy, jeweils gegebenenfalls durch Halogen, Alkyl oder Halogenalkyl substituiertes Cycloalkyl oder Cycloalkylalkyl, für jeweils gegebenenfalls einfach bis fünffach durch Reste aus der Liste W³ substituiertes Aryl oder Arylalkyl, für -OR¹⁸ oder -NR¹⁷R¹⁸ stehen oder gemeinsam für eine Alkylenkette mit 2 bis 6 Gliedern, in welcher gegebenenfalls eine Methylengruppe durch Sauerstoff ersetzt ist, stehen,
- R²¹: für -OR¹⁸, -NR¹⁷R¹⁸ oder -N(R¹⁷)-COOR¹⁸ steht,
- R²², R²³ und R²⁴: unabhängig voneinander für Alkyl stehen,
- W¹: für Wasserstoff, Halogen, Cyano, Formyl, Nitro, Alkyl, Trialkylsilyl, Alkoxy, Halogenalkyl, Halogenalkoxy, Halogenalkenyloxy, Alkylcarbonyl, Alkoxycarbonyl, Pentafluorthio oder -S(O)ₒR⁶ steht,
- W²: für Halogen, Cyano, Formyl, Nitro, Alkyl, Trialkylsilyl, Alkoxy, Halogenalkyl, Halogenalkoxy, Alkylcarbonyl, Alkoxycarbonyl, Pentafluorthio, -S(O)ₒR⁶ oder -C(R¹⁷)=N-R²¹ steht,
- W³: für Halogen, Cyano, Nitro, Alkyl, Alkoxy, Halogenalkyl, Halogenalkoxy, Dialkylamino, -S(O)ₒR⁶, -COOR²⁵ oder -CONR²⁶R²⁷ steht,
- R²⁵: für Wasserstoff, Alkyl, Halogenalkyl, gegebenenfalls durch Halogen, Alkyl oder Halogenalkyl substituiertes Cycloalkyl oder für gegebenenfalls einfach bis fünffach durch Reste aus der Liste W⁴ substituiertes Phenyl steht,
- R²⁶ und R²⁷: unabhängig voneinander für Wasserstoff, Alkyl, Alkenyl, Halogenalkyl, Halogenalkenyl, Alkoxy, jeweils gegebenenfalls durch Halogen, Alkyl oder Halogenalkyl substituiertes Cycloalkyl oder Cycloalkylalkyl oder für jeweils gegebenenfalls einfach bis fünffach durch Reste aus der Liste W⁴ substituiertes Aryl oder Arylalkyl, für -OR²² oder -NR²³R²⁴ stehen oder gemeinsam für eine Alkylenkette mit 2 bis 6 Gliedern, in welcher gegebenenfalls eine Methylengruppe durch Sauerstoff ersetzt ist, stehen und
- W⁴: für Halogen, Cyano, Nitro, Alkyl, Alkoxy, Halogenalkyl, Halogenalkoxy, Dialkylamino, Alkoxycarbonyl, Dialkylaminocarbonyl oder -S(O)ₒR⁶ steht.

Die Verbindungen der Formel (I) können, auch in Abhängigkeit von der Art der Substituenten, als geometrische und/oder optische Isomere oder Isomerengemische, in unterschiedlicher Zusammensetzung vorliegen, die gegebenenfalls in üblicher Art und Weise getrennt werden können. Sowohl die reinen Isomeren als auch die Isomerengemische, deren Herstellung und Verwendung sowie diese enthaltende Mittel sind Gegenstand der vorliegenden Erfindung. Im folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren Verbindungen gemeint sind.

Weiterhin wurde gefunden, daß man die neuen Verbindungen der Formel (I) nach einem der im folgenden beschriebenen Verfahren erhält.
A) Cyclische Imine der Formel (I) 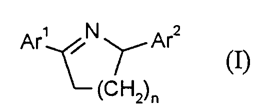 in welcher
   Ar¹, Ar² und n die oben angegebenen Bedeutungen haben,
   lassen sich herstellen, indem man Aminoketone der Formel (II) 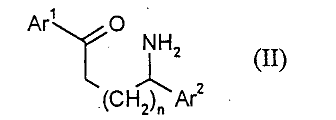 in welcher
   Ar¹, Ar² und n die oben angegebenen Bedeutungen haben,
   oder vorzugsweise deren sauren Salze gegebenenfalls in Gegenwart eines Säurebindemittels cyclokondensiert.
B) Cyclische Imine der Formel (I) lassen sich auch herstellen, indem man cyclische *O*-Methylsulfonyloxime der Formel (III) 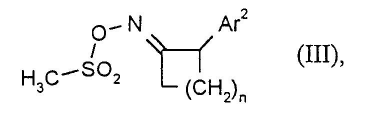 in welcher
   Ar² und n die oben angegebenen Bedeutungen haben,
   mit Aryl-Grignard-Verbindungen der Formel (IV)

   Ar¹―Mg―Hal (IV)

   in welcher
   Ar¹ die oben angegebene Bedeutung hat und
   Hal für Chlor, Brom oder Iod steht,
   in Gegenwart eines Verdünnungsmittels umsetzt.
C) Cyclische Imine der Formel (I-b) 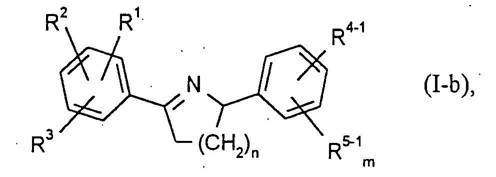 in welcher
   R¹, R², R³, n und m die oben angegebenen Bedeutungen haben,
   R⁴⁻¹ für A oder eine der folgenden Gruppierungen

   (m) -B―Z―D

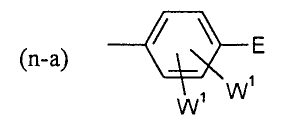

   steht, wobei
   A, B, D, E, W¹ und Z die oben angegebenen Bedeutungen haben und
   - R⁵⁻¹: .für Wasserstoff, Fluor, Cyano, Nitro, Alkyl, Alkoxy, Halogenalkyl, Halogenalkoxy, Alkoxyalkoxy oder -SR⁶ steht, wobei

   - R⁶: die oben angegebene Bedeutung hat,
   lassen sich herstellen, indem man Verbindungen der Formel (V) 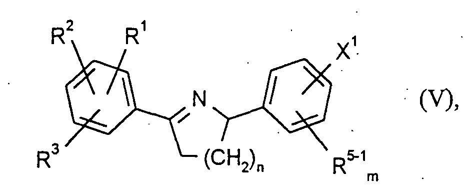 in welcher
   R¹, R², R³, R⁵⁻¹, n und m die oben angegebenen Bedeutungen haben und
   - X¹: für Brom, Iod oder -OSO₂CF₃ steht
   mit Boronsäuren der Formel (VI)

   R⁴⁻¹―B(OH)₂ (VI),

   in welcher
   - R⁴⁻¹: die oben angegebene Bedeutung hat,
   in Gegenwart eines Katalysators und in Gegenwart eines Säurebindemittels und in Gegenwart eines Lösungsmittels kuppelt.
D) Cyclische Imine der Formel (I-c) 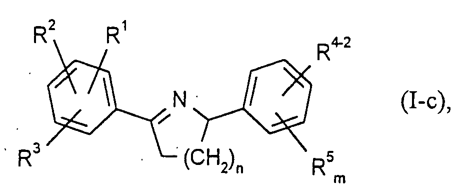 in welcher
   R¹, R², R³, R⁵, n und m die oben angegebenen Bedeutungen haben,
   - R⁴⁻²: für eine der folgenden Gruppierungen

   (m-b) -B―Z―D¹

   (n-b) -Y¹―E¹

   steht, in denen
   B und Z die oben angegebenen Bedeutungen haben,
   Y¹ für Sauerstoff oder Schwefel steht und
   D¹ und E¹ für die Gruppierung

   -(CH₂)ₚ―(CR¹⁵R¹⁶)_{q}―(CH₂)ᵣ―G

   stehen, in der
   R¹⁵, R¹⁶, G, p, q und r die oben angegebenen Bedeutungen haben,
   lassen sich herstellen, indem man cyclische Imine der Formel (I-d) 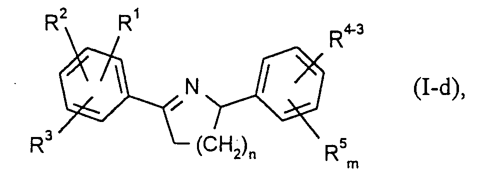 in welcher
   - R¹, R², R³, R³, n und m: die oben angegebenen Bedeutungen haben und
   - R⁴⁻³: für eine der folgenden Gruppierungen

   (m-c) -B―Z―H.

   (n-c) -Y¹―H

   steht, in denen
   B, Y¹ und Z die oben angegebenen Bedeutungen haben,
   mit Verbindungen der Formel (VII)

   Ab-(CH₂)ₚ―(CR¹⁵R¹⁶)_{q}―(CH₂)ᵣ―G (VII),

   in welcher
   - R¹⁵, R¹⁶, G, p, q und r: die oben angegebenen Bedeutungen haben und
   - Ab: für eine Abgangsgruppe steht,
   kondensiert.
E) Cyclische Imine der Formel (I-e) 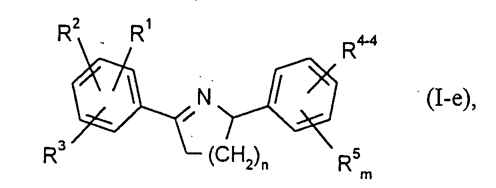
in welcher
- R¹, R², R³, R⁵, n und: m die oben angegebenen Bedeutungen haben und
- R⁴⁻⁴: für eine den Rest G enthaltenden Gruppierung aus der Beschreibung der erfindungsgemäßen Verbindungen der Formel (I) steht, wobei
G für eine der oben genannten Gruppierungen (e) bis (k) steht, lassen sich herstellen durch allgemein übliche und bekannte Derivatisierungen der entsprechenden Keto-Derivate, Carbonsäure-Derivate oder Nitrile, d.h. Verbindungen der Formel (I), in denen G für Cyano oder eine der Gruppierungen (a) bis (d) steht.

Weiterhin wurde gefunden, daß die neuen Verbindungen der Formel (I) bei guter Pflanzenverträglichkeit eine sehr gute Wirksamkeit als Schädlingsbekämpfungsmittel, insbesondere gegen Arthropoden in der Landwirtschaft aber auch gegen Parasiten in der Nutz- und Hobbytierhaltung aufweisen.

Die erfindungsgemäßen Verbindungen sind. durch die Formel (I) allgemein definiert. Bevorzugte Substituenten bzw. Bereiche der in den oben und nachstehend erwähnten Formeln aufgeführten Reste werden im folgenden erläutert.
- n: steht bevorzugt für 1, 2 oder 3.
- Ar¹: steht bevorzugt für den Rest 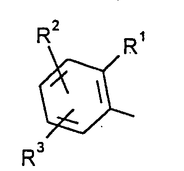
- Ar²: steht bevorzugt für den Rest 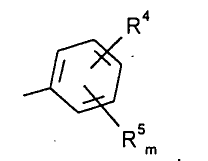
- m: steht bevorzugt für 0, 1, 2 oder 3.
- R¹: steht bevorzugt für Halogen, Cyano, Nitro, C₁-C₆-Alkyl, C₁-C₆-Alkoxy, C₁-C₆-Halogenalkyl oder C₁-C₆-Halogenalkoxy, für C₁-C₆-Alkoxy-C₁-C₆-alkyl, -S(O)ₒR⁶ oder -NR⁷R⁸.
- R² und R³: stehen unabhängig voneinander bevorzugt für Wasserstoff, Halogen, Cyano, Nitro, C₁-C₆-Alkyl, C₁-C₆-Alkoxy, C₁-C₆-Halogenalkyl oder C₁-C₆-Halogenalkoxy, für C₁-C₆-Alkoxy-C₁-C₆-alkyl, -S(O)ₒR⁶ oder -NR⁷R⁸.
- R⁴: steht bevorzugt für einen Substituenten in meta- oder para-Position aus der Reihe Halogen, Cyano, Tri-(C₁-C₆-alkyl)-silyl, -CO-NR¹⁰R¹¹, Tetrahydropyranyl oder eine der folgenden Gruppierungen

(l) -X―A

(m) -B―Z―D

(n) -Y―E .
- R⁵: steht bevorzugt für Wasserstoff, Halogen, Cyano, Nitro, C₁-C₁₆-Alkyl, C₁-C₁₆-Alkoxy, C₁-C₆-Halogenalkyl, C₁-C₆-Halogenalkoxy, C₁-C₈-Alkoxy-C₁-C₈-alkoxy oder -S(O)ₒR⁶.
- O: steht bevorzugt für 0, 1 oder 2.
- R⁶: steht bevorzugt für gegebenenfalls durch Fluor oder Chlor substituiertes C₁-C₆-Alkyl.
- R⁷ und R⁸: stehen unabhängig voneinander bevorzugt für Wasserstoff oder C₁-C₆-Alkyl, wie beispielsweise Methyl, Ethyl, n-Propyl, Isopropyl, n-Butyl, Isobutyl, sec.-Butyl, tert.-Butyl oder gemeinsam für C₂-C₅-Alkylen, wie beispielsweise -(CH₂)₄- oder -(CH₂)₅-.
- R¹⁰ und R¹¹: stehen unabhängig voneinander bevorzugt für Wasserstoff, C₁-C₆-Alkyl, C₁-C₆-Halogenalkyl oder für jeweils gegebenenfalls einfach bis dreifach durch Reste aus der Liste W¹ substituiertes Phenyl oder Phenyl-C₁-C₄-alkyl.
- X: steht bevorzugt für eine direkte Bindung, Sauerstoff, Schwefel, Carbonyl, Carbonyloxy, Oxycarbonyl, C₁-C₄-Alkylen, C₂-C₄-Alkenylen, C₂-C₄-Alkinylen, C₁-C₄-Alkylenoxy, C₁-C₄-Oxyalkylen, C₁-C₄-Thioalkylen, C₁-C₄-Alkylendioxy oder Di-C₁-C₄-alkylsilylen.
- A: steht bevorzugt für jeweils gegebenenfalls einfach bis vierfach durch Reste aus der Liste W¹ substituiertes Phenyl, Naphthyl oder Tetrahydronaphthyl oder für jeweils gegebenenfalls einfach bis vierfach durch Reste aus der Liste W² substituiertes 5- bis 10-gliedriges, 1 oder 2 aromatische Ringe enthaltendes Heterocyclyl mit 1 bis 4 Heteroatomen, welches 0 bis 4 Stickstoffatome, 0 bis 2 Sauerstoffatome und 0 bis 2 Schwefelatome enthält, (insbesondere Furyl, Benzofuryl, Thienyl, Benzothienyl, Oxazolyl, Benzoxazolyl, Thiazolyl, Benzthiazolyl, Pyrrolyl, Pyridyl, Pyrimidyl, 1,3,5-Triazinyl, Chinolinyl, Isochinolinyl, Indolyl, Purinyl, Benzodioxolyl, Indanyl, Benzodioxanyl oder Chromanyl).
- B: steht bevorzugt für gegebenenfalls einfach oder zweifach durch Reste aus der Liste W¹ substituiertes p-Phenylen.
- Z: steht bevorzugt für Sauerstoff oder Schwefel.
- D: steht bevorzugt für Wasserstoff, C₁-C₁₆-Alkyl, C₂-C₁₆-Alkenyl, C₂-C₆-Alkinyl, C₁-C₁₆-Halogenalkyl, C₂-C₁₆-Halogenalkenyl, jeweils gegebenenfalls durch Halogen, C₁-C₄-Alkyl, C₂-C₄-Alkenyl, C₂-C₄-Halogenalkenyl, Phenyl, Styryl, Halogenphenyl oder Halogenstyryl substituiertes C₃-C₈-Cycloalkyl oder C₃-C₈-Cycloalkyl-C₁-C₆-alkyl, für jeweils gegebenenfalls durch Halogen oder C₁-C₄-Alkyl substituiertes C₅-C₈-Cycloalkenyl oder C₅-C₈-Cycloalkenyl-C₁-C₄-alkyl, für jeweils gegebenenfalls durch Nitro, Halogen, C₁-C₆-Alkyl, C₁-C₆-Alkoxy, C₁-C₆-Halogenalkyl oder C₁-C₆-Halogenalkoxy substituiertes Phenyl-C₁-C₆-alkyl, Naphthyl-C₁-C₆-alkyl, Tetrahydronaphthyl-C₁-C₆-alkyl oder 5- oder 6-ringgliedriges Hetaryl-C₁-C₆-alkyl mit 1 oder 2 Heteroatomen aus der Reihe Stickstoff, Sauerstoff und Schwefel (insbesondere Furylmethyl, Thienylmethyl, Pyrrolylmethyl, Oxazolylmethyl, Isoxazolylmethyl, Thiazolylmethyl oder Pyridylmethyl), für -CO-R¹², -CO-NR¹³R¹⁴ oder für die Gruppierung

-(CH₂)ₚ―(CR¹⁵R¹⁶)_{q}―(CH₂)ᵣ―G
- Z und D: stehen auch bevorzugt gemeinsam für jeweils gegebenenfalls durch Nitro, Halogen, C₁-C₆-Alkyl, C₁-C₆-Alkoxy, C₁-H₆-Halogenalkyl oder C₁-C₆-Halogenalkoxy substituiertes Phenoxy-C₁-C₄-alkyl.
- Y: steht bevorzugt für eine direkte Bindung, Sauerstoff, Schwefel, Carbonyl, Carbonyloxy, Oxycarbonyl, C₁-C₄-Alkylen, C₂-C₄-Alkenylen, C₂-C₄-Alkinylen, C₁-C₄-Alkylenoxy, C₁-C₄-Oxyalkylen, C₁-C₄-Thioalkylen, C₁-C₄-Alkylendioxy oder für gegebenenfalls einfach oder zweifach durch Reste aus der Liste W¹ substituiertes p-Phenylen.
- E: steht bevorzugt für Wasserstoff, C₁-C₁₆-Alkyl, C₂-C₁₆-Alkenyl, C₂-C₆-Alkinyl, C₁-C₁₆-Halogenalkyl, C₂-C₁₆-Halogenalkenyl, gegebenenfalls durch Halogen, C₁-C₄-Alkyl, C₂-C₄-Alkenyl, C₂-C₄-Halogenalkenyl, Phenyl, Styryl, Halogenphenyl oder Halogenstyryl substituiertes C₃-C₈-Cycloalkyl, für gegebenenfalls durch Halogen oder C₁-C₄-Alkyl substituiertes C₅-C₈-Cycloalkenyl, für gegebenenfalls einfach bis vierfach durch Reste aus der Liste W¹ substituiertes Phenyl oder für jeweils gegebenenfalls einfach bis vierfach durch Reste aus der Liste W² substituiertes 5- oder 6-gliedriges Hetaryl mit 1 oder 2 Heteroatomen aus der Reihe Stickstoff, Sauerstoff und Schwefel (insbesondere Furyl, Thienyl, Pyrrolyl, Oxazolyl, Isoxazolyl, Thiazolyl oder Pyridyl) oder für die Gruppierung

-(CH₂)ₚ―(CR¹⁵R¹⁶)_{q}―(CH₂)ᵣ―G
- R¹²: steht bevorzugt für C₁-C₁₂-Alkyl, C₁-C₁₂-Alkoxy, C₂-C₁₂-Alkenyl, C₂-C₁₂-Alkenyloxy, jeweils gegebenenfalls durch Halogen, C₁-C₄-Alkyl, C₂-C₄-Alkenyl, C₁-C₄-Halogenalkyl oder C₂-C₄-Halogenalkenyl substituiertes C₃-C₈-Cycloalkyl, C₃-C₈-Cycloalkyloxy oder C₃-C₈-Cycloalkyl-C₁-C₆-alkyloxy oder für jeweils gegebenenfalls einfach bis vierfach durch Nitro, Halogen, C₁-C₁₂-Alkyl, C₁-C₁₂-Alkoxy, C₁-C₁₂-Halogenalkyl oder C₁-C₁₂-Halogenalkoxy substituiertes Phenyl oder Naphthyl.
- R¹³: steht bevorzugt für Wasserstoff oder C₁-C₁₂-Alkyl.
- R¹⁴: steht bevorzugt für C₁-C₁₂-Alkyl, C₁-C₁₂-Halogenalkyl, jeweils gegebenenfalls durch Halogen, C₁-C₄-Alkyl, C₂-C₄-Alkenyl, C₁-C₄-Halogenalkyl oder C₂-C₄-Halogenalkenyl substituiertes C₃-C₈-Cycloalkyl oder C₃-C₈-Cycloalkyl-C₁-C₆-alkyl oder für jeweils gegebenenfalls einfach bis vierfach durch Halogen, C₁-C₁₂-Alkyl, C₁-C₁₂-Alkoxy, C₁-C₁₂-Halogenalkyl oder C₁-C₁₂-Halogenalkoxy substituiertes Phenyl oder Phenyl-C₁-C₆-alkyl.
- p, q und r: stehen bevorzugt unabhängig voneinander für 0, 1, 2 oder 3, wobei deren Summe kleiner 6 ist.
- R¹⁵ und R¹⁶: stehen unabhängig voneinander bevorzugt für Wasserstoff oder C₁-C₄-Alkyl.
- G: steht bevorzugt für Cyano, für einen gegebenenfalls einfach bis dreifach durch Halogen, C₁-C₄-Alkyl oder C₁-C₄-Halogenalkyl und gegebenenfalls an der Verknüpfüngsstelle durch den Rest R¹⁷ substituierten 5- oder 6- gliedrigen Heterocyclus mit 1 bis 3 gleichen oder verschiedenen Heteroatomen aus der Reihe Stickstoff, Sauerstoff und Schwefel (insbesondere 5,6-Dihydrodioxazin-2-yl, 3-Pyridyl, 3-Furyl, 3-Thienyl, 2-Thiazolyl, 5-Thiazolyl, 2-Dioxolanyl, 1,3-Dioxan-2-yl, 2-Dithiolanyl, 1,3-Dithian-2-yl oder 1,3-Thioxan-2-yl) oder eine der folgenden Gruppierungen:

(a) -CO-R¹⁷

(b) -CO-OR¹⁸

(c) -CO-NR¹⁹R²⁰

(d) -CS-NR¹⁹R²⁰ 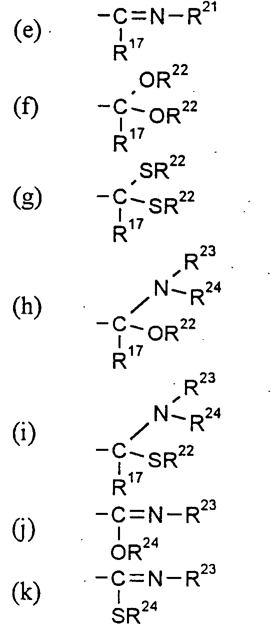
- R¹⁷: steht bevorzugt für Wasserstoff, C₁-C₆-Alkyl, C₂-C₆-Alkenyl, C₁-C₄-Halogenalkyl, C₂-C₆-Halogenalkenyl, gegebenenfalls durch Halogen, C₁-C₄-Alkyl oder C₁-C₄-Halogenalkyl substituiertes C₃-C₆-Cycloalkyl oder für gegebenenfalls einfach bis fünffach durch C₁-C₄-Alkylcarbonylamino, C₁-C₄-Alkylcarbonyl-C₁-C₄-alkylamino und/oder Reste aus der Liste W³ substituiertes Phenyl.
- R₁₈: steht bevorzugt für Wasserstoff, C₁-C₄-Alkyl, C₂-C₆-Alkenyl, C₁-C₄-Halogenalkyl, C₂-C₆-Halogenalkenyl, jeweils gegebenenfalls durch Halogen, C₁-C₄-Alkyl oder C₁-C₄-Halogenalkyl substituiertes C₃-C₆-Cycloalkyl oder C₃-C₆-Cycloalkyl-C₁-C₄-alkyl oder für gegebenenfalls einfach bis vierfach durch Reste aus der Liste W³ substituiertes C₆-C₁₀-Aryl-C₁-C₄-alkyl (insbesondere Phenyl-C₁-C₄-alkyl oder Naphthyl-C₁-C₄-alkyl).
- R¹⁹ und R²⁰: stehen unabhängig voneinander bevorzugt für Wasserstoff, C₁-C₄-Alkyl, C₃-C₆-Alkenyl, C₁-C₄-Halogenalkyl, C₃-C₆-Halogenalkenyl, C₁-C₄-Alkoxy, jeweils gegebenenfalls durch Halogen, C₁-C₄-Alkyl oder C₁-C₄-Halogenalkyl substituiertes C₃-C₆-Cycloalkyl oder C₃-C₆-Cycloalkyl-C₁-C₄-alkyl, für jeweils gegebenenfalls einfach bis fünffach durch Reste aus der Liste W³ substituiertes Phenyl oder Phenyl-C₁-C₄-alkyl, für -OR¹⁸ oder -NR¹⁷R¹⁸ oder gemeinsam für eine Alkylenkette mit 4 bis 6 Gliedern, in welcher gegebenenfalls eine Methylengruppe durch Sauerstoff ersetzt ist.
- R²¹: steht bevorzugt für -OR¹⁸, -NR¹⁷R¹⁸ oder -N(R¹⁷)-COOR¹⁸.
- R²², R²³ und R²⁴: stehen unabhängig voneinander bevorzugt für C₁-C₆-Alkyl.
- W¹: steht bevorzugt für Wasserstoff, Halogen, Cyano, Formyl, Nitro, C₁-C₆-Alkyl, Tri-C₁-C₄-alkylsilyl, C₁-C₁₆-Alkoxy, C₁-C₆-Halogenalkyl, C₁-C₆-Halogenalkoxy, C₂-C₆-Halogenalkenyloxy, C₁-C₆-Alkylcarbonyl, C₁-C₁₆-Alkoxycarbonyl, Pentafluorthio oder -S(O)ₒR⁶.
- W²: steht bevorzugt für Halogen, Cyano, Formyl, Nitro, C₁-C₆-Alkyl, Tri-C₁-C₄-alkylsilyl, C₁-C₁₆-Alkoxy, C₁-C₆-Halogenalkyl, C₁-C₆-Halogenalkoxy, C₁-C₆-Alkylcarbonyl, C₁-C₁₆-Alkoxycarbonyl, Pentafluorthio, -S(O)ₒR⁶ oder -C(R¹⁷)=N-R²¹.
- W³: steht bevorzugt für Halogen, Cyano, Nitro, C₁-C₄-Alkyl, C₁-C₄-Alkoxy, C₁-C₄-Halogenalkyl, C₁-C₄-Halogenalkoxy, Di-C₁-C₄-alkylamino, -S(O)ₒR⁶, -COOR²⁵ oder -CONR²⁶R²⁷.
- R²⁵: steht bevorzugt für Wasserstoff, C₁-C₄-Alkyl, C₁-C₄-Halogenalkyl, gegebenenfalls durch Halogen, C₁-C₄-Alkyl oder C₁-C₄-Halogenalkyl substituiertes C₃-C₇-Cycloalkyl oder für gegebenenfalls einfach bis fünffach durch Reste aus der Liste W⁴ substituiertes Phenyl.
- R²⁶ und R²⁷: stehen unabhängig voneinander bevorzugt für Wasserstoff, C₁-C₄-Alkyl, C₃-C₆-Alkenyl, C₁-C₄-Halogenalkyl, C₃-C₆-Halogenalkenyl, C₁-C₄-Alkoxy, jeweils gegebenenfalls durch Halogen, C₁-C₄-Alkyl oder C₁-C₄-Halogenalkyl substituiertes C₃-C₆-Cycloalkyl oder C₃-C₆-Cycloalkyl-C₁-C₄-alkyl oder für jeweils gegebenenfalls einfach bis fünffach durch Reste aus der Liste W⁴ substituiertes Phenyl oder Phenyl-C₁-C₄-alkyl, für -OR²² oder -NR²³R²⁴ oder gemeinsam für eine Alkylenkette mit 4 bis 6 Gliedern, in welcher gegebenenfalls eine Methylengruppe durch Sauerstoff ersetzt ist.
- W⁴: steht bevorzugt für Halogen, Cyano, Nitro, C₁-C₆-Alkyl, C₁-C₆-Alkoxy, C₁-C₆-Halogenalkyl, C₁-C₆-Halogenalkoxy, Di-C₁-C₄-alkylamino, C₁-C₆-Alkoxycarbonyl, Di-C₁-C₆-alkylaminocarbonyl oder -S(O)ₒR⁶.
- n: steht besonders bevorzugt für 1 oder 2.
- Ar¹: steht besonders bevorzugt für den Rest 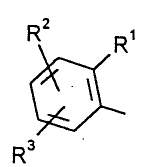
- Ar²: steht besonders bevorzugt für den Rest 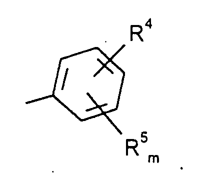
- m: steht besonders bevorzugt für 0, 1 oder 2.
- R¹: steht besonders bevorzugt für Fluor, Chlor, Brom, C₁-C₆-Alkyl, C₁-C₆-Alkoxy, jeweils durch Fluor oder Chlor substituiertes C₁-C₆-Alkyl oder C₁-C₆-Alkoxy, für C₁-C₆-Alkoxy-C₁-C₆-alkyl oder -S(O)ₒR⁶.
- R² und R³: stehen unabhängig voneinander besonders bevorzugt für Wasserstoff, Fluor, Chlor, Brom, Iod, C₁-C₆-Alkyl, C₁-C₆-Alkoxy, jeweils durch Fluor oder Chlor substituiertes C₁-C₆-Alkyl oder C₁-C₆-Alkoxy, für C₁-C₆-Alkoxy-C₁-C₆-alkyl oder -S(O)ₒR⁶.
- R⁴: steht besonders bevorzugt für einen Substituenten in meta- oder para-Position aus der Reihe Fluor, Chlor, Brom, Iod, Cyano, Tri-(C₁-C₄-alkyl)-silyl, -CO-NR¹⁰R¹¹, Tetrahydropyranyl oder eine der folgenden Gruppierungen

(l) -X―A

(m) -B―Z―D

(n) -Y―E .
- R⁵: steht besonders bevorzugt für Wasserstoff, Fluor, Chlor, Brom, Iod, Cyano, Nitro, C₁-C₁₆-Alkyl, C₁-C₁₆-Alkoxy, jeweils durch Fluor oder Chlor substituiertes C₁-C₆-Alkyl oder C₁-C₆-Alkoxy, für C₁-C₈-Alkoxy-C₁-C₈-alkoxy, oder -S(O)ₒR⁶.
- o: steht besonders bevorzugt für 0, 1 oder 2.
- R⁶: steht besonders bevorzugt für C₁-C₄-Alkyl oder jeweils durch Fluor oder Chlor substituiertes Methyl oder Ethyl.
- R¹⁰ und R¹¹: stehen unabhängig voneinander besonders bevorzugt für Wasserstoff, C₁-C₆-Alkyl, durch Fluor oder Chlor substituiertes C₁-C₆-Alkyl oder für jeweils gegebenenfalls einfach oder zweifach durch Reste aus der Liste W¹ substituiertes Phenyl oder Benzyl.
- X: steht besonders bevorzugt für eine direkte Bindung, Sauerstoff, Schwefel, Carbonyl, Carbonyloxy, Oxycarbonyl, C₁-C₄-Alkylen, C₂-C₄-Alkenylen, C₂-C₄-Alkinylen, C₁-C₄-Alkylenoxy, C₁-C₄-Oxyalkylen, C₁-C₄-Thioalkylen, C₁-C₄-Alkylendioxy oder Di-C₁-C₄-alkylsilylen.
- A: steht besonders bevorzugt für jeweils gegebenenfalls einfach bis dreifach durch Reste aus der Liste W¹ substituiertes Phenyl, Naphthyl oder Tetrahydronaphthyl oder für jeweils gegebenenfalls einfach bis dreifach durch Reste aus der Liste W² substituiertes 5- bis 10-gliedriges, 1 oder 2 aromatische Ringe enthaltendes Heterocyclyl mit 1 bis 4 Heteroatomen, welches 0 bis 4 Stickstoffatome, 0 bis 2 Sauerstoffatome und 0 bis 2 Schwefelatome enthält, (insbesondere Furyl, Benzofuryl, Thienyl, Benzothienyl, Oxazolyl, Benzoxazolyl, Thiazolyl, Benzthiazolyl, Pyrrolyl, Pyridyl, Pyrimidyl, 1,3,5-Triazinyl, Chinolinyl, Isochinolinyl, Indolyl, Purinyl, Benzodioxolyl, Indanyl, Benzodioxanyl oder Chromanyl).
- B: steht besonders bevorzugt für gegebenenfalls einfach oder zweifach durch Reste aus der Liste W¹ substituiertes p-Phenylen.
- Z: steht besonders bevorzugt für Sauerstoff oder Schwefel.
- D: steht besonders bevorzugt für Wasserstoff, C₁-C₁₆-Alkyl, C₂-C₁₆-Alkenyl, C₂-C₆-Alkinyl, jeweils durch Fluor oder Chlor substituiertes C₁-C₄-Alkyl oder C₂-C₄-Alkenyl, für jeweils gegebenenfalls durch Fluor, Chlor, Brom, C₁-C₄-Alkyl, C₂-C₄-Alkenyl, durch Fluor oder Chlor substituiertes C₂-C₄-Alkenyl, durch Phenyl, Styryl, jeweils durch Fluor, Chlor oder Brom substituiertes Phenyl oder Styryl substituiertes C₃-C₆-Cycloalkyl oder C₃-C₆-Cycloalkyl-C₁-C₄-alkyl, für jeweils gegebenenfalls durch Fluor, Chlor, Brom oder C₁-C₄-Alkyl substituiertes C₅-C₆-Cycloalkenyl oder C₅-C₆-Cycloalkenyl-C₁-C₄-alkyl, für jeweils gegebenenfalls durch Nitro, Fluor, Chlor, Brom, C₁-C₆-Alkyl, C₁-C₆-Alkoxy, jeweils durch Fluor oder Chlor substituiertes C₁-C₄-Alkyl oder C₁-C₄-Alkoxy substituiertes Phenyl-C₁-C₄-alkyl, Naphthyl-C₁-C₄-alkyl, Tetrahydronaphthyl-C₁-C₆-alkyl oder 5- oder 6-ringgliedriges Hetaryl-C₁-C₄-alkyl mit 1 oder 2 Heteroatomen aus der Reihe Stickstoff, Sauerstoff und Schwefel, insbesondere Furylmethyl, Thienylmethyl, Pyrrolylmethyl, Oxazolylmethyl, Isoxazolylmethyl, Thioazolylmethyl oder Pyridylmethyl) für -CO-R¹², -CO-NR¹³R¹⁴ oder die Gruppierung

-(CH₂)ₚ―(CR¹⁵R¹⁶)_{q}―(CH₂)ᵣ―G .
- Z und D: stehen auch besonders bevorzugt gemeinsam für gegebenenfalls durch Nitro, Fluor, Chlor, Brom, C₁-C₄-Alkyl, C₁-C₄-Alkoxy, jeweils durch Fluor oder Chlor substituiertes C₁-C₄-Alkyl oder C₁-C₄-Alkoxy substituiertes Phenoxy-C₁-C₃-alkyl.
- Y: steht besonders bevorzugt für eine direkte Bindung, Sauerstoff, Schwefel, Carbonyl, Carbonyloxy, Oxycarbonyl, C₁-C₄-Alkylen, C₂-C₄-Alkenylen, C₂-C₄-Alkinylen, C₁-C₄-Alkylenoxy, C₁-C₄-Oxyalkylen, C₁-C₄-Thioalkylen, C₁-C₄-Alkylendioxy oder für gegebenenfalls einfach oder zweifach durch Reste aus der Liste W¹ substituiertes p-Phenylen.
- E: steht besonders bevorzugt für Wasserstoff, C₁-C₁₆-Alkyl, C₂-C₁₆-Alkenyl, C₂-C₆-Alkinyl, jeweils durch Fluor oder Chlor substituiertes C₁-C₄-Alkyl oder C₂-C₄-Alkenyl, für gegebenenfalls durch Fluor, Chlor, Brom, C₁-C₄-Alkyl, C₂-C₄-Alkenyl, durch Fluor oder Chlor substituiertes C₂-C₄-Alkenyl, durch Phenyl, Styryl oder jeweils durch Fluor, Chlor oder Brom substituier-tes Phenyl oder Styryl substituiertes C₃-C₆-Cycloalkyl, für gegebenenfalls durch Fluor, Chlor, Brom oder C₁-C₄-Alkyl substituiertes C₅-C₆-Cycloalkenyl, für gegebenenfalls einfach bis dreifach durch Reste aus der Liste W¹ substituiertes Phenyl oder für jeweils gegebenenfalls einfach oder zweifach durch Reste aus der Liste W² substituiertes 5- oder 6-gliedriges Hetaryl mit 1 oder 2 Heteroatomen aus der Reihe Stickstoff, Sauerstoff und Schwefel (insbesondere. Furyl, Thienyl, Pyrrolyl, Oxazolyl, Isoxazolyl, Thiazolyl oder Pyridyl) oder für die Gruppierung

-(CH₂)ₚ―(CR¹⁵R¹⁶)_{q}―(CH₂)ᵣ―G .
- R¹²: steht besonders bevorzugt für C₁-C₆-Alkyl, C₁-C₆-Alkoxy, C₂-C₆-Alkenyl, C₂-C₆-Alkenyloxy, jeweils gegebenenfalls durch Fluor, Chlor, C₁-C₃-Alkyl oder jeweils durch Fluor oder Chlor substituiertes C₁-C₂-Alkyl oder C₂-C₃-Alkenyl substituiertes C₃-C₆-Cycloalkyl, C₃-C₆-Cycloalkyloxy oder C₃-C₆-Cycloalkyl-C₁-C₂-alkyloxy oder für gegebenenfalls einfach oder zweifach durch Fluor, Chlor, Brom, Iod, C₁-C₄-Alkyl, C₁-C₄-Alkoxy oder jeweils durch Fluor oder Chlor substituiertes C₁-C₃-Alkyl oder C₁-C₄-Alkoxy substituiertes Phenyl.
- R¹³: steht besonders bevorzugt für Wasserstoff oder C₁-C₄-Alkyl.
- R¹⁴: steht besonders bevorzugt für C₁-C₄-Alkyl oder für jeweils gegebenenfalls einfach oder zweifach durch Fluor, Chlor, Brom, C₁-C₄-Alkyl oder jeweils durch Fluor oder Chlor substituiertes C₁-C₄-Alkyl oder C₁-C₄-Alkoxy substituiertes Phenyl oder Benzyl.
- p, q und r: stehen besonders bevorzugt unabhängig voneinander für 0, 1, 2 oder 3, wobei deren Summe kleiner 6 ist.
- R¹⁵ und R¹⁶: stehen unabhängig voneinander besonders bevorzugt für Wasserstoff oder C₁-C₄-Alkyl.
- G: steht besonders bevorzugt für Cyano, für einen gegebenenfalls einfach bis dreifach durch Fluor, Chlor, Brom, C₁-C₄-Alkyl oder durch Fluor oder Chlor substituiertes C₁-C₄-Alkyl und gegebenenfalls an der Verknüpfungsstelle durch den Rest R¹⁷ substituierten 5- oder 6-gliedrigen Heterocyclus mit 1 bis 3 gleichen oder verschiedenen Heteroatomen aus der Reihe Stickstoff, Sauerstoff und Schwefel (insbesondere 5,6-Dihydrodioxazin-2-yl, 3-Pyridyl, 3-Furyl, 3-Thienyl, 2-Thiazolyl, 5-Thiazolyl, 2-Dioxolanyl, 1,3-Dioxan-2-yl, 2-Dithiolanyl, 1,3-Dithian-2-yl oder 1,3-Thioxan-2-yl) oder eine der folgenden Gruppierungen:

(a) -CO-R¹⁷

(b) -CO-OR¹⁸

(c) -CO-NR¹⁹R²⁰

(d) -CS-NR¹⁹R²⁰ 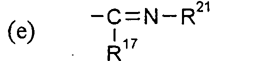 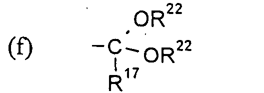 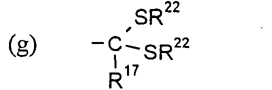 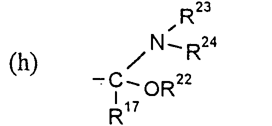 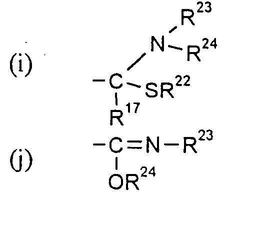 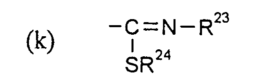
- R¹⁷: steht besonders bevorzugt für Wasserstoff, C₁-C₆-Alkyl, C₂-C₆-Alkenyl, jeweils durch Fluor oder Chlor substituiertes C₁-C₄-Alkyl oder C₂-C₆-Alkenyl, gegebenenfalls durch Fluor, Chlor, C₁-C₄-Alkyl oder durch Fluor oder Chlor substituiertes C₁-C₄-Alkyl substituiertes C₃-C₆-Cycloalkyl oder für gegebenenfalls einfach bis dreifach durch C₁-C₄-Alkylcarbonylamino, C₁-C₄-Alkylcarbonyl-C₁-C₄-alkylamino und/oder Reste aus der Liste W³ substituiertes Phenyl.
- R¹⁸: steht besonders bevorzugt für Wasserstoff, C₁-C₄-Alkyl, C₃-C₆-Alkenyl, jeweils durch Fluor oder Chlor substituiertes C₁-C₄-Alkyl oder C₃-C₆-Alkenyl, jeweils gegebenenfalls durch Fluor, Chlor, C₁-C₄-Alkyl oder durch Fluor oder Chlor substituiertes C₁-C₄-Alkyl substituiertes C₃-C₆-Cycloalkyl oder C₃-C₆-Cycloalkyl-C₁-C₄-alkyl oder für jeweils gegebenenfalls einfach bis dreifach durch Reste aus der Liste W³ substituiertes Phenyl-C₁-C₄-alkyl oder Naphthyl-C₁-C₄-alkyl.
- R¹⁹ und R²⁰: stehen unabhängig voneinander besonders bevorzugt für Wasserstoff, C₁-C₄-Alkyl, C₃-C₆-Alkenyl, jeweils durch Fluor oder Chlor substituiertes C₁-C₄-Alkyl oder C₃-C₆-Alkenyl, für C₁-C₄-Alkoxy, jeweils gegebenenfalls durch Fluor, Chlor, C₁-C₄-Alkyl oder durch Fluor oder Chlor substituiertes C₁-C₄-Alkyl substituiertes C₃-C₆-Cycloalkyl oder C₃-C₆-Cycloalkyl-C₁-C₄-alkyl, für jeweils gegebenenfalls einfach bis dreifach durch Reste aus der Liste W³ substituiertes Phenyl oder Phenyl-C₁-C₄-alkyl, für -OR¹⁸ oder -NR¹⁷R¹⁸ oder gemeinsam für -(CH₂)₅-, -(CH₂)₆- oder -(CH₂)₂-O-(CH₂)₂-.
- R²¹: steht besonders bevorzugt für -OR¹⁸, -NR¹⁷R¹⁸ oder -N(R¹⁷)-COOR¹⁸.
- R²², R²³ und R²⁴: stehen unabhängig voneinander besonders bevorzugt für C₁-C₄-Alkyl.
- W¹: steht besonders bevorzugt für Wasserstoff, Fluor, Chlor, Brom, Iod, Cyano, Formyl, Nitro, C₁-C₄-Alkyl, C₁-C₄-Alkoxy, jeweils durch Fluor oder Chlor substituiertes C₁-C₄-Alkyl oder C₁-C₄-Alkoxy, für C₁-C₄-Alkylcarbonyl, C₁-C₄-Alkoxycarbonyl oder -S(O)ₒR⁶.
- W²: steht besonders bevorzugt für Fluor, Chlor, Brom, Cyano, Formyl, Nitro, C₁-C₄-Alkyl, C₁-C₄-Alkoxy, jeweils durch Fluor oder Chlor substituiertes C₁-C₄-Alkyl oder C₁-C₄-Alkoxy, für C₁-C₄-Alkylcarbonyl, C₁-C₄-Alkoxycarbonyl, -S(O)ₒR⁶ oder -C(R¹⁷)=N-R²¹.
- W³: steht besonders bevorzugt für Fluor, Chlor, Brom, Cyano, Nitro, C₁-C₄-Alkyl, C₁-C₄-Alkoxy, jeweils durch Fluor oder Chlor substituiertes C₁-C₄-Alkyl oder C₁-C₄-Alkoxy, für Di-C₁-C₄-alkylamino, -S(O)ₒR⁶, -COOR²⁵ oder -CONR²⁶R²⁷.
- R²³: steht besonders bevorzugt für Wasserstoff, C₁-C₄-Alkyl, durch Fluor oder Chlor substituiertes C₁-C₄-Alkyl, gegebenenfalls durch Fluor, Chlor, C₁-C₄-Alkyl oder durch Fluor oder Chlor substituiertes C₁-C₄-Alkyl substituiertes C₃-C₆-Cycloalkyl oder für gegebenenfalls einfach bis dreifach durch Reste aus der Liste W⁴ substituiertes Phenyl.
- R²⁶ und R²⁷: stehen unabhängig voneinander besonders bevorzugt für Wasserstoff, C₁-C₄-Alkyl, C₃-C₆-Alkenyl, jeweils durch Fluor oder Chlor substituiertes C₁-C₄-Alkyl oder C₃-C₆-Alkenyl, für C₁-C₄-Alkoxy, jeweils gegebenenfalls durch Fluor, Chlor, C₁-C₄-Alkyl oder durch Fluor oder Chlor substituiertes C₁-C₄-Alkyl substituiertes C₃-C₆-Cycloalkyl oder C₃-C₆-Cycloalkyl-C₁-C₄-alkyl oder für jeweils gegebenenfalls einfach bis dreifach durch Reste aus der Liste W⁴ substituiertes Phenyl oder Phenyl-C₁-C₄-alkyl, für -OR²² oder -NR²³R²⁴ oder gemeinsam für -(CH₂)₅-, -(CH₂)₆- oder -(CH₂)₂-O-(CH₂)₂-.
- W⁴: steht besonders bevorzugt für Fluor, Chlor, Brom, Cyano, Nitro, C₁-C₄-Alkyl, C₁-C₄-Alkoxy, jeweils durch Fluor oder Chlor substituiertes C₁-C₄-Alkyl oder C₁-C₄-Alkoxy, Di-C₁-C₄-alkylamino, C₁-C₄-Alkoxycarbonyl, Di-C₁-C₆-alkylaminocarbonyl oder -S(O)ₒR⁶.
- n: steht ganz besonders bevorzugt für 1 oder 2, hervorgehoben für 1.
- Ar¹: steht ganz besonders bevorzugt für den Rest 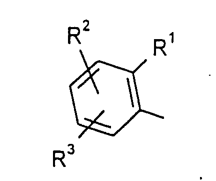
- Ar²: steht ganz besonders bevorzugt für den Rest 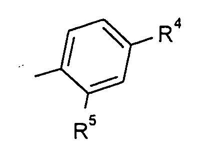
- R¹: steht ganz besonders bevorzugt für Fluor, Chlor, Brom, Methyl, Ethyl, n-Propyl, Isopropyl, n-Butyl, Isobutyl, sec.-Butyl, tert.-Butyl, Methoxy, Ethoxy, n-Propoxy, Isopropoxy, n-Butoxy, Isobutoxy, sec.-Butoxy, tert.-Butoxy.
- R² und R³: stehen unabhängig voneinander ganz besonders bevorzugt für Wasserstoff, Fluor, Chlor, Brom, Methyl, Ethyl, n-Propyl, Isopropyl, n-Butyl, Isobutyl, sec.-Butyl, tert.-Butyl, Methoxy, Ethoxy, n-Propoxy, Isopropoxy, n-Butoxy, Isobutoxy, sec.-Butoxy, tert.-Butoxy.
- R⁴: steht ganz besonders bevorzugt für einen Substituenten in meta- oder para-Position aus der Reihe Fluor, Chlor, Brom, Iod, Cyano, -CO-NR¹⁰R¹¹, Tetrahydropyranyl oder eine der folgenden Gruppierungen

(l) -X―A

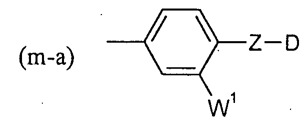

(n) -Y―E .
- R⁵: steht ganz besonders bevorzugt für Wasserstoff, Fluor, Chlor, Brom, Methyl, Ethyl, Methoxy, Ethoxy, Methylthio, Ethylthio, Trifluormethyl, Difluormethoxy, Trifluormethoxy oder Trifluormethylthio.
- o: steht ganz besonders bevorzugt für 0 oder 2.
- R⁶: steht ganz besonders bevorzugt für Methyl, Ethyl, n-Propyl, Isopropyl, Difluormethyl oder Trifluormethyl.
- R¹⁰ und R¹¹: stehen unabhängig voneinander ganz besonders bevorzugt für Wasserstoff, Methyl, Ethyl, n-Propyl, Isopropyl, n-Butyl, Isobutyl, sec.-Butyl, tert.-Butyl oder für jeweils gegebenenfalls einfach durch einen Rest aus der Liste W¹ substituiertes Phenyl oder Benzyl.
- X: steht ganz besonders bevorzugt für eine direkte Bindung, Sauerstoff, Schwefel, Carbonyl, -CH₂-, -(CH₂)₂-, -CH=CH- (E oder Z), -C≡C-, -CH₂O-, -(CH₂)₂O-, -CH(CH₃)O-, -OCH₂-, -O(CH₂)₂-, -SCH₂-, -S(CH₂)₂-, -SCH(CH₃)-, C₁-C₄-Alkylendioxy, insbesondere -OCH₂O-, -O(CH₂)₂O- oder -OCH(CH₃)O-.
- A: steht ganz besonders bevorzugt für gegebenenfalls einfach oder zweifach durch Reste aus der Liste W¹ substituiertes Phenyl oder für jeweils gegebenenfalls einfach oder zweifach durch Reste aus der Liste W² substituiertes Furyl, Benzofuryl, Thienyl, Benzothienyl, Oxazolyl, Benzoxazolyl, Thiazolyl, Benzthiazolyl, Pyrrolyl, Pyridyl, Pyrimidyl, 1,3,5-Triazinyl, Chinolinyl, Isochinolinyl, Indolyl, Purinyl, Benzodioxolyl, Indanyl, Benzodioxanyl oder Chromanyl.
- Z: steht ganz besonders bevorzugt für Sauerstoff oder Schwefel.
- D: steht ganz besonders bevorzugt für Wasserstoff, Methyl, Ethyl, n-Propyl, Isopropyl, n-Butyl, Isobutyl, sec.-Butyl, tert.-Butyl, die isomeren Pentyle, die isomeren Hexyle, n-Heptyl, n-Octyl, n-Isooctyl, n-Nonyl, n-Decyl, n-Undecyl, n-Dodecyl, n-Tridecyl, n-Tetradecyl, n-Pentadecyl, n-Hexadecyl, 2-Propenyl, Butenyl, Pentenyl, Hexenyl, Propargyl, Butinyl, Pentinyl, -CF₃, -CHF₂, -CClF₂, -CF₂CHFCl, -CF₂CH₂F, -CF₂CHF₂, -CF₂CCl₃, -CH₂CF₃, -CF₂CHFCF₃, -CH₂CF_{z}CHF₂, -CH₂CF₂CF₃, für jeweils gegebenenfalls einfach bis dreifach durch Fluor, Chlor, Brom, Methyl, Ethyl, n-Propyl, Isopropyl, n-Butyl, Isobutyl, sec.-Butyl, tert.-Butyl, Ethenyl, 1-Propenyl, 2,2-Dimethylethenyl, -CH=CCl₂, Phenyl, Styryl, jeweils durch Fluor, Chlor oder Brom substituiertes Phenyl oder 4-Chlorstyryl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl, für jeweils gegebenenfalls durch Fluor, Chlor, Methyl, Ethyl, n-Propyl, Isopropyl, n-Butyl, Isobutyl, sec.-Butyl oder tert.-Butyl substituiertes Cyclopentenyl, Cyclohexenyl, Cyclohexenylmethyl oder Cyclopentenylmethyl, für jeweils gegebenenfalls einfach oder zweifach durch Nitro, Fluor, Chlor, Brom, Methyl, Ethyl, n-Propyl, Isopropyl, n-Butyl, Isobutyl, sec.-Butyl, tert.-Butyl, Methoxy, Ethoxy, n-Propoxy, Isopropoxy, n-Butoxy, Isobutoxy, sec.-Butoxy, tert.-Butoxy, Trifluormethyl, Trifluormethoxy, Difluormethoxy oder Chlordifluormethoxy substituiertes Benzyl, Phenethyl, Naphthylmethyl, Tetrahydronaphthylmethyl, Furylmethyl, Thienylmethyl, Pyrrolylmethyl, Oxazolylmethyl, Isoxazolylmethyl, Thiazolylmethyl oder Pyridylmethyl, für -CO-R¹², -CO-NR¹³R¹⁴ oder die Gruppierung

-(CH₂)ₚ―(CR¹⁵R¹⁶)_{q}―(CH₂)ᵣ―G
- Z und D: stehen auch ganz besonders bevorzugt gemeinsam für gegebenenfalls einfach oder zweifach durch Nitro, Fluor, Chlor, Brom, Methyl, Ethyl, n-Propyl, i-Propyl, Methoxy, Ethoxy, n-Propoxy, i-Propoxy, Trifluormethyl, Trifluormethoxy, Difluormethoxy oder Chlordifluormethoxy substituiertes Phenoxymethyl.
- Y: steht ganz besonders bevorzugt für eine direkte Bindung, Sauerstoff, Schwefel, Carbonyl, -CH₂-, -(CH₂)₂-, -CH=CH- (E oder Z), -C≡C-, -CH₂O-, -(CH₂)₂O-, -CH(CH₃)O-, -OCH₂-, -O(CH₂)₂-, -SCH₂-, -S(CH₂)₂-, -SCH(CH₃)-, C₁-C₄-Alkylendioxy, insbesondere -OCH₂O- oder -O(CH₂)₂O- oder für gegebenenfalls einfach durch einen Rest aus der Liste W¹ substituiertes p-Phenylen.
- E: steht ganz besonders bevorzugt für Wasserstoff, Methyl, Ethyl, n-Propyl, Isopropyl, n-Butyl, Isobutyl, sec.-Butyl, tert.-Butyl, die isomeren Pentyle, die isomeren Hexyle, n-Heptyl, n-Octyl, n-Isooctyl, n-Nonyl, n-Decyl, n-Undecyl, n-Dodecyl, n-Tridecyl, n-Tetradecyl, n-Pentadecyl, n-Hexadecyl, 2-Propenyl, Butenyl, Pentenyl, Hexenyl, Propargyl, Butinyl, Pentinyl, -CF₃, -CHF₂, -CClF₂, -CF₂CHFCl, -CF₂CH₂F, -CF₂CHF₂, -CF₂CCl₃, -CH₂CF₃, -CF₂CHFCF₃, -CH₂CF₂CHF₂, -CH₂CF₂CF₃, für jeweils gegebenenfalls einfach bis dreifach durch Fluor, Chlor, Brom, Methyl, Ethyl, n-Propyl, Isopropyl, n-Butyl, Isobutyl, sec.-Butyl, tert.-Butyl, Ethenyl, 1-Propenyl, 2,2-Dimethylethenyl, -CH=CCl₂, Phenyl, Styryl, jeweils durch Fluor, Chlor oder Brom substituiertes Phenyl oder durch 4-Chlorstyryl substituiertes Cyclopropyl, Cyclobutyl, Cyclopentyl oder Cyclohexyl, für jeweils gegebenenfalls durch Fluor, Chlor, Methyl, Ethyl, n-Propyl, Isopropyl, n-Butyl, Isobutyl, sec.-Butyl oder tert.-Butyl substituiertes Cyclopentenyl oder Cyclohexenyl, für gegebenenfalls einfach oder zweifach durch Reste aus der Liste W¹ substituiertes Phenyl, für jeweils gegebenenfalls einfach oder zweifach durch Reste aus der Liste W² substituiertes Furyl, Thienyl, Pyrrolyl, Oxazolyl, Isoxazolyl, Thiazolyl oder Pyridyl, oder für die Gruppierung

-(CH₂)ₚ―(CR¹⁵R¹⁶)q―(CH₂)ᵣ―G
- R¹²: steht ganz besonders bevorzugt für Methyl; Ethyl, n-Propyl, Isopropyl, n-Butyl, Isobutyl, sec.-Butyl, tert.-Butyl, Methoxy, Ethoxy, n-Propoxy, Isopropoxy, n-Butoxy, Isobutoxy, sec.-Butoxy, tert.-Butoxy, Cyclopropyl, Cyclohexyl, Cyclohexyloxy, Cyclohexylmethyloxy, Phenyl, 2-Chlorphenyl, 3-Chlorphenyl, 2,6-Difluorphenyl, 2,4-Dichlorphenyl, 3,4-Dichlorphenyl, 2-Trifluormethoxyphenyl oder 4-Trifluormethoxyphenyl.
- R¹³: steht ganz besonders bevorzugt für Wasserstoff.
- R¹⁴: steht ganz besonders bevorzugt für Methyl, Ethyl oder gegebenenfalls einfach durch Chlor substituiertes Phenyl.
- .p, q und r: stehen ganz besonders bevorzugt unabhängig voneinander für 0, 1, 2 oder 3, wobei deren Summe kleiner 4 ist.
- R¹⁵ und R¹⁶: stehen unabhängig voneinander ganz besonders bevorzugt für Wasserstoff, Methyl, Ethyl, n-Propyl, Isopropyl, n-Butyl, Isobutyl, sec.-Butyl, tert.-Butyl.
- G: steht ganz besonders bevorzugt für Cyano, für jeweils gegebenenfalls einfach bis dreifach durch Fluor, Chlor, Brom, Methyl, Ethyl, n-Propyl, Isopropyl oder Trifluormethyl und gegebenenfalls an der Verknüpfungsstelle durch den Rest R¹⁷ substituiertes 5,6-Dihydrodioxazin-2-yl, 3-Pyridyl, 3-Furyl, 3-Thienyl, 2-Thiazolyl, 5-Thiazolyl, 2-Dioxolanyl, 1,3-Dioxan-2-yl, 2-Dithiolanyl, 1,3-Dithian-2-yl oder 1,3-Thioxan-2-yl oder eine der folgenden Gruppierungen:

(a) -CO-R¹⁷

(b) -CO-OR¹⁸

(c) -CO-NR¹⁹R²⁰

(d) -CS-NR¹⁹R²⁰ 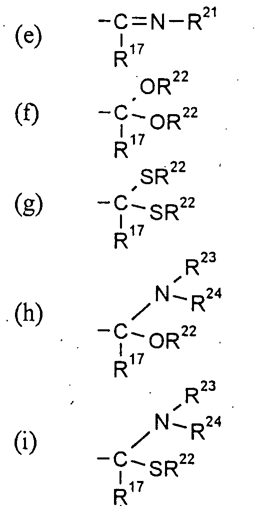
- R¹⁷: steht ganz besonders bevorzugt für Wasserstoff, Methyl, Ethyl, n-Propyl, Isopropyl, n-Butyl, Isobutyl, sec.-Butyl, tert.-Butyl, die isomeren Pentyle, die isomeren Hexyle -CF₃, -CHF₂, -CClF₂, -CF₂CHFCl, -CF₂CH₂F, -CF₂CHF₂, -CF₂CCl₃, -CH₂CF₃, C₃-C₆-Alkenyl, einfach bis dreifach durch Fluor oder Chlor substituiertes C₃-C₆-Alkenyl, für jeweils gegebenenfalls einfach oder zweifach durch Fluor, Chlor, Methyl, Ethyl, n-Propyl, Isopropyl, -CF₃, -CHF₂, -CClF₂, -CF₂CHFCl, -CF₂CH₂F, -CF₂CHF₂, -CF₂CCl₃ oder -CH₂CF₃ substituiertes Cyclopropyl, Cyclopentyl oder Cyclohexyl oder für gegebenenfalls einfach oder zweifach durch Methylcarbonylamino, Ethylcarbonylamino, Methylcarbonyl-methylamino und/oder Reste aus der Liste W³ substituiertes Phenyl.
- R¹⁸: steht ganz besonders bevorzugt für Wasserstoff, Methyl, Ethyl, n-Propyl, Isopropyl, n-Butyl, Isobutyl, sec.-Butyl, tert.-Butyl, -CH₂CF₃, Allyl, jeweils gegebenenfalls einfach oder zweifach durch Fluor, Chlor, Methyl, Ethyl, n-Propyl, Isopropyl -CF₃, -CHF₂, -CClF₂, -CF₂CHFCl, -CF₂CH₂F, -CF₂CHF₂, -CF₂CCl₃ oder -CH₂CF₃ substituiertes Cyclopropyl, Cyclopentyl, Cyclohexyl, Cyclopropylmethyl, Cyclopentylmethyl, Cyclohexylmethyl, Cyclopropylethyl, Cyclopentylethyl oder Cyclohexylethyl oder für jeweils gegebenenfalls einfach oder zweifach durch Reste aus der Liste W³ substituiertes Benzyl oder Phenethyl.
- R¹⁹ und R²⁰: stehen unabhängig voneinander ganz besonders bevorzugt für Wasserstoff, Methyl, Ethyl, n-Propyl, Isopropyl, n-Butyl, Isobutyl, sec.-Butyl, tert.-Butyl, -CH₂CF₃, Methoxy, Ethoxy, Allyl, für jeweils gegebenenfalls einfach oder zweifach durch Fluor, Chlor, Methyl, Ethyl, n-Propyl, Isopropyl oder Trifluormethyl substituiertes Cyclopropyl, Cyclopentyl, Cyclohexyl, Cyclopropylmethyl, Cyclopentylmethyl oder Cyclohexylmethyl, für jeweils gegebenenfalls einfach oder zweifach durch Reste aus der Liste W³ substituiertes Phenyl, Benzyl oder Phenethyl, für -OR¹⁸ oder -NR¹⁷R¹⁸.
- R²¹: steht ganz besonders bevorzugt für -OR¹⁸, -NR¹⁷R¹⁸ oder -N(R¹⁷)-COOR¹⁸.
- R²², R²³ und R²⁴: stehen unabhängig voneinander besonders bevorzugt für Methyl, Ethyl, n-Propyl oder Isopropyl.
- W¹: steht ganz besonders bevorzugt für Wasserstoff, Fluor, Chlor, Brom, Cyano, Formyl, Nitro, Methyl, Ethyl, n-Propyl, Isopropyl, n-Butyl, Isobutyl, sec.-Butyl, tert.-Butyl, Methoxy, Ethoxy, n-Propoxy, Isopropoxy, n-Butoxy, Isobutoxy, sec.-Butoxy, tert.-Butoxy, -CF₃, -CHF₂, -CClF₂, -CF₂CHFCl, -CF₂CH₂F, -CF₂CHF₂, -CF₂CCl₃, -CH₂CF₃, -CF₂CHFCF₃, -CH₂CF₂CHF₂, -CH₂CF₂CF₃, Trifluormethoxy, Difluormethoxy, Chlordifluormethoxy, Acetyl, Propionyl, Butyryl, Isobutyryl, Methoxycarbonyl, Ethoxycarbonyl, n-Propoxycarbonyl, Isopropoxycarbonyl, n-Butoxycarbonyl, Isobutoxycarbonyl, sec.-Butoxycarbonyl, tert.-Butoxycarbonyl oder -S(O)ₒR⁶.
- W²: steht ganz besonders bevorzugt für Fluor, Chlor, Brom, Cyano, Methyl, Ethyl, n-Propyl, Isopropyl, Trifluormethyl, Trifluormethoxy, Difluormethoxy, Chlordifluormethoxy, Acetyl, Trifluormethylthio, -CH=N-OCH₃, -CH=N-OC₂H₅, -CH=N-OC₃H₇, -C(CH₃)=N-OCH₃, -C(CH₃)=N-OC₂H₅, -C(CH₃)=N-OC₃H₇, -C(C₂H₅)=N-OCH₃,-C(C₂H₅)=N-OC₂H₅ oder -C(C₂H₅)=N-OC₃H₇.
- W³: steht ganz besonders bevorzugt für Fluor, Chlor, Cyano, Nitro, Methyl, Ethyl, Methoxy, Ethoxy, Methylthio, Trifluormethyl, Trifluormethoxy, Trifluormethylthio, Dimethylamino, Diethylamino, -COOR²⁵ oder -CONR²⁶R²⁷.
- R²⁵: steht ganz besonders bevorzugt für Wasserstoff, Methyl, Ethyl, n-Propyl, Isopropyl, tert.-Butyl, -CH₂CF₃, für jeweils gegebenenfalls einfach oder zweifach durch Fluor, Chlor, Methyl, Ethyl, n-Propyl, Isopropyl oder -CF₃ substituiertes Cyclopropyl, Cyclopentyl oder Cyclohexyl oder für gegebenenfalls einfach oder zweifach durch Reste aus der Liste W⁴ substituiertes Phenyl.
- R²⁶ und R²⁷: stehen unabhängig voneinander ganz besonders bevorzugt für Wasserstoff, Methyl, Ethyl, n-Propyl, Isopropyl, n-Butyl, Isobutyl, sec.-Butyl, tert.-Butyl, -CH₂CF₃, Methoxy, Ethoxy, Allyl, für jeweils gegebenenfalls einfach oder zweifach durch Fluor oder Chlor substituiertes Cyclopropyl, Cyclopentyl, Cyclohexyl, Cyclopropylmethyl, Cyclopentylmethyl oder Cyclohexylmethyl, für jeweils gegebenenfalls einfach oder zweifach durch Reste aus der Liste W⁴ substituiertes Phenyl, Benzyl oder Phenethyl, für -OR²² oder -NR²³R²⁴.
- W⁴: steht ganz besonders bevorzugt für Fluor, Chlor, Brom, Cyano, Nitro, Methyl, Ethyl, tert.-Butyl, Methoxy, Ethoxy, Methylthio, Trifluormethyl, Trifluormethoxy oder Trifluormethylthio.

Weiterhin bevorzugt sind Verbindungen der Formel (I-a) 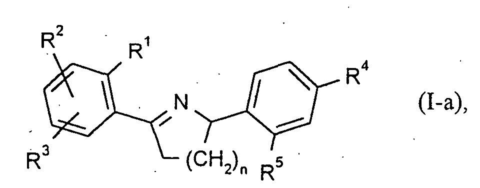 in welcher
- R¹, R², R³, R⁵ und n: die oben genannten allgemeinen, bevorzugten, besonders bevorzugten oder ganz besonders bevorzugten Bedeutungen haben,
- R⁴: für einfach oder zweifach durch Reste aus der Liste W¹ substituiertes Phenyl oder eine der folgenden Gruppierungen

(m-b) -B―O―D

(l) -Y―E

steht,
- B: für gegebenenfalls einfach durch einen Rest aus der Liste W¹ substituiertes p-Phenylen steht,
- Y: für eine direkte Bindung oder für gegebenenfalls einfach oder zweifach durch Reste aus der Liste W¹ substituiertes p-Phenylen steht und
- D und E: die oben genannten ganz besonders bevorzugten Bedeutungen haben, wobei
G für Cyano oder eine der folgenden Gruppierungen

(a) -CO-R¹⁷

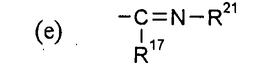

steht, in denen
R¹⁷ und R²¹ die oben genannten allgemeinen, bevorzugten, besonders bevorzugten oder ganz besonders bevorzugten Bedeutungen haben und
- W¹: die oben genannte allgemeine, bevorzugte, besonders bevorzugte oder ganz besonders bevorzugte Bedeutung hat.

Weiterhin bevorzugt sind Verbindungen der Formel (I-f) 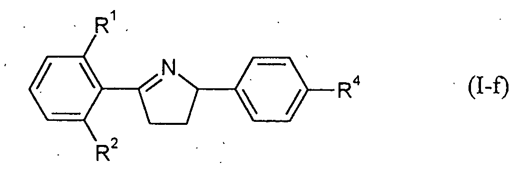 in welcher
- R¹: für Halogen, insbesondere Fluor oder Chlor, speziell Fluor steht,
- R²: für Halogen, insbesondere Fluor oder Chlor, speziell Fluor steht und
- R⁴: für
a) einfach oder zweifach durch Reste aus der Liste W² substituiertes Phenyl oder
b) einfach oder zweifach durch Reste aus der Liste W² substituiertes Hetaryl (insbesondere Furyl, Thienyl, Pyrrolyl, Oxazolyl, Isoxazolyl, Thiazolyl oder Pyridyl, speziell Thienyl) steht.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

Erfindungsgemäß ganz besonders bevorzugt werden die Verbindungen der Formel. (I), in welchen eine Kombination der vorstehend als ganz besonders bevorzugt aufgeführten Bedeutungen vorliegt.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können einfach oder mehrfach substituiert sein, wobei bei Mehrfachsubstitutiorien die Substituenten gleich oder verschieden sein können. Mehrere Reste mit denselben Indizes wie beispielsweise m Reste R⁵ für m > 1, können gleich oder verschieden sein.

Verwendet man beispielsweise [1-(4-Ethyl-2-methyl-phenyl)-5-(2-methylbenzoyl)-1-pentyl]-ammonium-trifluoracetat als Ausgangsstoff, so kann der Reaktionsablauf des erfindungsgemäßen Verfahrens (A) durch das folgende Formelschema wiedergegeben werden:

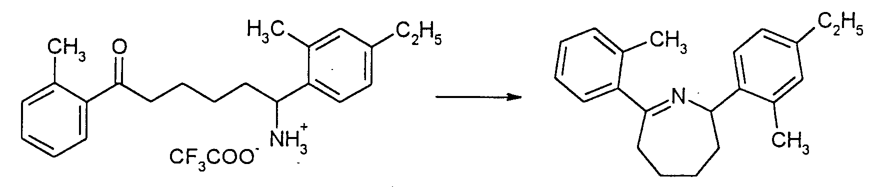

Verwendet man beispielsweise 2-(4-Methoxyphenyl)-cyclopentanon-*O*-methansulfonyloxim und 2-Tolylmagnesiumbromid als Ausgangsstoffe, so kann der Reaktionsablauf des erfindungsgemäßen Verfahrens (B) durch das folgende Formelschema wiedergegeben werden:

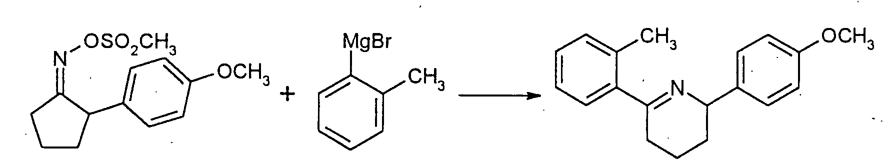

Verwendet man beispielsweise 2-(2-Methylphenyl)-5-(4-iodphenyl)-3,4-dihydro-2H-pyrrol und 4-Cyanomethoxyphenylboronsäure als Ausgangsstoffe, so kann der Reaktionsablauf des erfindungsgemäßen Verfahrens (C) durch das folgende Formelschema wiedergegeben werden:

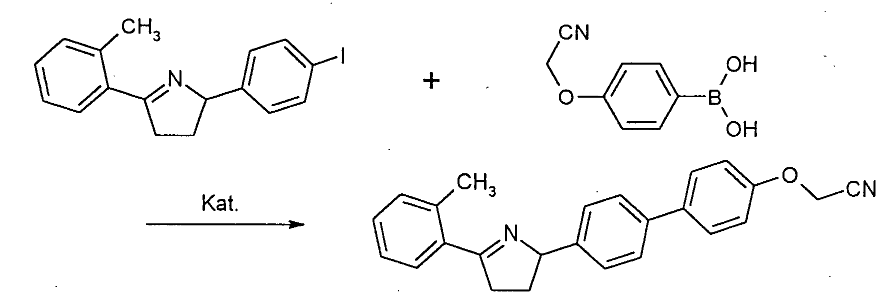

Verwendet man beispielsweise 2-(2-Brom-4-fluor-6-methyl-phenyl)-5-(3'-chlor-4'hydroxybiphenyl-4-yl)-3,4-dihydro-2H-pyrrol und α-Bromvaleriansäuremethylester als Ausgangsstoffe, so kann der Reaktionsablauf des erfindungsgemäßen Verfahrens (D) durch das folgende Formelschema wiedergegeben werden:

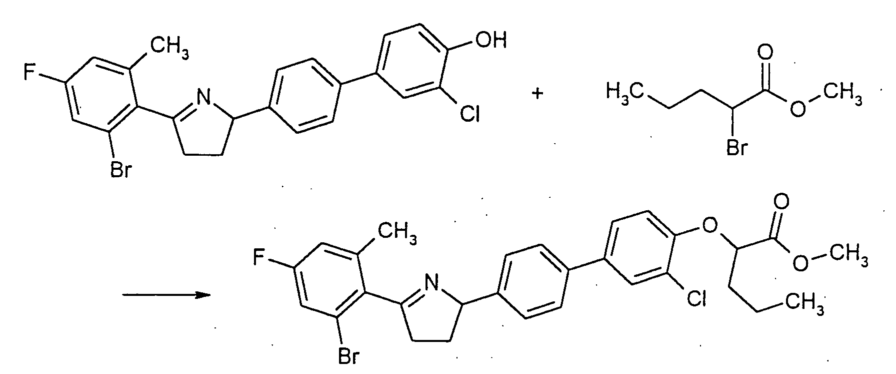

Verwendet man beispielsweise 6-(4'-Cyclopropylcarbonylmethoxy-3-trifluormethoxy-biphenyl-4-yl)-2-(2-methylphenyl)-3,4,5,6-tetrahydropyridin und *O*-Methylhydroxylamin als Ausgangsstoffe, so kann der Reaktionsablauf des erfindungsgemäßen Verfahrens (E) durch das folgende Formelschema wiedergegeben werden:

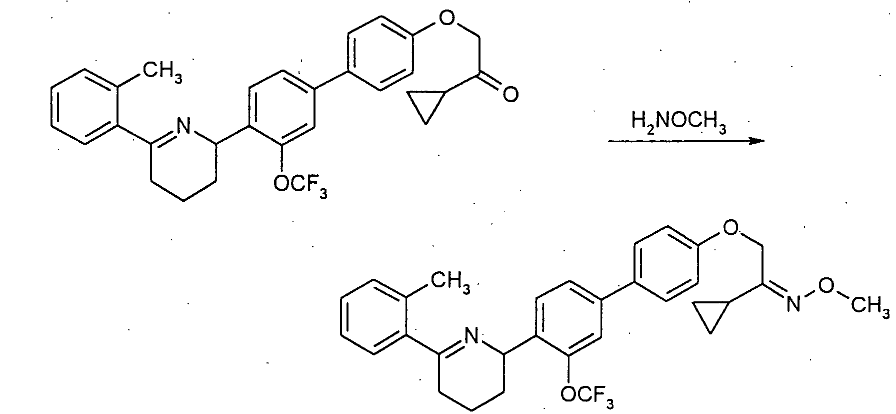

Die zur Durchführung des erfindungsgemäßen Verfahrens (A) benötigten Aminoketone sind durch die Formel (II) allgemein definiert. In dieser Formel haben Ar¹, Ar² und n vorzugsweise diejenigen Bedeutungen, die bereits im Zusammenhang mit der Beschreibung der cyclischen Imine der Formel (I) als bevorzugt genannt wurden. Die Aminoketone der Formel (II) sind neu.

Aminoketone der Formel (II) lassen sich z.B. herstellen, indem man in einem Verfahren (A.a) die BOC (tert.-Butoxycarbonyl)-Schutzgruppe der Aminoketon-Derivate der Formel (VIII), welche ebenfalls neu sind, gemäß dem folgendem Reaktionsschema abspaltet:

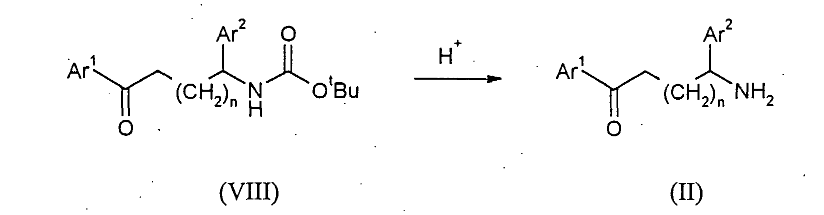

Die Reaktion läßt sich gegebenenfalls Gegenwart eines Lösungsmittels wie z.B. Dichlormethan mittels üblicher Methoden zur Abspaltung einer tert.-Butoxycarbonyl-Aminoschutzgruppe, vorzugsweise durch Acidolyse mit Trifluoressigsäure durchführen (vgl. z.B. T. W. Greene, P. G. M. Wuts, Protective Groups in Organic Synthesis, 2. Ed., John Wiley & Sons, New York 1991). Die Aminoketone der Formel (II) werden dabei bevorzugt als Salze einer organischen oder anorganischen Brønstedt-Säure wie beispielsweise Fluorwasserstoff, Chlorwasserstoff, Schwefelsäure, Phosphorsäure, Ameisensäure, Essigsäure, Benzoesäure, Citronensäure, Trifluoressigsäure, Methansulfonsäure, Trifluormethansulfonsäure oder Toluolsulfonsäure isoliert.

Aminoketon-Derivate der Formel (VIII) lassen sich z.B. herstellen, indem man BOC-geschützte Lactame der Formel (IX) mit metallierten Aromaten der Formel (X) bei Temperaturen zwischen 0°C und 80°C gemäß dem folgenden Reaktionsschema umsetzt:

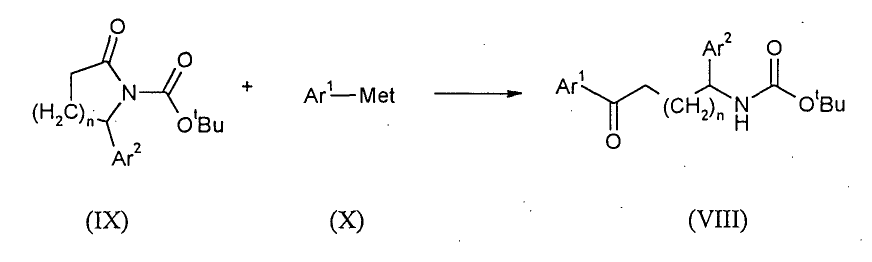

In der Formel (X) steht Met für einen einwertigen Metallrest wie Li, MgI, MgBr oder MgCl.

Metallierte Aromaten der Formel (X) sind zum Teil bekannt oder können nacl bekannten Methoden wie z.B. Lithiierung oder Grignard-Reaktion aus den ent sprechenden Aromaten oder Halogenaromaten hergestellt werden.

Geschützte Lactame der Formel (IX) erhält man beispielsweise, indem mar Lactame der Formel (XI) nach üblichen Methoden wie z.B. Metallierung mi Butyllithium und Umsetzung mit Di-tert.-butyldicarbonat BOC-schützt (vgl. z.B T. W. Greene, P. G. M. Wuts, Protective Groups in Organic Synthesis, 2. Ed. John Wiley & Sons, New York 1991).

Lactame der Formel (XI) lassen sich z.B. von ω-Alkoxylactamen der Formel (XII) ausgehend nach zwei Methoden herstellen. Man kann diese mit Aromaten der Formel (XIII) in Gegenwart eines sauren Katalysators, wie beispielsweise Schwefelsäure, Essigsäure oder Aluminiumchlorid und gegebenenfalls in Gegen wart eines Verdünnungsmittels, wie beispielsweise Dichlormethan oder Acetonitri gemäß dem folgenden Reaktionsschema umsetzen:

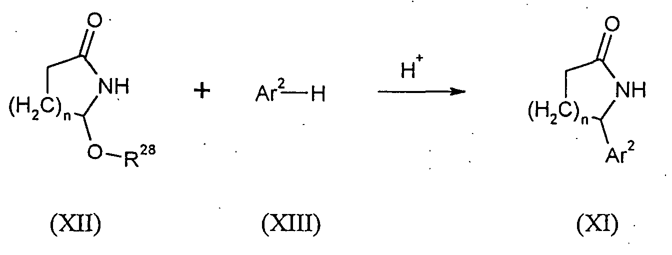

Alternativ kann man mit Aryl-Grignard-Verbindungen der Formel (XIV) in Gegenwart eines Verdünnungsmittels, wie beispielsweise Tetrahydrofuran gemäß dem folgenden Reaktionsschema umsetzen [vgl. Org. Prep. Proced. Int. 25, 255 (1993)]:

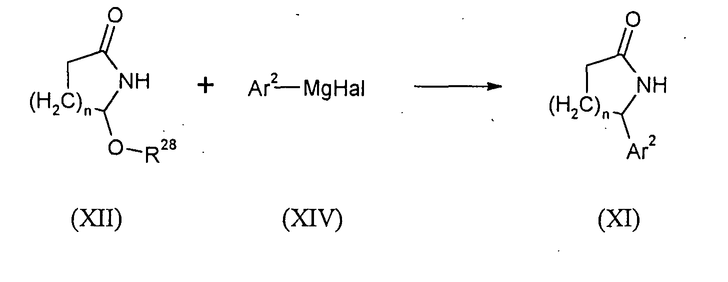

In Formel (XII) steht R²⁸ für Methyl oder Ethyl. In Formel (XIV) steht Hal für Chlor, Brom oder Iod.

Die ω-Alkoxylactame der Formel (XII) sind bekannt, z.T. kommerziell erhältlich und lassen sich z.B. aus den entsprechenden unsubstituierten Imiden durch kathodische oder Natriumboranat-Reduktion oder aus den unsubstituierten Lactamen durch anodische Oxidation, jeweils in Gegenwart von Methanol oder Ethanol herstellen (vgl. z.B. J. Org. Chem. **56,** 1822 (1991); Synthesis **1980,** 315).

Die Aromaten der Formel (XIII) sind Benzolderivate, die allgemein bekannt sind oder nach einer weiten Palette allgemein bekannter Methoden der organischen Chemie hergestellt werden können.

Die Aryl-Grignard-Verbindungen der Formel (XIV) können nach üblicher Weise aus den entsprechenden Arylhalogeniden und Magnesium hergestellt werden. Arylhalogenide sind allgemein bekannte Verbindungen der organischen Chemie.

Lactame der Formel (XI) lassen sich z.B. auch herstellen, indem man substituierte ω-Benzoylcarbonsäuren der Formel (XV) mit einem Reagenz, bereitet aus Ammoniumcarbonat und Ameisensäure bei Siedetemperatur gemäß folgendem Reaktionsschema cyclisiert [vgl. Recl. Trav. Chim. Bays-Bas 81, 788 (1962)]:

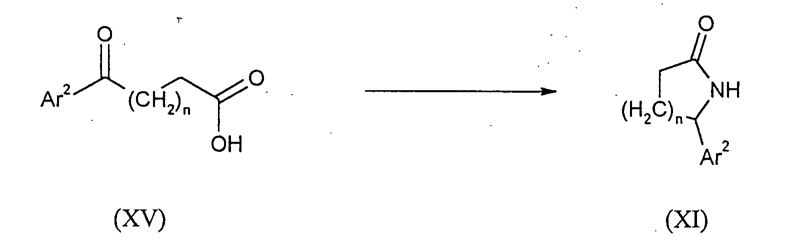

Die hierfür benötigten ω-Benzoylcarbonsäuren der Formel (XV) lassen sich z.B. herstellen, indem man die Dicarbonsäureanhydride der Formel (XVI) mit Aromaten der Formel (XIII) in Gegenwart einer Lewis-Säure wie beispielsweise Aluminiumchlorid und gegebenenfalls in Gegenwart eines Verdünnungsmittels wie beispielsweise Benzol gemäß dem folgenden Reaktionsschema umsetzt [vgl. Recl. Trav. Chim. Bays-Bas 81, 788 (1962)]:

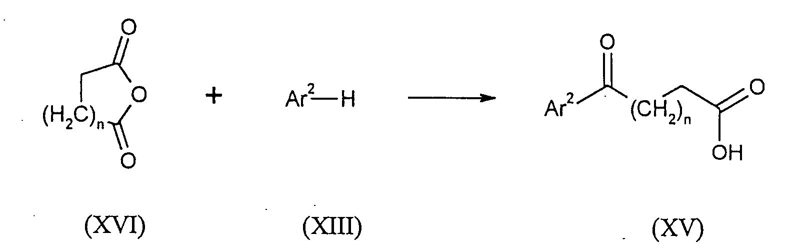

Die hierfür benötigten Anhydride sind (Bernsteinsäure- und Glutarsäureanhydrid) oder waren (Adipinsäureanhydrid) kommerziell erhältlich [Zur Herstellung von Adipinsäureanhydrid vgl. z.B. Chem. Ber. **120,** 285 (1987)].

Für den Fall, daß Ar² im erfindungsgemäßen Wirkstoff der Formel (I) wie in der weiter oben angegebenen Formel (I-b) für ein gegebenenfalls substituiertes Biphenylyl steht, kann man die entsprechenden Biphenyllactame der Formel (XI-a) in einer vorteilhaften Variante des hier beschriebene Verfahrens herstellen, indem man analog zum oben und weiter unten beschriebenen Verfahren (C) bestimmte Phenyllactame der Formel (XVII) mit Boronsäuren der Formel (VI) gemäß dem folgenden Reaktionsschema umsetzt:

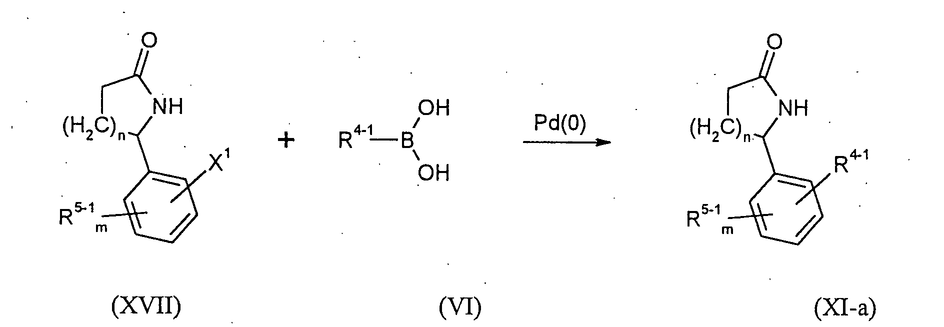

Die Phenyllactame der Formel (XVII), in denen X¹ für Brom oder Iod steht, sind eine Teilmenge der Verbindungen der Formel (XI), deren Herstellung oben angegeben ist. Die Phenyllactame der Formel (XVII), in denen X¹ für Trifluormethansulfonyl steht, lassen sich analog wie bei Verfahren (C) beschrieben aus den entsprechenden Verbindungen der Formel (XI), in denen Ar² durch R⁴ = Hydroxy substituiert ist, herstellen.

Die neuen Aminoketone der Formel (II) lassen sich z.B. auch herstellen, indem man in einem Verfahren (A.b) die Nitrogruppe der Nitroketone der Formel (XVIII), welche ebenfalls neu sind, gemäß dem folgendem Reaktionsschema reduziert:

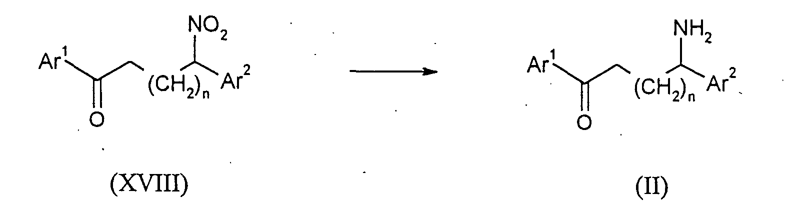

Die Reduktion kann durch katalytische Hydrierung oder andere allgemein bekannte Methoden zur Reduktion von Nitrogruppen durchgeführt werden (vgl. z.B. Houben-Weyl, Methoden der Organischen Chemie, Georg Thieme Verlag Stuttgart, Band **11/1,** 394-409 und Band **4/1c,** 490-506). Bevorzugt sind die Methoden, bei denen im sauren Medium gearbeitet wird, weil die Aminoketone der Formel (II) vorzugsweise als Salze isoliert werden.

Nitroketone der Formel (XVIII) lassen sich z.B. herstellen, indem man ω-Chloralkylphenylketone der Formel (XXI) in Gegenwart eines Verdünnungsmittels wie z.B. Methanol, Ethanol, einem anderen niederen aliphatischen Alkohol oder auch Tetrahydrofuran und in Gegenwart eines Säurebindemittels wie z.B. Natriumhydrid oder eines Alkalialkoholates, vorzugsweise des entsprechenden als Verdünnungsmittel eingesetzten Alkohols, gemäß dem folgenden Reaktionsschema kondensiert:

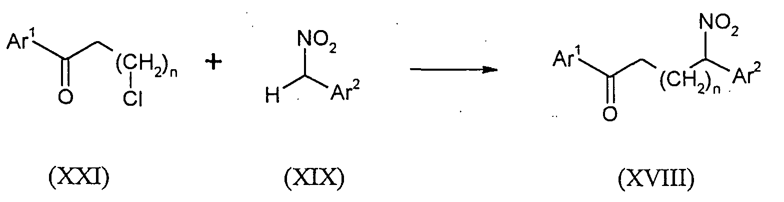

Die ω-Chloralkylphenylketone der Formel (XXI) sind z.T. kommerziell erhältlich, bekannt oder lassen sich auf bekannte Weise herstellen, wie z.B. durch Friedel-Crafts-Acylierung entsprechender Benzolderivate der Formel (XXII) (s. weiter unten) mit 3-Chlorpropionsäurechlorid, 4-Chlorbuttersäurechlorid oder 5-Chlorvaleriansäurechlorid.

Die Nitromethylbenzole der Formel (XIX) sind bekannt oder lassen sich auf bekannter Weise herstellen, wie z.B. durch Nitrieren entsprechender Toluole in der Seitenkette oder Umsetzung entsprechender Benzylhalogenide mit Silbernitrit [vgl. hierzu z.B. J. Am. Chem. Soc. 77, 6269 (1955); J. Am. Chem. Soc **86,** 2681 (1964); Houben-Weyl, Methoden der Organischen Chemie, Georg Thieme Verlag Stuttgart, Band **10/1,** 46-57 (Halogensubstitution), Band **E16,** 145-154 (Beide Methoden)]. Die hierfür benötigten Toluole oder Benzylhalogenide sind allgemein bekannte Verbindungen der organischen Chemie.

Die Nitroketone der Formel (XVIII), in welcher n gleich 1 ist (XVIII-a), lassen sich z.B. herstellen, indem man Michael-Additionen von Nitromethylbenzolen der Formel (XIX) an Phenylvinylketone der Formel (XX) in Gegenwart eines Verdünnungsmittels wie z.B. Methanol, Ethanol oder einem anderen niederen aliphatischen Alkohol und in Gegenwart eines Säurebindemittels wie z.B. vorzugsweise eines Alkalialkoholates des entsprechenden als Verdünnungsmittel eingesetzten. Alkohols gemäß dem folgenden Reaktionsschema durchführt (vgl. z.B. J. Prakt. Chem., Serie 4, 1, 57 (1955); Houben-Weyl, Methoden der Organischen Chemie, Georg Thieme Verlag Stuttgart, Band **10/1,** 199-206):

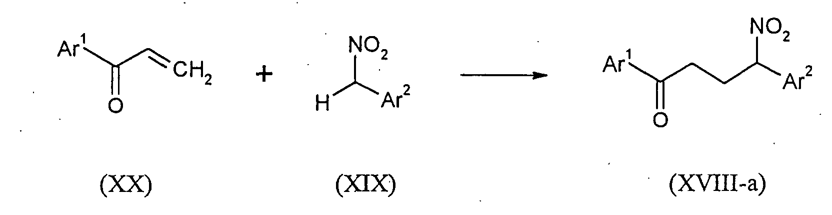

Die Phenylvinylketone der Formel (XX) lassen sich z.B. herstellen, indem man Chlorwasserstoff aus β-Chlorpropiophenonen der Formel (XXI-a), welche sich z.B. durch Friedel-Crafts-Acylierung entsprechender Benzolderiyate der Formel (XXII) mit 3-Chlorpropionsäurechlorid erhalten lassen, in Gegenwart eines Säurebindemittels wie beispielsweise Kaliumacetat in Gegenwart eines Verdünnungsmittels wie beispielsweise Methanol gemäß folgendem Reaktionsschema abspaltet [vgl. z.B. J. Prakt. Chem., Serie 4, 1, 57 (1955)]:

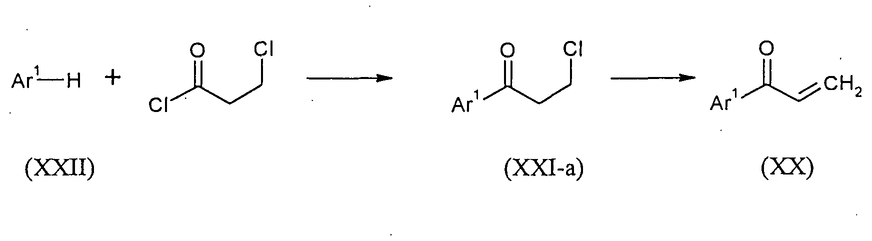

Die Benzolderivate der Formel (XXII) sind zum Teil kommerziell erhältlich, bekannt oder lassen sich nach allgemein bekannten Methoden der Aromatenchemie herstellen.

Phenylvinylketone der Formel (XX) lassen sich auch herstellen, indem man *O*-Methyl-methylbenzohydroxamate der Formel (XXIII) mit Vinylmagnesiumbromid gemäß folgendem Reaktionsschema umsetzt:

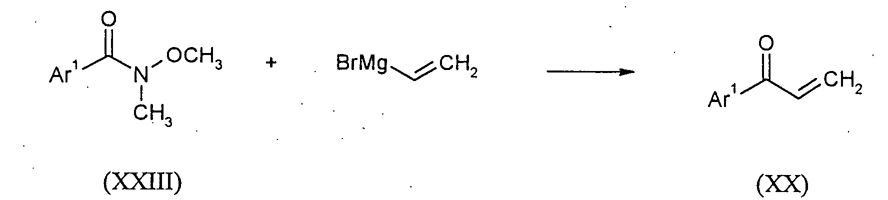

*O*-Methyl-methylbenzohydroxamate der Formel (XXIII) sind z.T. bekannt ("Weinreb-Amide") oder können nach bekannten Methoden z.B. aus den entsprechenden Benzoesäure-Derivaten hergestellt werden [vgl. z.B. Tetrahedron Lett. 22, 3815 (1981)].

Da die Phenylvinylketone der Formel (XX) z.T. empfindlich sind, setzt man in einer bevorzugten Variante zur Herstellung der Nitroketone der Formel (XVIII-a), diese direkt mit Nitromethylbenzolen der Formel (XIX) weiter um.

Nitroketone der Formel (XVIII-a) lassen sich auch herstellen, indem man gemäß nachfolgendem Reaktionsschema Enamine von Methylphenylketonen der Formel (XXVI) an α-Nitrostyrole der Formel (XXVII) addiert und das Reaktionprodukt sauer hydrolisiert:

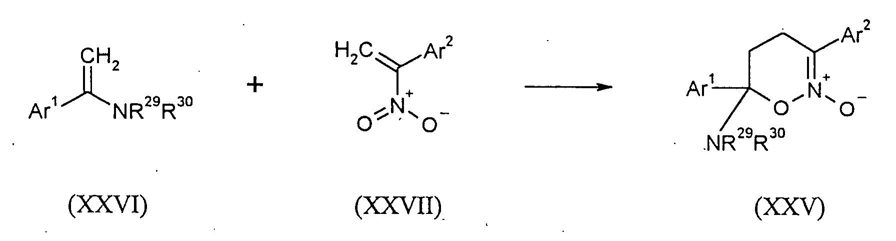

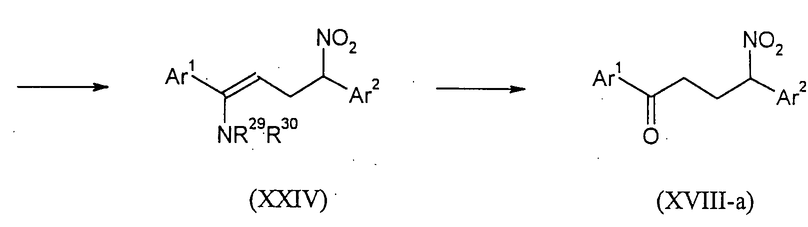

In Formeln (XXIV), (XXV) und (XXVI) stehen R²⁹ und R³⁰ gemeinsam mit dem Stickstoffatom, an das sie gebunden sind für einen cyclischen Aminorest, wie z.B. 1-Pyrrolidino, 1-Piperidino oder 4-Morpholino.

Die Addition verläuft zumeist in einer [4+2]-Cycloaddition zu isolierbaren 1,2-Oxazin-N-oxid-Derivaten der Formel (XXV) und wird gegebenenfalls in Gegenwart eines unpolaren Verdünnungsmittel wie z.B. Diethylether bei beispielsweise -80° bis +20°C durchgeführt. Zur Hydrolyse verwendet man z.B wäßrige Mineralsäuren wie Salzsäure gegebenenfalls in Gegenwart von Methanol oder Ethanol [vgl. z.B. Helv. Chim. Acta **68,** 162 (1985); Tetrahedron **45,** 2099 (1989)]. In vielen Fällen ist es vorteilhaft, zunächst die Ringöffnung zu Verbindungen der Formel (XXIV) durch einfaches Lösen des 1,2-Oxazin-N-oxid-Derivates in Methanol oder Ethanol durchzuführen, da sonst unerwünschte Nef-Reaktion zu der entsprechenden Diketo-Verbindung als Konkurrenzreaktion auftritt [vgl. z.B. Tetrahedron **45,** 2099 (1989)].

Die Enamine der Formel (XXVI) sind teilweise bekannt oder lassen sich z.B aus den entsprechend substituierten Acetophenonen und den cyclischen Aminen nach Standardverfahren herstellen (z.B. Org. Syntheses Vol. **58,** 56, John Wiley & Sons, New York). Die hierfür benötigten Acetophenone sind teilweise käuflich, bekannt oder lassen sich nach bekannten Methoden der Aromatenchemie herstellen.

Die Nitrostyrole der Formel (XXVII) sind teilweise bekannt oder lassen sich z.B. durch Formylierung der Nitromethylbenzole der oben angegebenen Formel (XIX) herstellen (vgl. z.B. Houben-Weyl, Methoden der Organischen Chemie, Georg Thieme Verlag, Stuttgart, Band **E16**, 215).

Die neuen Aminoketone der Formel (II) lassen sich z.B. auch herstellen, indem man in einem Verfahren (A.c) Imine der Formel (XXVIII) gemäß dem folgenden Reaktionsschema hydrolysiert:

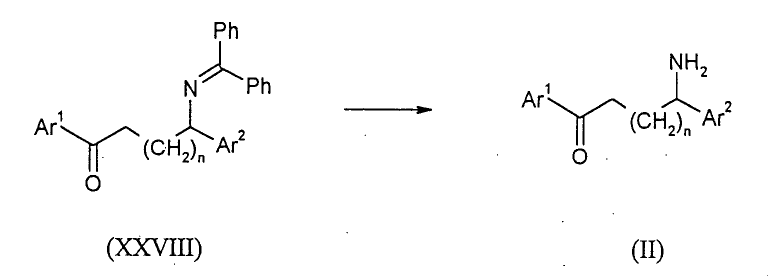

Die Hydrolyse läßt sich nach allgemein bekannten Methoden z.B. mit wäßriger Salzsäure durchführen. Dabei isoliert man die Aminoketone der Formel (II) auch hier vorzugsweise, wie weiter oben beschrieben, als ihre Salze, beispielsweise als Hydrochloride.

Die Imine der Formel (XXVIII), in welcher n gleich 1 ist (XXVIII-a), lassen sich z.B. herstellen, indem man Michael-Additionen von N-Diphenylmethylenbenzylaminen der Formel (XXIX) an Phenylvinylketone der Formel (XX) gemäß dem folgendem Reaktionsschema durchführt:

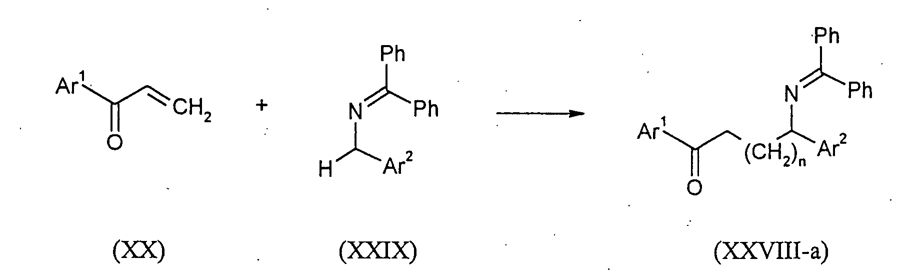

Die Addition wird in Gegenwart eines Säurebindemittels und in Gegenwart eines Verdünnungsmittels wie z.B. Acetonitril oder Dichlormethan und gegebenenfalls in Gegenwart eines Reaktionshilfsmittels z.B. bei Raumtemperatur durchgeführt. Als Säurebindemittel dient vorzugsweise wäßriges Alkali wie 50%-ige Natronlauge in Gegenwart eines Phasentransferkatalysators wie z.B. Triethylbenzylammoniumchlorid als Reaktionshilfsmittel [vgl. z.B. Synth. Commun.17, 211 (1987)].

Die Herstellung der Phenylvinylketone der Formel (XX) ist weiter oben beschrieben. Die *N*-Diphenylmethylenbenzylamine der Formel (XXIX) erhält man z.B. durch Umsetzung der entsprechenden Benzylamine mit Benzophenon (vgl. z.B. Tetrahedron. Lett. 1978, 2641). Die hierfür benötigten Benzylamine sind bekannt oder können nach bekannten Methoden wie z.B. Aminolyse der entsprechenden Benzylhalogenide (siehe oben) hergestellt werden.

Die zur Durchführung des erfindungsgemäßen Verfahrens (B) benötigten cyclischen *O*-Methansulfonyloxime sind durch die Formel (III) allgemein definiert. In dieser Formel haben Ar² und n vorzugsweise diejenigen Bedeutungen, die bereits im Zusammenhang mit der Beschreibung der cyclischen Imine der Formel (I) als bevorzugt genannt wurden. Die *O*-Methansulfonyloxime der Formel (III) sind neu.

Die *O*-Methylsulfonyloxime der Formel (III) lassen sich herstellen, indem man z.B. gemäß dem nachfolgenden Reaktionsschema cyclische α-Arylketone der Formel (XXXI) zunächst nach allgemein bekannten Methoden in ihre Oxime der Formel (XXX) überführt und diese anschließend mit Methansulfonylchlorid analog der Mesylierung von Alkoholen umsetzt:

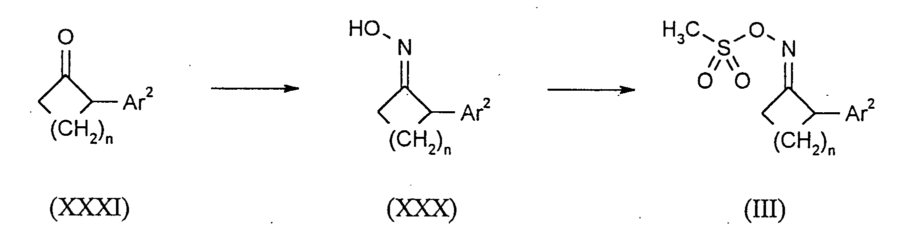

Cyclische α-Arylketone der Formel (XXXI) lassen sich z.B. herstellen, indem man gemäß nachfolgendem Reaktionsschema 1-Arylcycloalkene der Formel (XXXIII) nach üblichen Methoden, wie beispielweise mit m-Chlorperbenzoesäure, zu Oxiranen der Formel (XXXII) epoxidiert und diese anschließend durch saure Aufarbeitung isomerisiert [vgl. z.B. Tetrahedron 30, 2027 (1974)]:

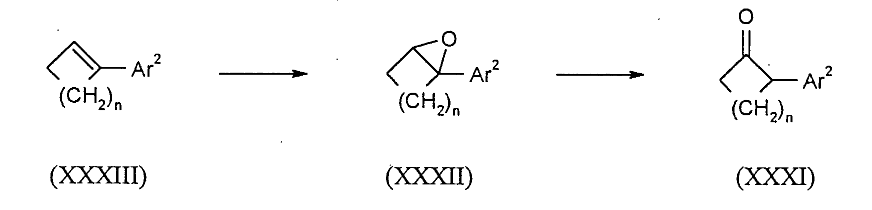

Natürlich kann man auch auf anderen Wegen erhaltene Oxirane der Formel (XXXII) z.B. durch Schütteln einer Lösung in Chloroform mit 20%-iger Schwefelsäure zu cyclischen α-Arylketonen der Formel (XXXI) isomerisieren.

1-Arylcycloalkene der Formel (XXXIII) lassen sich z.B. herstellen, indem man gemäß dem nachfolgenden Reaktionsschema weiter vorne beschriebene Aryl-Grignard-Verbindungen der Formel (XIV) mit Ketonen der Formel (XXXV) unter üblichen Grignard-Bedingungen umsetzt und die z.B. auf diese Weise erhaltenen cyclischen Benzylalkohole der Formel (XXXIV) dehydratisiert:

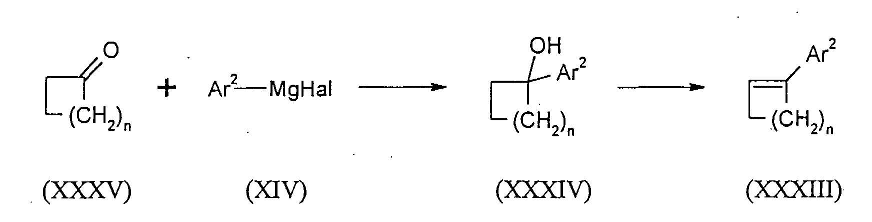

Die Dehydratisierung kann man beispielsweise durchführen, indem man den Alkohol in einem wenig polaren Lösungsmittel wie Hexan löst und bei z.B. 0° bis 20°C mit halbkonzentrierter Schwefelsäure verrührt [vgl. z.B. Tetrahedron 30, 2027 (1974)].

Die Ketone der Formel (XXXV), Cyclobutanon, Cyclopentanon und Cyclohexanon sind alle kommerziell erhältlich.

Die weiterhin zur Durchführung des erfindungsgemäßen Verfahrens (B) benötigten Aryl-Grignard-Verbindungen sind durch die Formel (IV) allgemein definiert. In dieser Formel hat Ar¹ vorzugsweise diejenige Bedeutung, die bereits im Zusammenhang mit der Beschreibung der cyclischen Imine der Formel (I) als bevorzugt genannt wurde.

Aryl-Grignard-Verbindungen der Formel (IV) sind bekannt oder lassen sich durch Grignard-Reaktion aus entsprechenden Arylhalogeniden und Magnesium herstellen. Arylhalogenide sind allgemein bekannte Verbindungen der organischen Chemie.

Die zur Durchführung des erfindungsgemäßen Verfahrens (C) benötigten cyclischen Imine der Formel (V) sind, soweit X¹ für Brom oder Iod steht, Teilmengen der erfindungsgemäßen Verbindungen der allgemeinen Formel (I) und lassen sich beispielsweise nach den Verfahren (A) oder (B) herstellen. Für den Fall, daß X¹ für Trifluormethansulfonyl steht, lassen sie sich die Verbindungen der Formel (V-a) durch Umsetzung von Hydroxyverbindungen der Formel (I-f), welche ebenfalls nach den Verfahren (A) oder (B) hergestellt werden können, mit Trifluormethansulfonylchlorid oder Trifluormethansulfonsäureanhydrid in Gegenwart eines Säurebindemittels wie z.B. Pyridin und gegebenenfalls in Gegenwart eines Verdünnungsmittels gemäß folgendem Reaktionsschema herstellen:

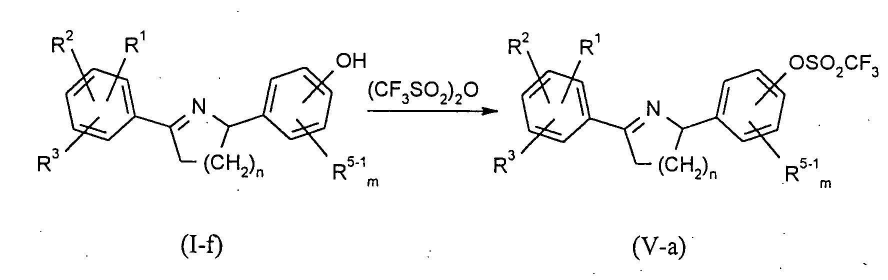

Die ebenfalls zur Durchführung des erfindungsgemäßen Verfahrens (C) benötigten Boronsäuren sind durch die Formel (VI) allgemein definiert. In dieser Formel hat R⁴⁻¹ vorzugsweise diejenige Bedeutung, die bereits im Zusammenhang mit der Beschreibung der cyclischen Imine der Formel (I-b) als bevorzugt genannt wurden.

Aromatische Boronsäuren der Formel (VI) sind bekannt oder können nach bekannten Methoden hergestellt werden [vgl. Chem. Rev. 45, 2457 (1995); Pure Appl. Chem. 66, 213 (1994)].

Die zur Durchführung des erfindungsgemäßen Verfahrens (D) benötigten cyclischen Imine der Formel (I-d) sind Teilmengen der erfindungsgemäßen Verbindungen der allgemeinen Formel (I) und lassen sich beispielsweise nach den Verfahren (A) bis (C) herstellen.

Die weiteren zur Durchführung des erfindungsgemäßen Verfahrens (D) benötigten Verbindungen sind durch die Formel (VII) definiert. In dieser Formel haben R¹⁵, R¹⁶, G, p, q und r vorzugsweise diejenigen Bedeutungen, die bereits im Zusammenhang mit der Beschreibung der cyclischen Imine der Formel (I) als bevorzugt genannt wurden. Ab steht für eine übliche Abgangsgruppe wie z.B. Halogen, insbesondere Chlor oder Brom; Alkylsulfonyloxy, insbesondere Methylsulfonyloxy; oder gegebenenfalls substituiertes Arensulfonyloxy, insbesondere Phenylsulfonyloxy, p-Chlorsulfonyloxy oder p-Tolylsulfonyloxy.

Die Verbindungen der Formel (VII) sind allgemein bekannte Verbindungen der Organischen Chemie.

Das erfindungsgemäße Verfahren (A) wird gegebenenfalls in Gegenwart eines geeigneten Säureakzeptors durchgeführt. Als solche kommen alle üblichen anorganischen oder organischen Basen in Frage. Hierzu gehören vorzugsweise Erdalkalimetall- oder Alkalimetallhydroxide, -amide, -alkoholate, -acetate, -carbonate oder -hydrogencarbonate, wie beispielsweise Natrium-, Kalium- oder Ammoniumhydroxid, Natriumamid, Lithiumdiisopropylamid, Natrium-methylat, Natrium-ethylat, Kalium-tert.-butylat, Natrium-, Kalium-, Calcium- oder Ammoniumacetat, Natrium-, Kalium- oder Ammoniumcarbonat, Natriumhydrogen- oder Kaliumhydrogencarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Das erfindungsgemäße Verfahren (A) wird gegebenenfalls ín Gegenwart eines Verdünnungsmittels durchgeführt. Hierfür kommen Wasser, organische Lösungsmittel und Mischungen davon in Betracht. Beispielhaft seien genannt: aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Methylenchlorid, Chloroform, Tetrachlormethan, Dichlor-, Trichlorethan oder Tetrachlorethylen; Ether, wie Diethyl-, Diisopropyl-, Methyl-t-butyl-, Methyl-t-amylether, Dioxan, Tetrahydrofuran, 1,2-Dimethoxyethan, 1,2-Diethoxyethan, Diethylenglykoldimethylether oder Anisol; Ketone, wie Aceton, Butanon, Methyl-isobutylketon oder Cyclohexanon; Nitrile, wie Acetonitril, Propionitril, n-oder i-Butyronitril oder Benzonitril; Amide, wie Formamid, N,N-Dimethylformamid, N,N-Dimethylacetamid, N-Methylformanilid, N-Methylpyrrolidon oder Hexamethylphosphorsäuretriamid; N-Oxide wie N-Methylmorpholin-N-oxid; Ester wie Methyl-, Ethyl- oder Butylacetat; Sulfoxide, wie Dimethylsulfoxid; Sulfone, wie Sulfolan; Alkohole, wie Methanol, Ethanol, n- oder i-Propanol, n-, iso-, sekoder tert-Butanol, Ethandiol, Propan-1,2-diol, Ethoxyethanol, Methoxyethanol, Diethylenglykolmonomethylether, Diethylenglykolmonoethylether; Wasser.

Die Reaktionstemperatur kann bei dem erfindungsgemäßen Verfahren (A) innerhalb eines größeren Bereiches variiert werden. Im allgemeinen arbeitet man bei Temperaturen zwischen -50°C und +150°C, bevorzugt zwischen -20°C und +100°C.

Bei der Durchführung des erfindungsgemäßen Verfahrens (A) werden das Salz der Verbindung der Formel (II) und die Base im allgemeinen im äquimolaren Verhältnis eingesetzt.

In einer bevorzugten Variante des Verfahrens wird das Aminoketon der Formel (II) auf einem der Wege (A.a) bis (A.c) hergestellt und ohne Isolierung "in einem Topf" durch Zugabe einer Base gemäß Verfahren (A) cyclokondensiert.

Als Verdünnungsmittel zur Durchführung des erfindungsgemäßen Verfahrens (B) kommen inerte organische Lösungsmittel und Mischungen davon in Betracht. Beispielhaft seien genannt: aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Methylenchlorid, Chloroform, Tetrachlormethan, Dichlor-, Trichlorethan oder Tetrachlorethylen; Ether, wie Diethyl-, Diisopropyl-, Methyl-t-butyl-, Methyl-t-amylether, Dioxan, Tetrahydrofuran, 1,2-Dimethoxyethan, 1,2-Diethoxyethan, Diethylenglykoldimethylether oder Anisol.

Bevorzugt setzt man eine Lösung der Grignard-Verbindung der Formel (IV) in einem Ether und eine Lösung des *O*-Methylsulfonyloxim der Formel (III) in einem Kohlenwasserstoff ein.

Die Reaktionstemperatur kann bei dem erfindungsgemäßen Verfahren (B) innerhalb eines größeren Bereiches variiert werden. Im allgemeinen arbeitet man bei Temperaturen zwischen -100°C und +50°C, bevorzugt zwischen -80°C und +30°C.

Bei der Durchführung des erfindungsgemäßen Verfahrens (B) werden Grignard-Verbindung der Formel (IV) und *O*-Methylsulfonyloxim der Formel (III) im molaren Verhältnis von 1:1 bis 3:1, vorzugsweise 1:1 bis 2:1 eingesetzt.

Zur Durchführung des erfindungsgemäßen Verfahrens (C) sind Palladium(0)-Komplexe als Katalysator geeignet. Bevorzugt wird beispielsweise Tetrakis(triphenylphosphin)palladium.

Als Säureakzeptors zur Durchführung des erfindungsgemäßen Verfahrens (C) kommen anorganische oder organische Basen in Frage. Hierzu gehören vorzugsweise Erdalkalimetall- oder Alkalimetallhydroxide, -acetate, -carbonate oder -hydrogencarbonate, wie beispielsweise Natrium-, Kalium-, Barium- oder Ammoniumhydroxid, Natrium-, Kalium-, Calcium- oder Ammoniumacetat, Natrium-, Kalium- oder Ammoniumcarbonat, Natriumhydrogen- oder Kaliumhydrogencarbonat, Alkalifluoride, wie beispielsweise Cäsiumfluorid, sowie tertiäre Amine, wie Trimethylamin, Triethylamin, Tributylamin, N,N-Dimethylanilin, N,N-Dimethylbenzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Als Verdünnungsmittel zur Durchführung des erfindungsgemäßen Verfahrens (C) kommen Wasser, organische Lösungsmittel und Mischungen davon in Betracht. Beispielhaft seien genannt: aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Methylenchlorid, Chloroform, Tetrachlormethan, Dichlor-, Trichlorethan oder Tetrachlorethylen; Ether, wie Diethyl-, Diisopropyl-, Methyl-t-butyl-, Methyl-t-amylether, Dioxan, Tetrahydrofuran, 1,2-Dimethoxyethan, 1,2-Diethoxyethan, Diethylenglykoldimethylether oder Anisol; Alkohole, wie Methanol, Ethanol, n- oder i-Propanol, n-, iso-, sek- oder tert-Butanol, Ethandiol, Propan-1,2-diol, Ethoxyethanol, Methoxyethanol, Diethylenglykolmonomethylether, Diethylenglykolmonoethylether.

Die Reaktionstemperatur kann bei dem erfindungsgemäßen Verfahren (C) innerhalb eines größeren Bereiches variiert werden. Im allgemeinen arbeitet man bei Temperaturen zwischen 0°C und +140°C, bevorzugt zwischen 50°C und +100°C.

Bei der Durchführung des erfindungsgemäßen Verfahrens (C) werden Boronsäure der Formel (VI) und Verbindung der Formel (V) im molaren Verhältnis von 1:1 bis 3:1, vorzugsweise 1:1 bis 2:1 eingesetzt. Vom Katalysator setzt man im allgemeinen 0,005 bis 0,5 Mol, vorzugsweise 0,01 mol bis 0,1 Mol pro Mol der Verbindung der Formel (V) ein. Die Base setzt man im allgemeinen in einem Überschuß ein.

Das erfindungsgemäße Verfahren (D) wird vorzugsweise in Gegenwart eines geeigneten Säureakzeptors durchgeführt. Als solche kommen alle üblichen anorganischen oder organischen Basen in Frage. Hierzu gehören vorzugsweise Erdalkalimetall- oder Alkalimetallhydride, -hydroxide, -amide, -alkoholate, -acetate, -carbonate oder -hydrogencarbonate, wie beispielsweise Natriumhydrid, Natrium-, Kalium- oder Ammoniumhydroxid, Natriumamid, Lithiumdiisopropylamid, Natrium-methylat, Natrium-ethylat, Kalium-tert.-butylat, Natrium-, Kalium-, Calciumoder Ammoniumacetat, Natrium-, Kalium- oder Ammoniumcarbonat, Natriumhydrogen- oder Kaliumhydrogencarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Das erfindungsgemäße Verfahren (D) kann in Gegenwart eines geeigneten Phasentransferkatalysators durchgeführt werden. Als Beispiele für solche Katalysatoren seien genannt: Tetrabutylammoniumiodid, -bromid oder -chlorid, Tributylmethylphosphoniumbromid, Trimethyl-C₁₃/C₁₅-alkylammoniumchlorid oder -bromid, Dibenzyldimethylammonium-methylsulfat, Dimethyl-C₁₂/C₁₄-alkylbenzylammoniumchlorid, 15-Krone-5, 18-Krone-6 oder Tris-[2-(2-methoxyethoxy)-ethyl]-amin.

Das erfindungsgemäße Verfahren (D) wird vorzugsweise ín Gegenwart eines Verdünnungsmittels durchgeführt. Hierfür kommen z.B. alle bei Verfahren (A) aufgelisteten Lösungsmittel in Frage.

Die Reaktionstemperatur kann bei dem erfindungsgemäßen Verfahren (D) innerhalb eines größeren Bereiches variiert werden. Im allgemeinen arbeitet man bei Temperaturen zwischen -20°C und +100°C, bevorzugt zwischen 0°C und 60°C.

Bei der Durchführung des erfindungsgemäßen Verfahrens (D) arbeitet man im allgemeinen mit ungefähr äquimolaren Mengen der Edukte. Man kann jedoch auch einen Überschuß der Verbindung der Formel (VII) verwenden.

Die Umsetzungen gemäß dem erfindungsgemäßen Verfahren E) sind dem Fachmann bekannte Derivatisierungsreaktionen insbesondere von Carbonsäureestern und Ketonen (vgl. z.B. Houben-Weyl, Methoden der Organischen Chemie, Georg Thieme Verlag, Stuttgart, Bd. VII/2b, insbesonders 1912 ff; Bd. VIII zu Carbonsäureestern und deren Derivaten; Bd. E5, insbesondere S. 812 ff und die darin zitierte Literatur).

Die Umsetzungen der erfindungsgemäßen Verfahren können bei Normaldruck oder unter erhöhtem Druck durchgeführt werden, vorzugsweise wird bei Normaldruck gearbeitet. Die Aufarbeitung geschieht nach üblichen Methoden der Organischen Chemie. Die Endprodukte werden vorzugsweise durch Kristallisation, chromatographische Reinigung oder durch Entfernung der flüchtigen Bestandteile gegebenenfalls im Vakuum gereinigt.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorratsund Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:
Aus der Ordnung der Isopoda z.B. Oniscus asellus, Armadillidium vulgare, Porcellio scaber.
   Aus der Ordnung der Diplopoda z.B. Blaniulus guttulatus.
   Aus der Ordnung der Chilopoda z.B. Geophilus carpophagus, Scutigera spec.
   Aus der Ordnung der Symphyla z.B. Scutigerella immaculata.
   Aus der Ordnung der Thysanura z.B. Lepisma saccharina.
   Aus der Ordnung der Collembola z.B. Onychiurus armatus.
   Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
   Aus der Ordnung der Dermaptera z.B. Forficula auricularia.
   Aus der Ordnung der Isoptera z.B. Reticulitermes spp..
   Aus der Ordnung der Anoplura z.B. Pediculus humanus corporis, Haematopinus spp., Linognathus spp.
   Aus der Ordnung der Mallophaga z.B. Trichodectes spp., Damalinea spp.
   Aus der Ordnung der Thysanoptera z.B. Hercinothrips femoralis, Thrips tabaci.
   Aus der Ordnung der Heteroptera z.B. Eurygaster spp., Dysdercus intermedius, Piesma quadrata, Cimex lectularius, Rhodnius prolixus, Triatoma spp.
   Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Aphis pomi, Eriosoma lanigerum, Hyalopterus arundinis, Phylloxera vastatrix, Pemphigus spp., Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.
   Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
   Aus der Ordnung der Hymenoptera z.B. Diprion spp., Hoplocampa spp., Lasius spp., Monomorium pharaonis, Vespa spp.
   Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.
   Aus der Ordnung der Siphonaptera z.B. Xenopsylla cheopis, Ceratophyllus spp..
   Aus der Ordnung der Arachnida z.B. Scorpio maurus, Latrodectus mactans.
   Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp..

Die erfindungsgemäßen Wirkstoffe der Formel (I) zeichnen sich insbesondere durch hervorragende Wirkung gegen Larven des Meerettichblattkäfers (Phaedon cochleariae), Raupen des Eulenfalters (Spodoptera frugiperda), Larven der Grünen Reiszikade (Nephotettix cincticeps), Pfirsichblattläuse (Myzus persicae) und alle Stadien der gemeinen Spinnmilbe (Tetranychus urticae).

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoff-imprägnierte Natur- und synthetische Stoffe sowie Feinstverkapselungen in polymeren Stoffen.

Diese Formulierungen werden in bekannter Weise hergestellt, z. B. und bevorzugt durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

Z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen, wie Insektiziden, Lockstoffen, Sterilantien, Bakteriziden, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a..

Besonders günstige Mischpartner sind z.B. die folgenden:

### Fungizide:

2-Aminobutan; 2-Anilino-4-methyl-6-cyclopropyl-pyrimidin; 2',6'-Dibromo-2-methyl-4'-trifluoromethoxy-4'-trifluoro-methyl-1,3-thiazol-5-carboxanilid; 2,6-Dichloro-N-(4-trifluoromethylbenzyl)-benzamid; (E)-2-Methoxyimino-N-methyl-2-(2-phenoxyphenyl)-acetamid; 8-Hydroxyquinolinsulfat; Methyl-(E)-2-{2-[6-(2-cyanophenoxy)-pyrimidin-4-yloxy]-phenyl}-3-methoxyacrylat; Methyl-(E)-methoximino-[alpha-(o-tolyloxy)-o-tolyl]acetat; 2-Phenylphenol (OPP), Aldimorph, Ampropylfos, Anilazin, Azaconazol,
Benalaxyl, Benodanil, Benomyl, Binapacryl, Biphenyl, Bitertanol, Blasticidin-S, Bromuconazole, Bupirimate, Buthiobate,
Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodine, Drazoxolon,
Edifenphos, Epoxyconazole, Ethirimol, Etridiazol,
Fenarimol, Fenbuconazole, Fenfuram, Fenitropan, Fenpiclonil, Fenpropidin, Fenpropimorph, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
Guazatine,
Hexachlorobenzol, Hexaconazol, Hymexazol,
Imazalil, Imibenconazol, Iminoctadin, Iprobenfos (IBP), Iprodion, Isoprothiolan, Kasugamycin, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
Mancopper, Mancozeb, Maneb, Mepanipyrim, Mepronil, Metalaxyl, Metconazol, Methasulfocarb, Methfuroxam, Metiram, Metsulfovax, Myclobutanil,
Nickel-dimethyldithiocarbamat, Nitrothal-isopropyl, Nuarimol,
Ofurace, Oxadixyl, Oxamocarb, Oxycarboxin,
Pefurazoat, Penconazol, Pencycuron, Phosdiphen, Phthalid, Pimaricin, Piperalin, Polycarbamate, Polyoxin, Probenazol, Prochloraz, Procymidon, Propamocarb, Propiconazole, Propineb, Pyrazophos, Pyrifenox, Pyrimethanil, Pyroquilon,
Quintozen (PCNB),
Schwefel und Schwefel-Zubereitungen,
Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
Validamycin A, Vinclozolin,
Zineb, Ziram.

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, Bendiocarb, Benfuracarb, Bensultap, Betacyluthrin, Bifenthrin, BPMC, Brofenprox, Bromophos A, Bufencarb, Buprofezin, Butocarboxim, Butylpyridaben,
Cadusafos, Carbaryl, Carbofuran, Carbophenothion, Carbosulfan, Cartap, CGA 157 419, CGA 184699, Chloethocarb, Chlorethoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat, Dimethylvinphos, Dioxathion, Disulfoton,
Edifenphos, Emamectin, Esfenvalerat, Ethiofencarb, Ethion, Ethofenprox, Ethoprophos, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivermectin, Lamda-cyhalothrin, Lufenuron,
Malathion, Mecarbam, Mervinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, NI 25, Nitenpyram,
Omethoat, Oxamyl, Oxydemeton M, Oxydeprofos,
Parathion A, Parathion M, Permethrin, Phenthoat, Phorat, Phosalon, Phosmet, Phosphamidon, Phoxim, Pirimicarb, Pirimiphos M, Primiphos A, Profenofos, Promecarb, Propaphos, Propoxur, Prothiofos, Prothoat, Pymetrozin, Pyrachlophos, Pyradaphenthion, Pyresmethrin, Pyrethrum, Pyridaben, Pyrimidifen, Pyriproxifen,
Quinalphos,
RH 5992,
Salithion, Sebufos, Silafluofen, Sulfotep, Sulprofos,
Tebufenozid, Tebufenpyrad, Tebupirimphos, Teflubenzuron, Tefluthrin, Temephos, Terbam, Terbufos, Tetrachlorvinphos, Thiafenox, Thiodicarb, Thiofanox, Thiometon, Thionazin, Thuringiensin, Tralomethrin, Triarathen, Triazophos, Triazuron,
Trichlorfon, Triflumuron, Trimethacarb,
Vamidothion, XMC, Xylylcarb, YI 5301 / 5302, Zetamethrin.

Auch eine Mischung mit anderen bekannten Wirkstoffen, wie Herbiziden oder mit Düngemitteln und Wachstumsregulatoren ist möglich.

Der erfindungsgemäße Wirkstoff kann ferner in seinen handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Die erfindungsgemäßen Wirkstoffe wirken nicht nur gegen Pflanzen-, Hygieneund Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:

Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..

Aus der Ordnung der Siphonapterida z.B. Pulex spp., Ctenocephalides spp., Xenopsylla spp., Ceratophyllus spp..

Aus der Ordnung der Heteropterida z.B. Cimex spp., Triatoma spp., Rhodnius spp., Panstrongylus spp..

Aus der Ordnung der Blattarida z.B. Blatta orientalis, Periplaneta americana, Blattela germanica, Supella spp..

Aus der Unterklasse der Acaria (Acarida) und den Ordnungen der Meta- sowie Mesostigmata z.B. Argas spp., Ornithodorus spp., Otabius spp., Ixodes spp., Amblyomma spp., Boophilus spp., Dermacentor spp., Haemaphysalis spp., Hyalomma spp., Rhipicephalus spp., Dermanyssus spp., Raillietia spp., Pneumonyssus spp, Sternostoma spp., Varroa spp..

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Beispielsweise zeigen sie eine hervorragende Wirksamkeit gegen alle larvalen Stadien der Fliege *Lucilia cuprina* und alle Entwicklungsstadien der Zecke *Amblyomma variegatum.*

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen der Formel (I) eine hohe insektizide Wirkung gegen Insekten zeigen, die technische Materialien zerstören.

Beispielhaftund vorzugsweise - ohne jedoch zu limitieren - seien die folgenden Insekten genannt:

Käfer wie
Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus

Hautflügler wie
Sirex juvencus, Urocerus gigas, Urocerus gigas taignus, Urocerus augur

Termiten wie
Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus.

Borstenschwänze, wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz und Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen: Bauholz, Holzbalken, Eisenbahnschwellen, Brückenteile, Bootsstege, Holzfahrzeuge, Kisten, Paletten, Container, Telefonmasten, Holzverkleidungen, Holzfenster und -türen, Sperrholz, Spanplatten, Tischlerarbeiten oder Holzprodukte, die ganz allgemein beim Hausbau oder in der Bautischlerei Verwendung finden.

Die Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindeöl und/oder Monochlornaphthalin, vorzugsweise a-Monochlornaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, daß das Lösungsmittelgemisch ebenfalls eine Verdunstungszahl über 35 und,einen Flammpunkt oberhalb 30°C, vorzugsweise oberhalb 45°C, aufweist und daß das Insektizid-Fungizid--Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches durch ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glykolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel(gemisch) oder ein Weichmacher(gemisch) ersetzt werden. Diese Zusätze sollen einer Verflüchtigung der Wirkstoffe sowie einer Kristallisation bzw. Ausfällem vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der WO 94/29 268 genannten Insektizide und Fungizide in Frage. Die in diesem Dokument genannten Verbindungen sind ausdrücklicher Bestandteil der vorliegenden Anmeldung.

Als ganz besonders bevorzugte Zumischpartner seien Insektizide, wie Chlorpyriphos, Phoxim, Silafluofin, Alphamethrin, Cyfluthrin, Cypermethrin, Deltamethrin, Permethrin, Imidacloprid, M-25, Flufenoxuron, Hexaflumuron und Triflumuron, sowie Fungizide, wie Epoxyconazole, Hexaconazole, Azaconazole, Propiconazole, Tebuconazole, Cyproconazole, Metconazole, Imazalil, Dichlorfluanid, Tolylfluanid, 3-Iod-2-propinyl-butylcarbamat, N-Octyl-isothiazolin-3-on und 4,5-Dichlor-N-octylisothiazolin-3-on genannt.

Die Herstellung und die Verwendung der erfindungsgemäßen Wirkstoffe gehen aus den nachfolgenden Beispielen hervor.

### Herstellbeispiele

### Beispiel I-1

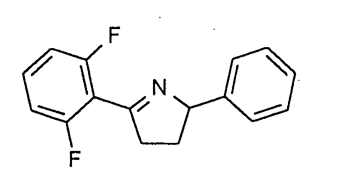

0,825 g 1-^{t}Butoxycarbonylamino-3-(2,6-difluorbenzoyl)-1-phenyl-propan (z.B. gemäß Bsp. VIII-1) wurden bei 0°C tropfenweise mit 1,6 ml Trifluoressigsäure versetzt. Es wurde auf Raumtemperatur erwärmen gelassen und 3 h nachgerührt. Anschließend wurde bei 0°C mit 1N Natronlauge alkalisch (pH 11) gestellt. Nach dreimaliger Extraktion mit Ethylacetat wurden die vereinigten Extrakte über Natriumsulfat getrocknet und im Vakuum eingedampft. Man erhielt 0,45 g (83 % der Theorie) 2-(2,6-Difluorphenyl)-5-phenyl-3,4-dihydro-2H-pyrrol.
¹H-NMR (400 MHz, CDCl₃) δ [ppm]: 1,75 (m, 1H, CHHCHPh); 2,85 (m, 1H, CHHCHPh); 3,00 (m, 2H, CH₂CN); 5,80 (t, 1H, NCHPh); 7,20-7,40 (m, 7H, ArH); 7,57 (m, 1H, ArH) (Ph = Phenyl).

### Beispiel I-2

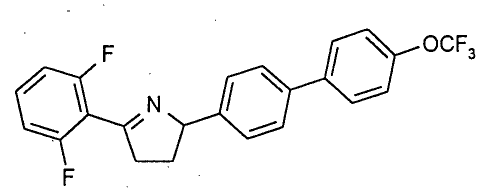

Analog zu Beispiel I-1 wurden aus 0,38 g 1-^{t}Butoxycarbonylamino-3-(2,6-difluorbenzoyl)-1-(4'-trifluormethoxybiphenyl-4-yl)-propan (z.B. gemäß Bsp. VIII-2) 0,36 g (96 % d.Th.) 2-(2,6-Difluorphenyl)-5-(4'-trifluormethoxybiphenyl-4-yl)-3,4-dihydro-2H-pyrrol erhalten.
¹H-NMR (500 MHz, CDCl₃) δ [ppm]: 2,10 (m, 1H,); 2,75 (m, 1H,); 3,29 (t, 2H); 5,59 (t, 1H); 7,05 (t, 2H); 7,29 (d: J ∼ 8 Hz, 2H); 7,42 (d: J = 7,4 Hz, 2H); 7,47 (m, 1H); 7,58 (d: J = 7,4 Hz, 2H); 7,60 (d: J = 7,4 Hz, 2H).

### Beispiel I-3

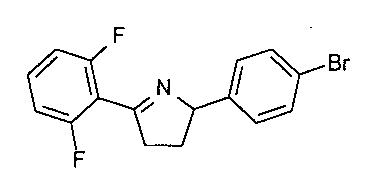

Analog zu Beispiel I-1 wurden aus 7,45 g 1-^{t}Butoxycarbonylamino-3-(2,6-difluorbenzoyl)-1-(4-bromphenyl)-propan (z.B. gemäß Bsp. VIII-3) 2,24 g (41 % d.Th.) 2-(2,6-Difluorphenyl)-5-(4-bromphenyl)-3,4-dihydro-2H-pyrrol erhalten.
¹H-NMR (400 MHz, CDCl₃) δ [ppm]: 1,70 (m, 1H, CHHCHPh); 2,58 (m, 1H, CHHCHPh); 3,00 (m, 2H, CH₂CN); 5,29 (t, 1H, NCHPh); 7,2-7,3 (m, 5H, ArH); 7,56 (m, 4H, ArH).

### Beispiel I-4

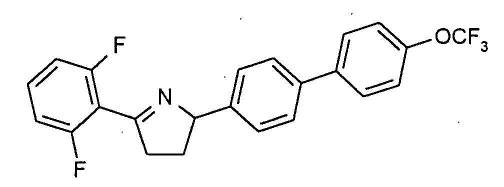

2,02 g 2-(2,6-Difluorphenyl)-5-(4-bromphenyl)-3,4-dihydro-2H-pyrrol (z.B. aus Bsp. I-3) wurden unter Argon in 20 ml Dimethoxyethan vorgelegt. Nacheinander gab man 2,02 g 4-Trifluormethoxyboronsäure sowie 0,346 g Tetrakis(triphenylphosphin)palladium. Nach 15 Min. wurden 9,6 ml 2M Sodalösung zugegeben, auf 80°C erwärmt und über Nacht gerührt. Nach Beendigung der Reaktion wurde in Wasser/Ethylacetat aufgenommen, die Phasen getrennt und die wäßrige Phase zweimal mit je ca. 100 ml Ethylacetat extrahiert. Die vereinigten organischen Phasen wurden mit gesättigter Kochsalzlösung gewaschen und über Magnesiumsulfat getrocknet. Durch Eindampfen wurden 1,90 g (76 % d.Th.) 2-(2,6-Difluorphenyl)-5-(4'-trifluormethoxybiphenyl-4-yl)-3,4-dihydro-2H-pyrrol (vgl. Bsp. I-2) erhalten.

### Beispiel I-5 bis I-41

Analog zu Beispiel I-4 bzw. gemäß den allgemeinen Angaben zur Herstellung wurden die in der nachfolgenden Tabelle 1 aufgeführten Verbindungen der Formel (I-f) erhalten.

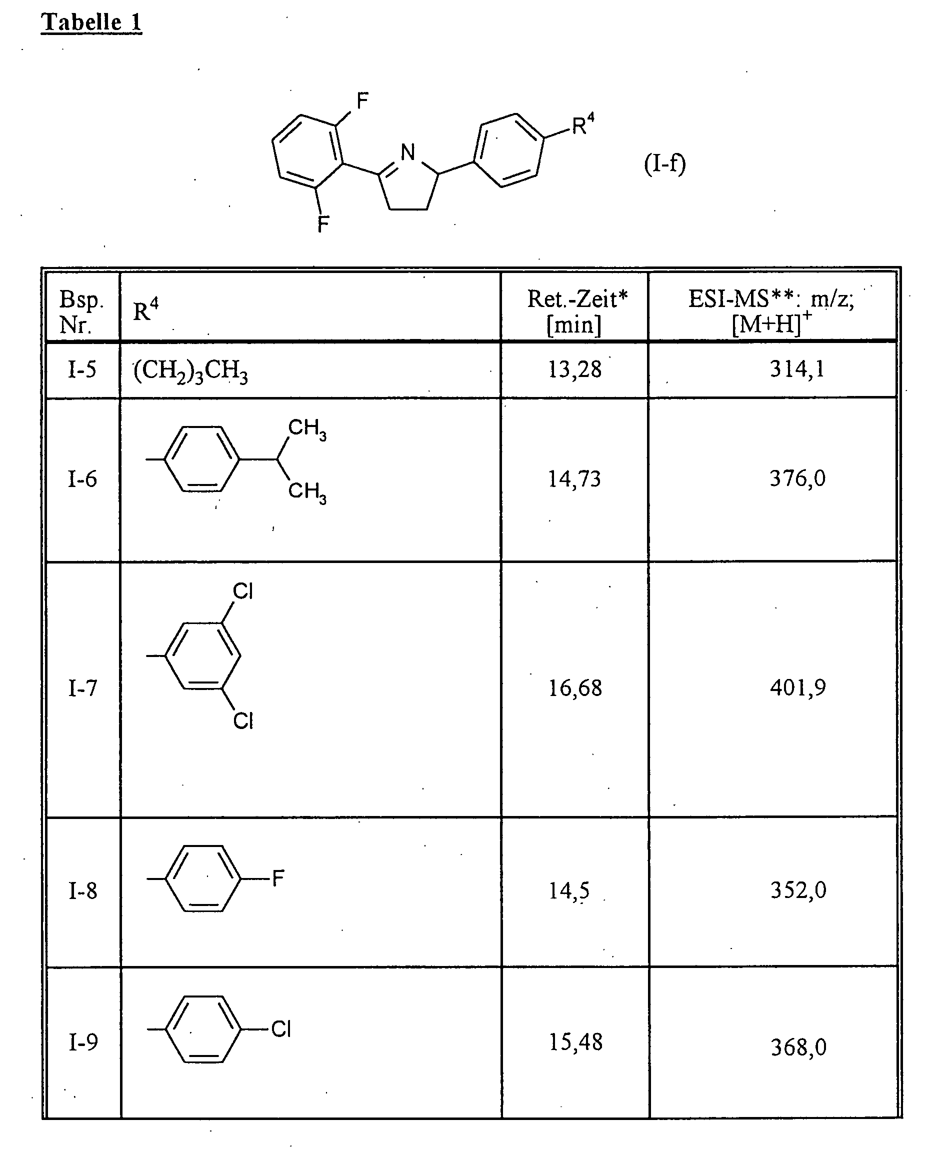

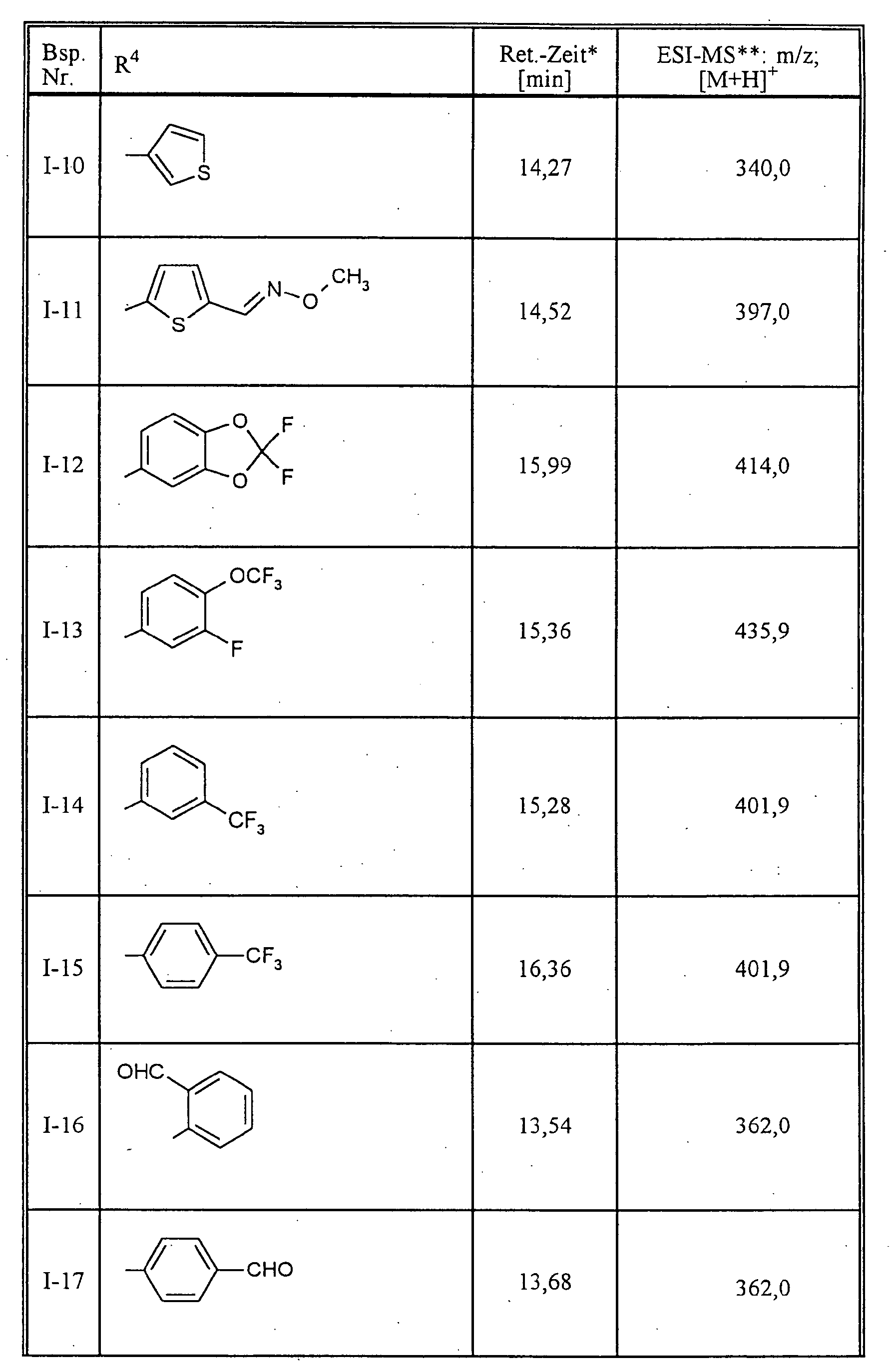

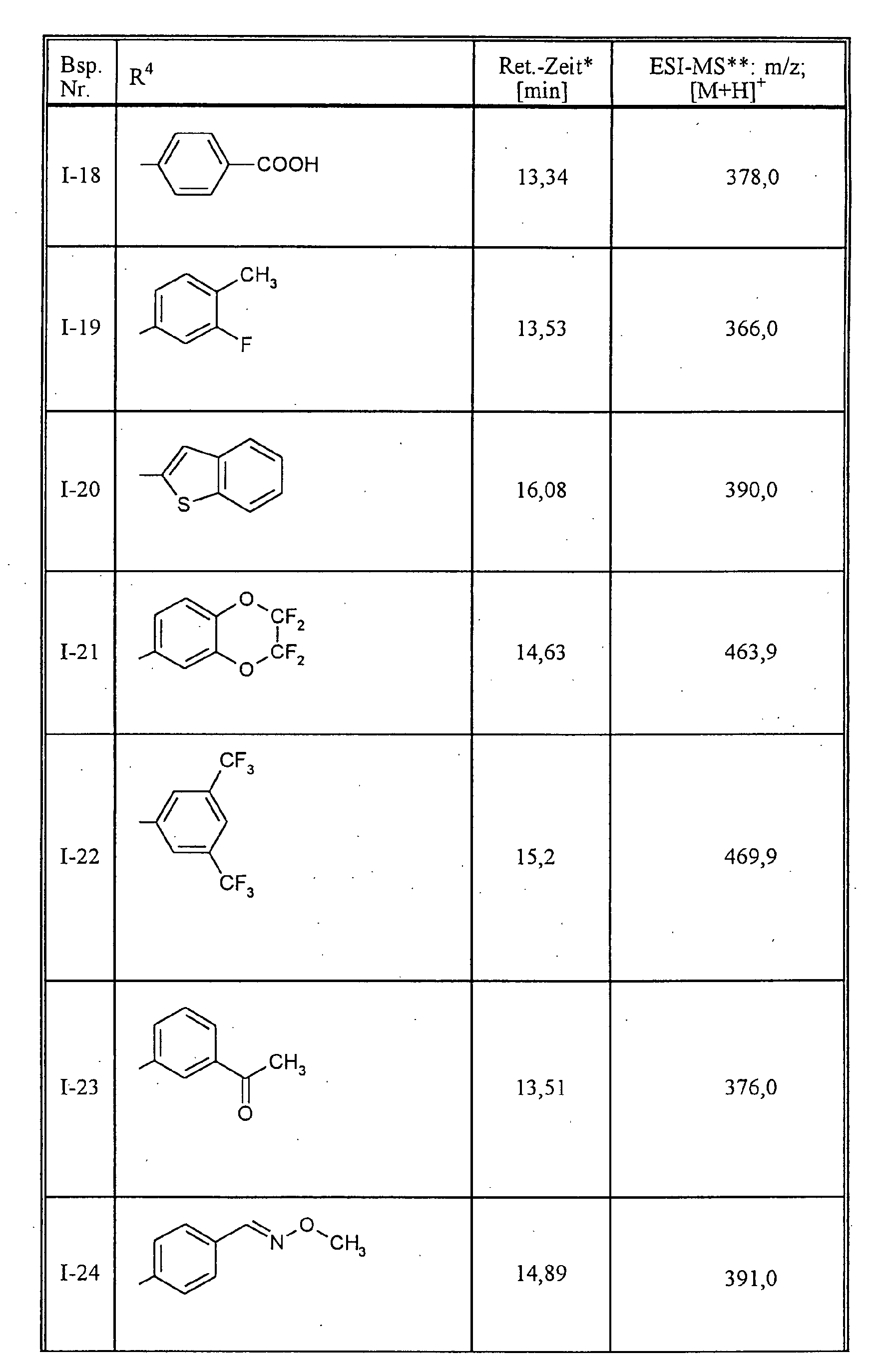

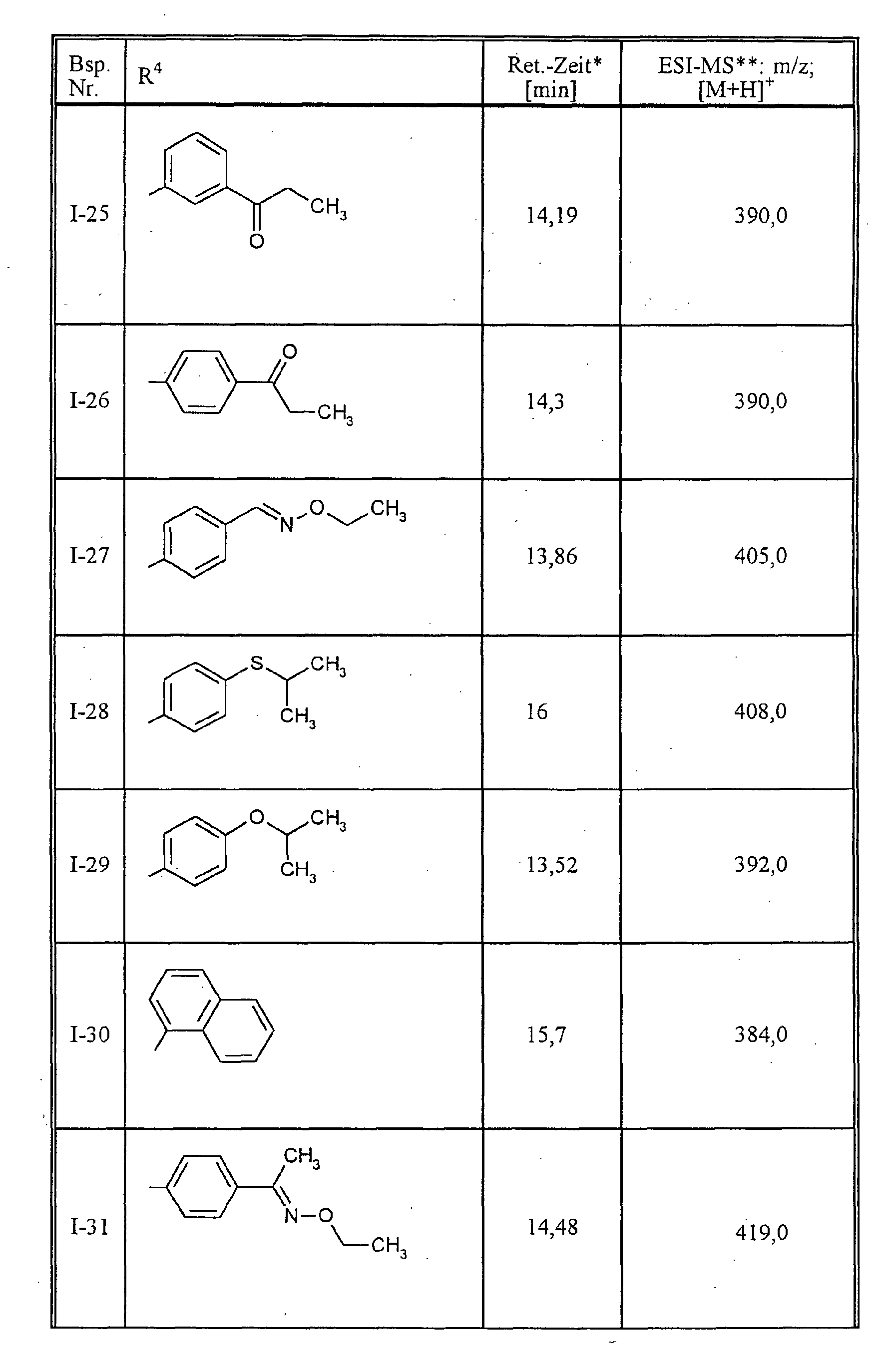

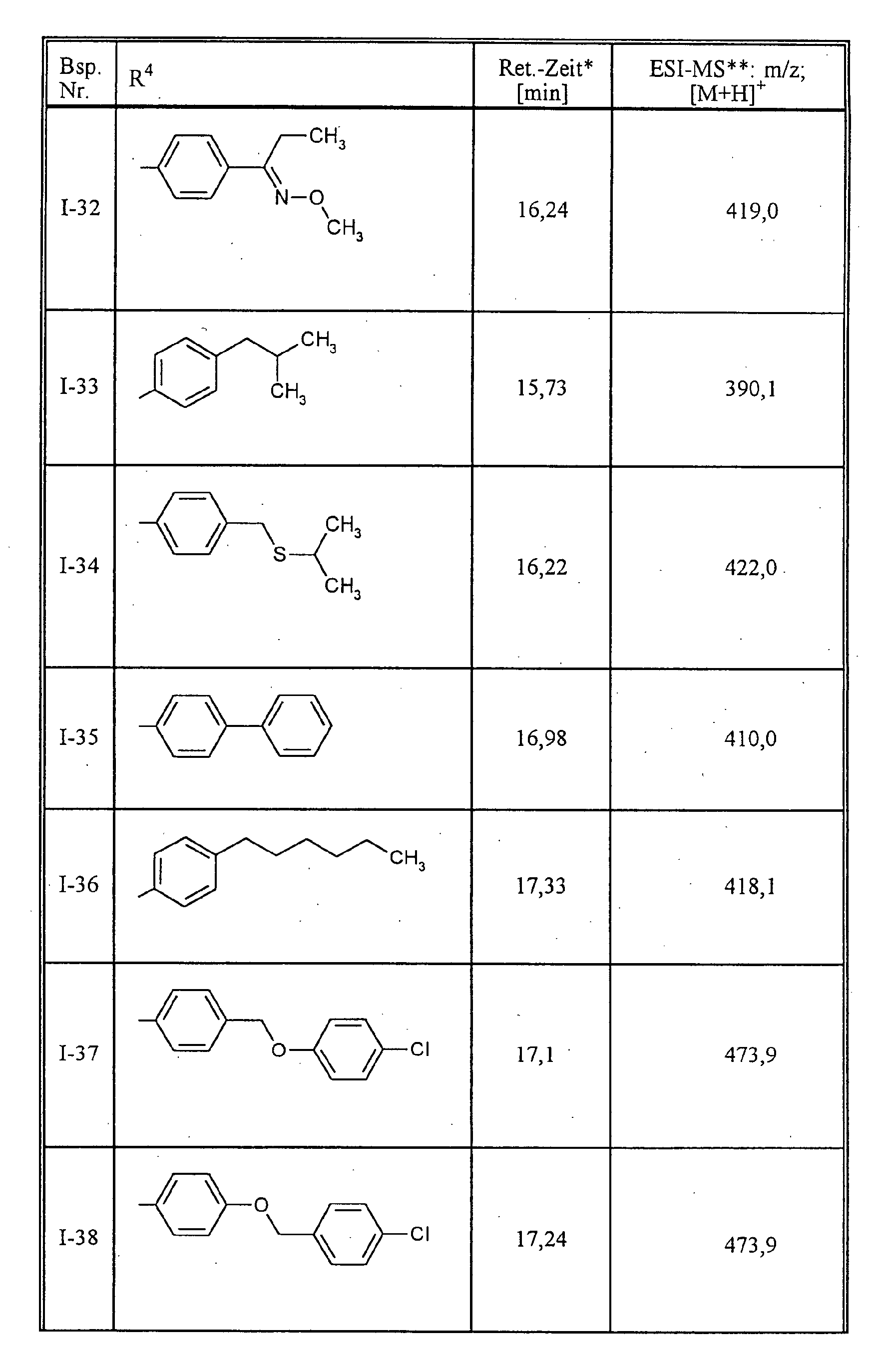

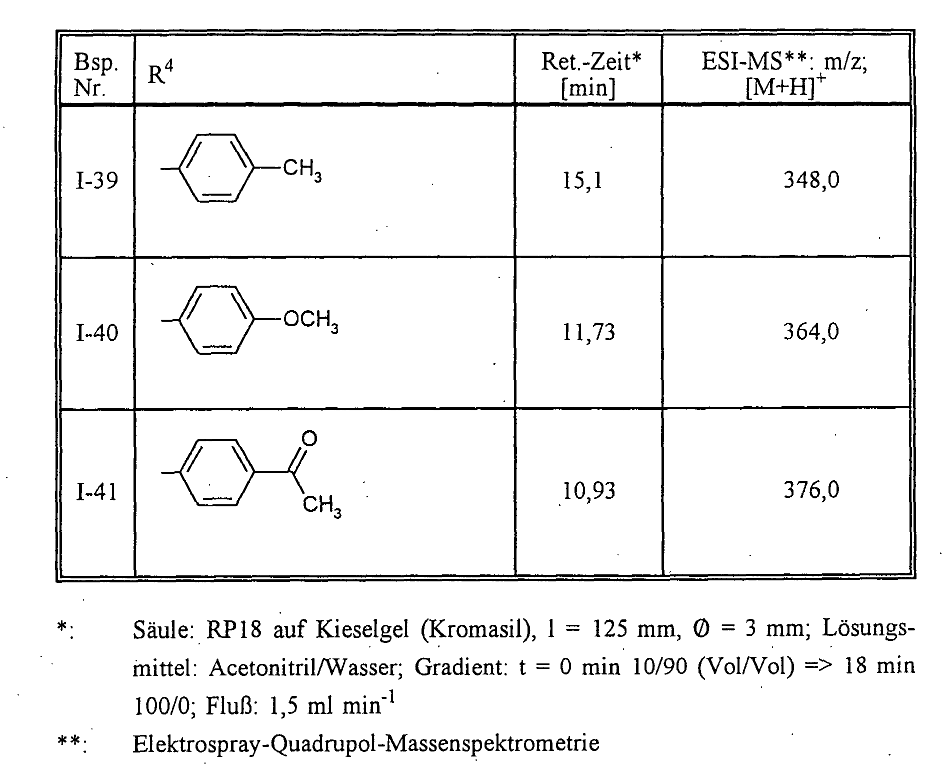

### Herstellung der Ausgangsprodukte

### γ-Ethoxy-γ-butyrolactam

9,91 g Succinimid wurden bei 0°C in 415 ml Ethanol vorgelegt und portionsweise mit insgesamt 5,53 g Natriumboranat versetzt. Es wurden bei dieser Temperatur über 4½ Stunden alle 15 Minuten 2 bis 3 Tropfen 2N ethanolischer Chlorwasserstoff zugetropft. Anschließend wurde mit weiterer Säure auf pH 3 angesäuert. Nach 1 Stunde Rühren bei 0°C wurde mit 1%-iger ethanolischer Kalilauge neutralisiert, weitere 15 Minuten gerührt und eingedampft. Der Rückstand wurde in Wasser aufgenommen und dreimal mit Dichlormethan extrahiert. Nach Trocknen über Natriumsulfat und Einengen wurden 7,16 g (55 % d.Th.) γ-Ethoxy-γ-butyrolactam erhalten.

### Beispiel XI-1

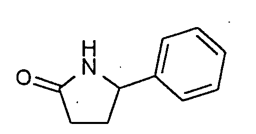

6,45 g γ-Ethoxy-γ-butyrolactam und 50 ml konz. Schwefelsäure wurden bei 0°C vorgelegt und 18,8 ml Benzol zugesetzt. Nach dem Auftauen wurde 4 Tage bei Raumtemperatur gerührt. Zur Aufarbeitung wurde auf Eis gegossen, dreimal mit Ethylacetat extrahiert, die vereinigten Extrakte je einmal mit Wasser und gesättigter Kochsalzlösung gewaschen, getrocknet und eingedampft. Es wurden 8,1 g (100 % d.Th.) γ-Phenyl-γ-butyrolactam erhalten.
¹H-NMR (400 MHz, d₆-DMSO) δ [ppm]: 1,75 (m, 1H); 2,23 (t, 2H); 2,45 (m, 1H); 4,67 (t, 1H); 7,26-7,39 (m, 5H); 8,08 (br, 1H)

### Beispiel XI-2

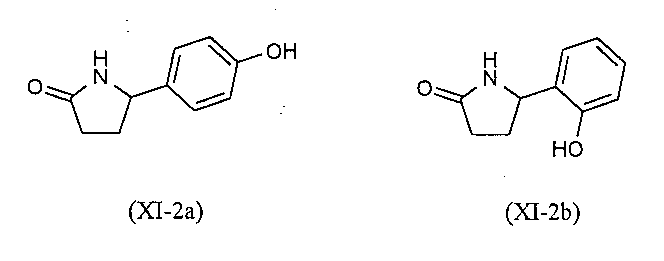

12,9 g γ-Ethoxy-γ-butyrolactam, 10 ml konz. Schwefelsäure und 90 ml Eisessig wurden bei 0°C vorgelegt und portionsweise mit insgesamt 18,8 g Phenol versetzt. Nach dem Auftauen wurde 2 Tage bei Raumtemperatur gerührt. Zur Aufarbeitung wurde auf Eis gegossen, dreimal mit Ethylacetat extrahiert, die vereinigten Extrakte je einmal mit Wasser und gesättigter Kochsalzlösung gewaschen, getrocknet und eingedampft. Aus der wäßrigen Phase kristallisierte nach einiger Zeit γ-2-Hydroxyphenyl-γ-butyrolactam (XI-2b) vom Schmelzpunkt 220°C aus (6,4 g 36 % d.Th). Der Eindampfrückstand wurde mit einem 1:1-Gemisch Cyclohexan/Ethylacetat verrührt und lieferte nach Absaugen 4,65 g γ-4-Hydroxyphenyl-γ-butyrolactam (XI-2a) vom Schmelzpunkt 183°C. Das Filtrat wurde eingedampft. Durch Umkristallisieren aus Dichlormethan/Hexan wurden weitere 3,35 g (gesamt: 45 % d.Th.) γ-4-Hydroxyphenyl-γ-butyrolactam erhalten.

### Beispiel XVII-2

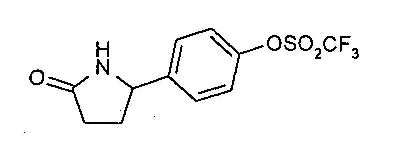

Zu 5,23 g γ-4-Hydroxyphenyl-γ-butyrolactam (z.B. aus Bsp. XI-2) in 60 ml Pyridin wurden bei 0°C 10 g Trifluormethansulfonsäureanhydrid getropft. Nach Rühren über Nacht bei Raumtemperatur wurde auf Eis gegossen, mit 10%-iger Salzsäure angesäuert und dreimal mit Ethylacetat extrahiert. Nach Trocknung und Abdampfen des Lösungsmittels wurden 6,4 g (70 % d.Th.) γ-4-Trifluormethylsulfonyloxyphenyl-γ-butyrolactam vom Schmelzpunkt 127°C erhalten.

### Beispiel XI-a-2

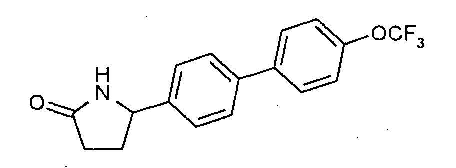

5,4 g γ-4-Trifluormethylsulfonyloxyphenyl-γ-butyrolactam (z.B. aus Bsp. XVII-2) wurden unter Argon in 43 ml Dimethoxyethan vorgelegt. Nacheinander gab man 5,87 g 4-Trifluormethoxyboronsäure sowie 1,01 g Tetrakis(triphenylphosphin)-palladium zu. Nach 15 Minuten wurden 28 ml 2M Sodalösung zugegeben, auf 80°C erwärmt und über Nacht gerührt. Nach Beendigung der Reaktion wurde in Wasser/Ethylacetat aufgenommen, die Phasen getrennt und die wäßrige Phase zweimal mit Ethylacetat extrahiert. Die vereinigten organischen Phasen wurden mit gesättigter Kochsalzlösung gewaschen und getrocknet. Durch Eindampfen wurden 5,5 g (98 % d.Th.) y-4'-Trifluormethoxybiphenyl-4-yl-γ-butyrolactam vom Schmelzpunkt 128°C erhalten.

### Beispiel XI-3

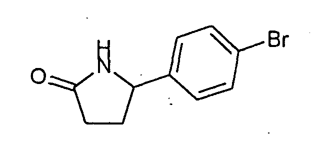

In einem 3 1-Dreihalskolben mit Rührer und Destillationsbrücke wurden 199,3 g Ammoniumformiat in 127,9 g Ameisensäure vorgelegt und 210 g aus Toluol umkristallisierte 4-Brombenzoylpropionsäure zugegeben. Darauf wurde der Kolben in ein 200°C heißes Ölbad getaucht. Bei 60°C beginnt der Kolbeninhalt unter Gasentwicklung in Lösung zu gehen. Über ca. 2 h wird bei von 140 bis auf 167°C steigender Sumpftemperatur wird ausdestilliert. Nach Abkühlen auf unter 60°C wurden vorsichtig 1 1 Dichlormethan zugegeben und ausgefallenes Salz durch Absaugen über eine Filternutsche abgetrennt. Die organische Phase wurde mit 1 1 Wasser gewaschen, über Magnesiumsulfat getrocknet und im Vakuum eingeengt. Zur Reinigung wurde das Rohprodukt wurde über 1 kg Kieselgel mit Dichlormethan/Ethanol/Triethylamin (95:5:3) filtriert und anschließend aus Methyl-tertbutyl-ether kristallisiert. Es wurden 38 g (19 % d.Th.) γ-4-Bromphenyl-γ-butyrolactam vom Schmelzpunkt 142°C erhalten.

### Beispiel IX-1

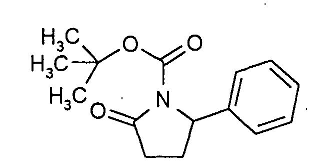

3,4 g γ-Phenyl-γ-butyrolactam (z.B aus Bsp. XI-1) wurden in 63 ml Tetrahydrofuran (THF) vorgelegt und bei -78°C mit 9,24 ml 2,4N Butyllithium-Lösung in n-Hexan versetzt. Nach halbstündigem Rühren bei dieser Temperatur wurde eine Lösung von 5,04 g Di-tert.-Butyldicarbonat in 20 ml THF unter weiterer Kühlung zugetropft, weitere 3 Stunden bei -78°C und dann über Nacht ohne Kühlung gerührt. Danach wurde mit gesättigter wäßriger Ammoniumchlorid-Lösung hydrolisiert, mit Wasser verdünnt und dreimal mit Ethylacetat extrahiert. Die vereinigten organischen Phasen wurden mit gesättigter Kochsalzlösung gewaschen und über Magnesiumsulfat getrocknet. Durch Eindampfen wurden 1,54 g (28 % d.Th.) N-^{t}Butoxycarbonyl-γ-phenyl-γ-butyrolactam erhalten.
¹H-NMR (400 MHz, d₆-DMSO) δ [ppm]: 1,18 (s, 9H); 1,73 (m, 1H); 2,40-2,60 (m, 3H); 5,10 (m, 1H), 7,24 (m, 2H); 7,30 (m, 1H); 7,38 (m, 2H)

### Beispiel IX-2

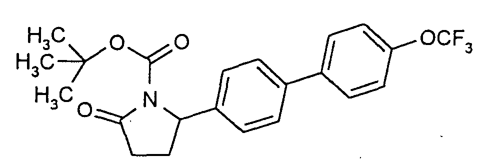

1,7 g γ-4'-Trifluormethoxybiphenyl-4-yl-γ-butyrolactam (z.B. aus Bsp. XI-a-2) wurden in 30 ml Tetrahydrofuran (THF) vorgelegt und bei -78°C mit 2,42 ml 2,4N Butyllithium-Lösung in n-Hexan versetzt. Nach halbstündigem Rühren bei dieser Temperatur wurde eine Lösung von 1,27 g Di-tert.-Butyldicarbonat in 10 ml THF unter weiterer Kühlung zugetropft. Dann wird die Kühlung entfernt und bei Raumtemperatur über Nacht gerührt. Danach wurde mit gesättigter wäßriger Ammoniumchlorid-Lösung hydrolisiert, mit 2N Salzsäure angesäuert und dreimal mit Dichlormethan extrahiert. Nach Trocknung und Eindampfen wurde das Produkt durch Säulenchromatographie gereinigt (stationäre Phase: Kieselgel; mobile Phase Gradient Cyclohexan:Ethylacetat = 5:1,3 bis 1,1:1). Es wurden 1,14 g (47 % d.Th.) teilkristallines *N*-^{t}Butoxycarbonyl-γ-4'-trifluormethoxybiphenyl-4-yl-γ-butyrolactam erhalten.
¹H-NMR (400 MHz, CDCl₃) δ [ppm]: 1,22 (s, 9H); 1,79 (m, 1H); 2,48-2,60 (m, 3H); 5,17 (m, 1H); 7,36 (d, 2H); 7,46 (d, 2H); 7,71 (d, 2H); 7,80 (d, 2H)

### Beispiel IX-3

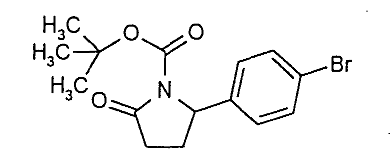

3,24 ml Diisopropylamin wurden in 90 ml THF bei -78°C vorgelegt und mit 9,24 ml 2,4N Butyllithium-Lösung in n-Hexan versetzt. Nach ½ h Rühren bei dieser Temperatur wurden eine Lösung von 5,02 g γ-4-Bromphenyl-γ-butyrolactam (z.B. aus Beispiel XI-3) in 20 ml THF zugetropft. Nach weiterer ½ h Rühren bei -78°C wurden 5,04 g Di-tert-Butyldicarbonat in 20 ml THF zugetropft, auftauen gelassen und bei Raumtemperatur über Nacht gerührt. Danach wurde mit gesättigter wäßriger Ammoniumchlorid-Lösung hydrolisiert, mit 2N Salzsäure angesäuert und mit 150 ml Dichlormethan dreimal extrahiert. Nach Trocknung über Magnesiumsulfat und Eindampfen wurde das Produkt durch Kristallisieren aus Dichlormethan/Hexan gereinigt. Es wurden insgesamt 7,61 g (97 % d.Th.) kristallines *N*-^{t}Butoxycarbonyl-γ-4-bromphenyl-γ-butyrolactam erhalten. Die reinste Kristallfraktion (2,34 g) schmolz bei 122-124°C.

### Beispiel VIII-1

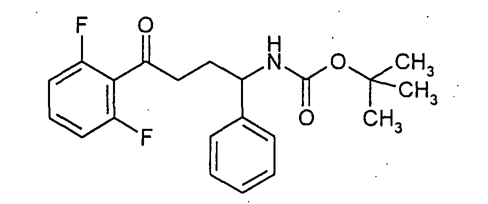

0,62 g 1,3-Difluorbenzol wurden in 15 ml THF vorgelegt und bei -78°C mit 2,4 ml 2,4N Butyllithium-Lösung in n-Hexan versetzt. Nach 1 Stunde Rühren wurde bei dieser Temperatur eine Lösung von 1,44 g *N*-^{t}Butoxycarbonyl-γ-phenyl-γ-butyrolactam (z.B. aus Bsp. IX-1) in 7 ml THF sehr langsam zugetropft. Es wurden 3 Stunden bei -78°C, dann über Nacht ohne Kühlung gerührt. Nach Hydrolyse mit Ammoniumchlorid-Lösung wurde dreimal mit Ethylacetat extrahiert, die vereinigten Extrakte wurden getrocknet und eingedampft. Aus Dichlormethan/Hexan wurden 1,03 g (50 % d.Th.) 1-^{t}Butoxycarbonylamino-3-(2,6-difluorbenzoyl)-1-(4'-trifluormethoxybiphenyl-4-yl)-propan erhalten.
¹H-NMR (400 MHz, d₆-DMSO) δ [ppm]: 1,33 (s, 9H); 1,94 (m, 2H); 2,89 (t, 2H); 4,54 (m, 1H); 7,22 (m, 3H); 7,30 (m, 4H); 7,42 (br d, 1H); 7,60 (m, 1H)

### Beispiel VIII-2

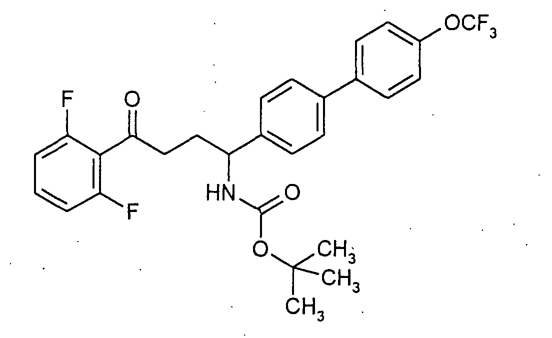

Analog zu Beispiel VIII-1 wurden aus 0,62 g 1,3-Difluorbenzol und 1,7 g N-^{t}Butoxycarbonyl-γ-4'-trifluormethoxybiphenyl-4-yl-γ-butyrolactam (z.B. aus Bsp. IX-2) 2,23 g (77 % d.Th.) 1-^{t}Butoxycarbonylamino-3-(2,6-difluorbenzoyl)-1-(4'trifluormethoxybiphenyl-4-yl)-propan als Öl erhalten.
¹H-NMR (400 MHz, CDCl₃) δ [ppm]: 1,40 (s, 9H); 2,20 (m, 2H); 2,97 (m, 2H); 4,75 (m, 1H); 6,93 (t, 2H); 7,28 (m, 3H); 7,38 (d, 2H); 7,53 (d, 2H); 7,58 (d, 2H)

### Beispiel VIII-3

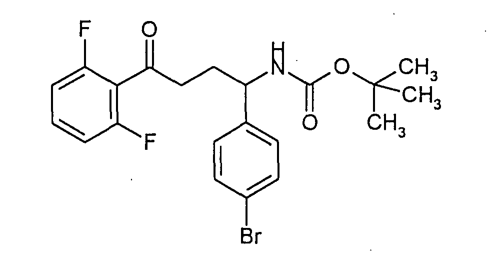

Analog zu Beispiel VIII-1 wurden aus 0,62 g 1,3-Difluorbenzol und 2,03 g N-tButoxycarbonyl-γ-4-bromphenyl-γ-butyrolactam (z.B. aus Bsp. IX-3) 2,51 g (93 % d.Th.) 1-^{t}Butoxycarbonylamino-3-(2,6-difluorbenzoyl)-1-(4-bromphenyl)-propan vom Schmelzbereich 111-115°C erhalten.

### Anwendungsbeispiele

### Beispiel A

### Phaedon-Larven-Test

| | | |
|---|---|---|
| Lösungsmittel | 7 Gewichtsteile | Dimethylformamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käfer-Larven abgetötet wurden; 0 % bedeutet, daß keine Käfer-Larven abgetötet wurden.

Bei diesem Test zeigt z.B. die Verbindung gemäß Herstellbeispiel I-2 bei einer beispielhaften Wirkstoffkonzentration von 0,1 % nach 7 Tagen einen Abtötungsgrad von 100 %.

### Beispiel B

### Spodoptera-Test

| | | |
|---|---|---|
| Lösungsmittel: | 7 Gewichtsteile | Dimethylformamid |
| Emulgator: | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Eulenfalters (Spodoptera frugiperda) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test zeigt z.B. die Verbindung gemäß Herstellbeispiel I-2 bei einer beispielhaften Wirkstoffkonzentration von 0,1 % nach 7 Tagen einen Abtötungsgrad von 100 %.

### Beispiel C

### Nephotettix-Test

| | | |
|---|---|---|
| Lösungsmittel: | 7 Gewichtsteile | Dimethylforamid |
| Emulgator: | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Reiskeimlinge (Oryzae sativa) werden durch Tauchen in die Wirkstoffzubereitung der gewünschten Konzentration behandelt und mit Larven der Grünen Reiszikade (Nephotettix cincticeps) besetzt, solange die Keimlinge noch feucht sind.

Nach der gewünschten Zeit wird die Abtötung in % bestimmt. Dabei bedeutet 100 %, daß alle Zikaden abgetötet wurden; 0 % bedeutet, daß keine Zikaden abgetötet wurden.

Bei diesem Test zeigt z.B. die Verbindung gemäß Herstellbeispiel I-1 bei einer beispielhaften Wirkstoffkonzentration von 0,1 % nach 6 Tagen einen Abtötungsgrad von 100 %.

### Beispiel D

### Myzus-Test

| | | |
|---|---|---|
| Lösungsmittel: | 7 Gewichtsteile | Dimethylformamid |
| Emulgator: | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea), die stark von der Pfirsichblattlaus (Myzus persicae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Blattläuse abgetötet wurden; 0 % bedeutet, daß keine Blattläuse abgetötet wurden.

Bei diesem Test zeigten z.B. die folgenden Verbindungen gute Wirksamkeit:

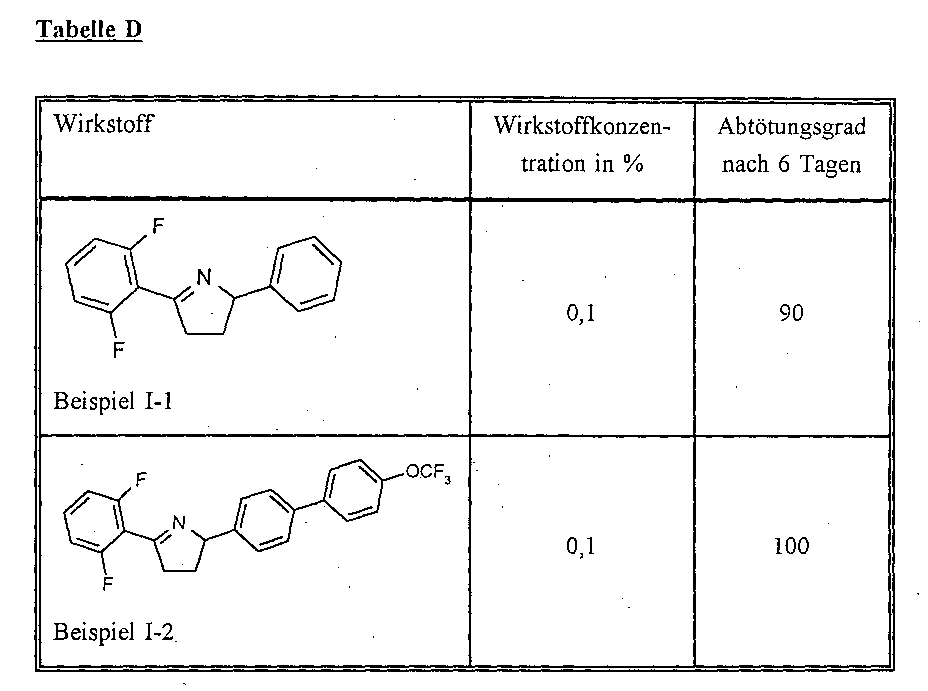

### Beispiel E

### Tetranychus-Test (OP-resistent/Tauchbehandlung)

| | | |
|---|---|---|
| Lösungsmittel: | 3 Gewichtsteile | Dimethylformamid |
| Emulgator: | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit emulgatorhaltigem Wasser auf die gewünschte Konzentration.

Bohnenpflanzen (Phaseolus vulgaris), die stark von allen Stadien der gemeinen Spinnmilbe (Tetranychus urticae) befallen sind, werden in eine Wirkstoffzubereitung der gewünschten Konzentration getaucht.

Nach der gewünschten Zeit wird die Wirkung in % bestimmt. Dabei bedeutet 100 %, daß alle Spinnmilben abgetötet wurden; 0 % bedeutet, daß keine Spinnmilben abgetötet wurden.

Bei diesem Test zeigte nach 7 Tagen z.B. die Verbindung gemäß Herstellbeispiel I-2 bei einer beispielhaften Wirkstoffkonzentration von 0,001 % einen Abtötungsgrad von 100 %.

### Beispiel F

### Test mit Fliegenlarven / Entwicklungshemmende Wirkung

| | |
|---|---|
| Testtierez | Alle larvalen Stadien von Lucilia cuprina (OP-resistent) [Puppen und Adulte (ohne Kontakt zum Wirkstoff)] |
| Lösungsmittel | 35 Gewichtsteile Ethylenglykolmonomethylether 35 Gewichtsteile Nonylphenolpolyglykolether |

Zwecks Herstellung einer geeigneten Formulierung vermischt man 3 Gewichtsteile Wirkstoff mit 7 Teilen des oben angegebenen Lösungsmittel-Emulgator-Gemisches und verdünnt das so erhaltene Emulsionskonzentrat mit Wasser auf die jeweils gewünschte Konzentration.

30 bis 50 Larven je Konzentration werden auf in Glasröhrchen befindliches Pferdefleisch (1 cm³) gebracht, auf welches 500 µl der zu testenden Verdünnung pipettiert werden. Die Glasröhrchen werden in Kunststoffbecher gestellt, deren Boden mit Seesand bedeckt ist, und im klimatisierten Raum (26°C ± 1,5°C, 70 % rel. Feuchte ± 10 %) aufbewahrt. Die Wirkungskontrolle erfolgt nach 24 Stunden und 48 Stunden (larvizide Wirkung). Nach dem Auswandern der Larven (ca. 72 h) werden die Glasröhrchen entfernt und gelochte Kunststoffdeckel auf die Becher gesetzt. Nach 1½-facher Entwicklungsdauer (Schlupf der Kontrollfliegen) werden die geschlüpften Fliegen und die Puppen/Puppenhüllen ausgezählt.

Als Kriterium für die Wirkung gilt der Eintritt des Todes bei den behandelten Larven nach 48 h (larvizider Effekt), bzw. die Hemmung des Adultschlupfes aus den Puppen bzw. die Hemmung der Puppenbildung. Als Kriterium für die in-vitro-Wirkung einer Substanz gilt die Hemmung der Fliegenentwicklung bzw. ein Entwicklungsstillstand vor dem Adulten-Stadium. Dabei bedeutet 100 % larvizide Wirkung, daß nach 48 Stunden alle Larven abgestorben sind. 100 % entwicklungsinhibitorische Wirkung bedeutet, daß keine adulten Fliegen geschlüpft sind.

In diesem Test hatte z.B. die Verbindung gemäß Herstellbeispiel I-1 bei einer beispielhaften Wirkstoffkonzentration von 1000 ppm eine Wirkung von 100 %.

### Beispiel G

### Nymphenhäutungstest an mehrwirtigen Zecken

| | |
|---|---|
| Testtiere | Amblyomma variegatium, vollgesogene Zeckennymphen |
| Lösungsmittel | 35 Gewichtsteile Ethylenglykolmonomethylether 35 Gewichtsteile Nonylphenolpolyglykolether |

Zwecks Herstellung einer geeigneten Formulierung vermischt man 3 Gewichtsteile Wirkstoff mit 7 Teilen des oben angegebenen Lösungsmittel-Emulgator-Gemisches und verdünnt das so erhaltene Emulsionskonzentrat mit Wasser auf die jeweils gewünschte Konzentration.

10 vollgesogene Nymphen werden in die zu testende Wirkstoffzubereitung 1 Minute getaucht. Die Tiere werden auf mit Filterscheiben bestückte Petrischalen (∅ 9,5 cm) überführt und abgedeckt. Nach 4 Wochen Aufbewahrung in einem klimatisierten Raum wird die Häutungsrate bestimmt.

Dabei bedeutet 100 %, daß sich alle Tiere normal gehäutet haben; 0 % bedeutet, daß sich keine Tiere normal gehäutet haben.

In diesem Test hatten z.B. die Verbindungen gemäß den Herstellbeispielen I-1 und I-2 bei einer beispielhaften Wirkstoffkonzentration von 1000 ppm eine Wirkung von jeweils 100 %.

## Patentansprüche

1. Verbindungen der Formel (XI), 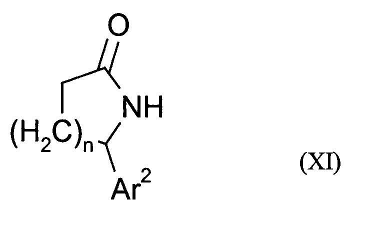 in welcher
n für 1, 2 oder 3 steht,
Ar² für den Rest 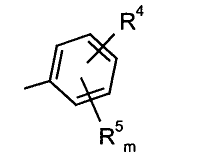 steht, in denen
m für 0, 1, 2 oder 3 steht,
R⁴ für einen Substituenten in meta- oder para-Position aus der Reihe Halogen, Cyano, Tri-(C₁-C₆-alkyl)-silyl, -CO-NR¹⁰R¹¹, Tetrahydropyranyl oder eine der folgenden Gruppierungen
(l) -X-A
(m) -B-Z-D
(n) -Y-E steht,
R⁵ für Wasserstoff, Halogen, Cyano, Nitro, C₁-C₁₆-Alkyl, C₁-C₁₆-Alkoxy, C₁-C₆-Halogenalkyl, C₁-C₆-Halogenalkoxy, C₁-C₈-Alkoxy-C₁-C₈-alkoxy oder -S(O)ₒR⁶ steht,
o für 0, 1 oder 2 steht,
R⁶ für gegebenenfalls durch Fluor oder Chlor substituiertes C₁-C₆-Alkyl steht,
R¹⁰ und R¹¹ unabhängig voneinander für Wasserstoff, C₁-C₆-Alkyl, C₁-C₆-Halogenalkyl oder für jeweils gegebenenfalls einfach bis dreifach durch Reste aus der Liste W¹ substituiertes Phenyl oder Phenyl-C₁-C₄-alkyl stehen,
X für eine direkte Bindung, Sauerstoff, Schwefel, Carbonyl, Carbonyloxy, Oxycarbonyl, C₁-C₄-Alkylen, C₂-C₄-Alkenylen, C₂-C₄-Alkinylen, C₁-C₄-Alkylenoxy, C₁-C₄-Oxyalkylen, C₁-C₄-Thioalkylen, C₁-C₄-Alkylendioxy oder Di-C₁-C₄-alkylsilylen steht,
A für jeweils gegebenenfalls einfach bis vierfach durch Reste aus der Liste W¹ substituiertes Phenyl, Naphthyl oder Tetrahydronaphthyl oder für jeweils gegebenenfalls einfach bis vierfach durch Reste aus der Liste W² substituiertes 5- bis 10-gliedriges, 1 oder 2 aromatische Ringe enthaltendes Heterocyclyl mit 1 bis 4 Heteroatomen steht, welches 0 bis 4 Stickstoffatome, 0 bis 2 Sauerstoffatome und 0 bis 2 Schwefelatome enthält,
B für gegebenenfalls einfach oder zweifach durch Reste aus der Liste W¹ substituiertes p-Phenylen steht,
Z für Sauerstoff oder Schwefel steht,
D für Wasserstoff, C₁-C₁₆-Alkyl, C₂-C₁₆-Alkenyl, C₂-C₆-Alkinyl, C₁-C₁₆-Halogenalkyl, C₂-C₁₆-Halogenalkenyl, jeweils gegebenenfalls durch Halogen, C₁-C₄-Alkyl, C₂-C₄-Alkenyl, C₂-C₄-Halogenalkenyl, Phenyl, Styryl, Halogenphenyl oder Halogenstyryl substituiertes C₃-C₈-Cycloalkyl oder C₃-C₈-Cycloalkyl-C₁-C₆-alkyl, für jeweils gegebenenfalls durch Halogen oder C₁-C₄-Alkyl substituiertes C₅-C₈-Cycloalkenyl oder C₅-C₈-Cycloalkenyl-C₁-C₄-alkyl, für jeweils gegebenenfalls durch Nitro, Halogen, C₁-C₆-Alkyl, C₁-C₆-Alkoxy, C₁-C₆-Halogenalkyl oder C₁-C₆-Halogenalkoxy substituiertes Phenyl-C₁-C₆-alkyl, Naphthyl-C₁-C₆-alkyl, Tetrahydronaphthyl-C₁-C₆-alkyl oder 5- oder 6-ringgliedriges Hetaryl-C₁-C₆-alkyl mit 1 oder 2 Heteroatomen aus der Reihe Stickstoff, Sauerstoff und Schwefel für -CO-R¹², -CO-NR¹³R¹⁴ oder für die Gruppierung
-(CH₂)ₚ-(CR¹⁵R¹⁶)_{q}-(CH₂)ᵣ-G
steht, oder
Z und D gemeinsam für gegebenenfalls durch Nitro, Halogen, C₁-C₆-Alkyl, C₁-C₆-Alkoxy, C₁-C₆-Halogenalkyl oder C₁-C₆-Halogenalkoxy substituiertes Phenoxy-C₁-C₄-alkyl stehen,
Y für eine direkte Bindung, Sauerstoff, Schwefel, Carbonyl, Carbonyloxy, Oxycarbonyl, C₁-C₄-Alkylen, C₂-C₄-Alkenylen, C₂-C₄-Alkinylen, C₁-C₄-Alkylenoxy, C₁-C₄-Oxyalkylen, C₁-C₄-Thioalkylen, C₁-C₄-Alkylendioxy oder für gegebenenfalls einfach oder zweifach durch Reste aus der Liste W¹ substituiertes p-Phenylen steht,
E für Wasserstoff, C₁-C₁₆-Alkyl, C₂-C₁₆-Alkenyl, C₂-C₆-Alkinyl, C₁-C₁₆-Halogenalkyl, C₂-C₁₆-Halogenalkenyl, gegebenenfalls durch Halogen, C₁-C₄-Alkyl, C₂-C₄-Alkenyl, C₂-C₄-Halogenalkenyl, Phenyl, Styryl, Halogenphenyl oder Halogenstyryl substituiertes C₃-C₈-Cycloalkyl, für gegebenenfalls durch Halogen oder C₁-C₄-Alkyl substituiertes C₅-C₈-Cycloalkenyl, für gegebenenfalls einfach bis vierfach durch Reste aus der Liste W¹ substituiertes Phenyl oder für jeweils gegebenenfalls einfach bis vierfach durch Reste aus der Liste W² substituiertes 5- oder 6-gliedriges Hetaryl mit 1 oder 2 Heteroatomen aus der Reihe Stickstoff, Sauerstoff und Schwefel oder für die Gruppierung
-(CH₂)ₚ-(CR¹⁵R¹⁶)_{q}-(CH₂)ᵣ-G
steht,
R¹² für C₁-C₁₂-Alkyl, C₁-C₁₂-Alkoxy, C₂-C₁₂-Alkenyl, C₂-C₁₂-Alkenyloxy, jeweils gegebenenfalls durch Halogen, C₁-C₄-Alkyl, C₂-C₄-Alkenyl, C₁-C₄-Halogenalkyl oder C₂-C₄-Halogenalkenyl substituiertes C₃-C₈-Cycloalkyl, C₃-C₈-Cycloalkyloxy oder C₃-C₈-Cycloalkyl-C₁-C₆-alkyloxy oder für jeweils gegebenenfalls einfach bis vierfach durch Nitro, Halogen, C₁-C₁₂-Alkyl, C₁-C₁₂-Alkoxy, C₁-C₁₂-Halogenalkyl oder C₁-C₁₂-Halogenalkoxy substituiertes Phenyl oder Naphthyl steht,
R¹³ für Wasserstoff oder C₁-C₁₂-Alkyl steht,
R¹⁴ für C₁-C₁₂-Alkyl, C₁-C₁₂-Halogenalkyl, jeweils gegebenenfalls durch Halogen, C₁-C₄-Alkyl, C₂-C₄-Alkenyl, C₁-C₄-Halogenalkyl oder C₂-C₄-Halogenalkenyl substituiertes C₃-C₈-Cycloalkyl oder C₃-C₈-Cycloalkyl-C₁-C₆-alkyl oder für jeweils gegebenenfalls einfach bis vierfach durch Halogen, C₁-C₁₂-Alkyl, C₁-C₁₂-Alkoxy, C₁-C₁₂-Halogenalkyl oder C₁-C₁₂-Halogenalkoxy substituiertes Phenyl oder Phenyl-C₁-C₆-alkyl steht,
p, q und r unabhängig voneinander für 0, 1, 2 oder 3 stehen, wobei deren Summe kleiner 6 ist,
R¹⁵ und R¹⁶ unabhängig voneinander für Wasserstoff oder C₁-C₄-Alkyl stehen,
G für Cyano, für einen gegebenenfalls einfach bis dreifach durch Halogen, C₁-C₄-Alkyl oder C₁-C₄-Halogenalkyl und gegebenenfalls an der Verknüpfungsstelle durch den Rest R¹⁷ substituierten 5- oder 6- gliedrigen Heterocyclus mit 1 bis 3 gleichen oder verschiedenen Heteroatomen aus der Reihe Stickstoff, Sauerstoff und Schwefel oder eine der folgenden Gruppierungen steht:
(a) -CO-R¹⁷
(b) -CO-OR¹⁸
(c) -CO-NR¹⁹R²⁰
(d) -CS-NR¹⁹R²⁰ 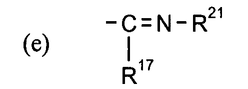 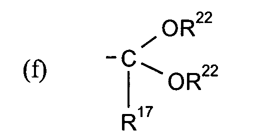 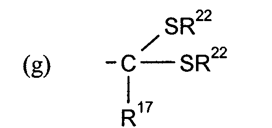 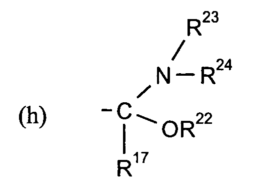 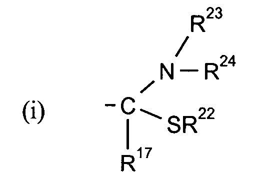 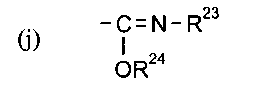 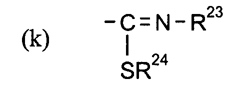
R¹⁷ für Wasserstoff, C₁-C₆-Alkyl, C₂-C₆-Alkenyl, C₁-C₄-Halogenalkyl, C₂-C₆-Halogenalkenyl, gegebenenfalls durch Halogen, C₁-C₄-Alkyl oder C₁-C₄-Halogenalkyl substituiertes C₃-C₆-Cycloalkyl oder für gegebenenfalls einfach bis fünffach durch C₁-C₄-Alkylcarbonylamino, C₁-C₄-Alkylcarbonyl-C₁-C₄-alkylamino und/oder Reste aus der Liste W³ substituiertes Phenyl steht,
R¹⁸ für Wasserstoff, C₁-C₄-Alkyl, C₂-C₆-Alkenyl, C₁-C₄-Halogenalkyl, C₂-C₆-Halogenalkenyl, jeweils gegebenenfalls durch Halogen, C₁-C₄-Alkyl oder C₁-C₄-Halogenalkyl substituiertes C₃-C₆-Cycloalkyl oder C₃-C₆-Cycloalkyl-C₁-C₄-alkyl oder für gegebenenfalls einfach bis vierfach durch Reste aus der Liste W³ substituiertes C₆-C₁₀-Aryl-C₁-C₄-alkyl steht,
R¹⁹ und R²⁰ unabhängig voneinander für Wasserstoff, C₁-C₄-Alkyl, C₃-C₆-Alkenyl, C₁-C₄-Halogenalkyl, C₃-C₆-Halogenalkenyl, C₁-C₄-Alkoxy, jeweils gegebenenfalls durch Halogen, C₁-C₄-Alkyl oder C₁-C₄-Halogenalkyl substituiertes C₃-C₆-Cycloalkyl oder C₃-C₆-Cycloalkyl-C₁-C₄-alkyl, für jeweils gegebenenfalls einfach bis fünffach durch Reste aus der Liste W³ substituiertes Phenyl oder Phenyl-C₁-C₄-alkyl, für -OR¹⁸ oder -NR¹⁷R¹⁸ oder gemeinsam für eine Alkylenkette mit 4 bis 6 Gliedern stehen, in welcher gegebenenfalls eine Methylengruppe durch Sauerstoff ersetzt ist,
R²¹ für -OR¹⁸, -NR¹⁷R¹⁸ oder -N(R¹⁷)-COOR¹⁸ steht,
R²², R²³ und R²⁴ unabhängig voneinander für C₁-C₆-Alkyl stehen,
W¹ für Wasserstoff, Halogen, Cyano, Formyl, Nitro, C₁-C₆-Alkyl, Tri-C₁-C₄-alkylsilyl, C₁-C₁₆-Alkoxy, C₁-C₆-Halogenalkyl, C₁-C₆-Halogenalkoxy, C₂-C₆-Halogenalkenyloxy, C₁-C₆-Alkylcarbonyl, C₁-C₁₆-Alkoxycarbonyl, Pentafluorthio oder -S(O)ₒR⁶ steht,
W² für Halogen, Cyano, Formyl, Nitro, C₁-C₆-Alkyl, Tri-C₁-C₄-alkylsilyl, C₁-C₁₆-Alkoxy, C₁-C₆-Halogenalkyl, C₁-C₆-Halogenalkoxy, C₁-C₆-Alkylcarbonyl, C₁-C₁₆-Alkoxycarbonyl, Pentafluorthio, -S(O)ₒR⁶ oder -C(R¹⁷)=N-R²¹ steht,
W³ für Halogen, Cyano, Nitro, C₁-C₄-Alkyl, C₁-C₄-Alkoxy, C₁-C₄-Halogenalkyl, C₁-C₄-Halogenalkoxy, Di-C₁-C₄-alkylamino, -S(O)ₒR⁶, -COOR²⁵ oder -CONR²⁶R²⁷ steht,
R²⁵ für Wasserstoff, C₁-C₄-Alkyl, C₁-C₄-Halogenalkyl, gegebenenfalls durch Halogen, C₁-C₄-Alkyl oder C₁-C₄-Halogenalkyl substituiertes C₃-C₇-Cycloalkyl oder für gegebenenfalls einfach bis fünffach durch Reste aus der Liste W⁴ substituiertes Phenyl steht,
R²⁶ und R²⁷ unabhängig voneinander für Wasserstoff, C₁-C₄-Alkyl, C₃-C₆-Alkenyl, C₁-C₄-Halogenalkyl, C₃-C₆-Halogenalkenyl, C₁-C₄-Alkoxy, jeweils gegebenenfalls durch Halogen, C₁-C₄-Alkyl oder C₁-C₄-Halogenalkyl substituiertes C₃-C₆-Cycloalkyl oder C₃-C₆-Cycloalkyl-C₁-C₄-alkyl oder für jeweils gegebenenfalls einfach bis fünffach durch Reste aus der Liste W⁴ substituiertes Phenyl oder Phenyl-C₁-C₄-alkyl, für -OR²² oder -NR²³R²⁴ oder gemeinsam für eine Alkylenkette mit 4 bis 6 Gliedern stehen, in welcher gegebenenfalls eine Methylengruppe durch Sauerstoff ersetzt ist und
W⁴ für Halogen, Cyano, Nitro, C₁-C₆-Alkyl, C₁-C₆-Alkoxy, C₁-C₆-Halogenalkyl, C₁-C₆-Halogenalkoxy, Di-C₁-C₄-alkylamino, C₁-C₆-Alkoxycarbonyl, Di-C₁-C₆-alkylaminocarbonyl oder -S(O)ₒR⁶ steht.

2. Verbindungen der Formel (XI-a) 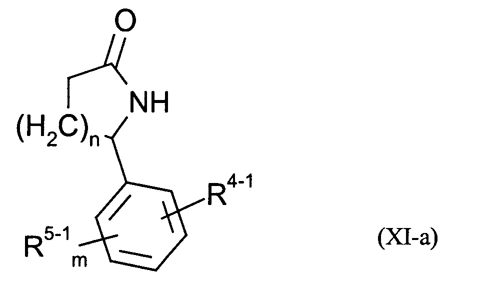 in welcher
n und m die in Anspruch 1 angegebenen Bedeutungen haben,
R⁴⁻¹ für A oder eine der folgenden Gruppierungen
(m) -B-Z-D 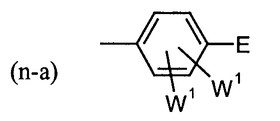
steht, wobei
A, B, D, E, W¹ und Z die in Anspruch 1 angegebenen Bedeutungen haben und
R⁵⁻¹ für Wasserstoff, Fluor, Cyano, Nitro, Alkyl, Alkoxy, Halogenalkyl, Halogenalkoxy, Alkoxyalkoxy oder -SR⁶ steht, wobei
R⁶ die in Anspruch 1 angegebene Bedeutung hat.
